# EUROPEAN PATENT SPECIFICATION

(11) **EP 1 549 670 B1**
(45) Date of publication and mention of the grant of the patent: **30.04.2008**
(21) Application number: 02807965.5
(22) Date of filing: 02.10.2002
(51) Int. Cl.: C07K 7/64, A61K 38/04

(54) **TEMPLATE-FIXED PEPTIDOMIMETICS WITH ANTIMICROBIAL ACTIVITY**
MATRIZENFIXIERTE PEPTIDOMIMETIKA MIT ANTIMIKROBIELLER WIRKUNG
PEPTIDOMIMETIQUES FIXES A UNE MATRICE AYANT UNE ACTION ANTIMICROBIENNE

(43) Date of publication of application: 06.07.2005
(73) Proprietor: Polyphor Ltd., 4123 Allschwil (CH); Universität Zürich, 8006 Zürich (CH)
(72) Inventor: Vrijbloed, Jan Wim, 4313 Möhlin (CH); Obrecht, Daniel, 4112 Bättwil (CH); Locioro, Sergio, 4153 Reinach (CH); Gombert, Frank, 79588 Huttingen (DE); Ludin, Christian, 4104 Oberwil (CH); Robinson, John Anthony, 8615 Wermatswil (CH); Lederer, Alexander, 8037 Zürich (CH); Shankaramma, Shivaprasad, Knoxville, TN 37919 (US)
(74) Representative: Braun, André jr.
(86) International application number: PCT/EP2002/011048
(87) International publication number: WO 2004/033489

(56) References cited:
- WO-A-01/16161
- WO-A-02/070547

## Description

The present invention provides template-fixed β-hairpin peptidomimetics incorporating a template-fixed chain of 12 α-amino acid residues which, depending on their positions in the chain, are Gly or Pro, or of certain types, as defined herein below, at least one of these residues being of the type of N-substituted glycines These template-fixed β-hairpin mimetics have broad spectrum antimicrobial activity. In addition, the present invention provides efficient synthetic processes by which these compounds can, if desired, be made in parallel library-format. These β-hairpin peptidomimetics show improved efficacy, bioavailability, half-life and most importantly a significantly enhanced ratio between antibacterial on the one hand, and hemolysis of red blood cells on the other.

The growing problem of microbial resistance to established antibiotics has stimulated intense interest in developing novel antimicrobial agents with new modes of action (H. Breithaupt, Nat. Biotechnol. 1999, 17, 1165-1169). One emerging class of antibiotics is based on naturally occurring cationic peptides (T. Ganz, R. I. Lehrer, Mol. Medicine Today 1999, 5, 292-297; R. M. Epand, H. J. Vogel, Biochim. Biophys. Acta 1999, 1462, 11-28). These include disulfide-bridged β-hairpin and β-sheet peptides (such as the *protegrins* [V. N.. M.; O. V. Shamova, H. A. Komeva, R. I. Lehrer, FEBS Lett. 1993, 327, 231-236], *tachyplesins* [T. Nakamura, H. Furunaka, T. Miyata, F. Tokunaga, T. Muta, S. Iwanaga, M. Niwa, T. Takao, Y. Shimonishi, Y.J. Biol. Chem. 1988, 263, 16709-16713], and the *defensins* [R. 1. Lehrer, A. K. Lichtenstein, T. Ganz, Annu. Rev. Immunol. 1993, 11, 105-128], amphipathic α-helical peptides (e.g. *cecropins, dermaseptins, magainins,* and *mellitins* [A. Tossi, L. Sandri, A. Giangaspero, Biopolymers 2000, 55, 4-30]), as well as other linear and loop-structured peptides. Although the mechanisms of action of antimicrobial cationic peptides are not yet fully understood, their primary site of interaction is the microbial cell membrane (H. W. Huang, Biochemistry 2000, 39, 8347-8352). Upon exposure to these agents, the cell membrane undergoes permeabilization, which is followed by rapid cell death. However, more complex mechanisms of action, for example, involving receptor-mediated signaling, cannot presently be ruled out (M. Wu, E. Maier, R. Benz, R. E. Hancock, Biochemistry 1999, 38, 7235-7242).

The antimicrobial activities of many of these cationic peptides usually correlate with their preferred secondary structures, observed either in aqueous solution or in membrane-like environments (N. Sitaram, R. Nagaraj, Biochim. Biophys. Acta 1999, 1462, 29-54). Structural studies by nuclear magnetic resonance (NMR) spectroscopy have shown that cationic peptides such as protegrin 1 (A. Aumelas, M. Mangoni, C. Roumestand, L. Chiche, E. Despaux, G. Grassy, B. Calas, A. Chavanieu, A. Eur. J. Biochem. 1996, 237, 575-583; R. L. Fahrner, T. Dieckmann, S. S. L. Harwig, R. I. Lehrer, D. Eisenberg, J. Feigon, J. Chem. Biol. 1996, 3, 543-550) and tachyplesin I (K. Kawano, T. Yoneya, T. Miyata, K. Yoshikawa, F. Tokunaga, Y. Terada, S. J. Iwanaga, S. J. Biol. Chem. 1990, 265, 15365-15367) adopt well defined β-hairpin conformations, due to the constraining effect of two disulfide bridges. In protegrin analogues lacking one or both of these disulfide bonds, the stability of the β-hairpin conformation is diminished, and the antimicrobial activity is reduced (J. Chen, T. J. Falla, H. J. Liu, M. A. Hurst, C. A. Fujii, D. A. Mosca, J. R. EmbreeD. J. Loury, P. A. Radel, C. C. Chang, L. Gu, J. C. Fiddes, Biopolymers 2000, 55, 88-98; S. L. Harwig, A. Waring, H. J. Yang, Y. Cho, L. Tan, R. I. Lehrer, R. J. Eur. J. Biochem. 1996, 240, 352-357; M. E. Mangoni, A. Aumelas, P. Charnet, C. Roumestand, L. Chiche, E. Despaux, G. Grassy, B. Calas, A. Chavanieu, FEBS Lett. 1996, 383, 93-98; H. Tamamura, T. Murakami, S. Noriuchi, K. Sugihara, A. Otaka, W. Takada, T. Ibuka, M. Waki, N. Tamamoto, N. Fujii, Chem. Pharm. Bull. 1995, 43, 853-858). Similar observations have been made in analogues of tachyplesin I (H. Tamamura, R. Ikoma, M. Niwa, S. Funakoshi, T. Murakami, N. Fujii, Chem. Pharm. Bull. 1993, 41, 978-980) and in hairpin-loop mimetics of rabbit defensin NP-2 (S. Thennarasu, R. Nagaraj, Biochem. Biophys. Res. Comm. 1999, 254, 281-283). These results show that the β-hairpin structure plays an important role in the antimicrobial activity and stability of these protegrin-like peptides. In the case of the cationic peptides preferring α-helical structures, the amphililic structure of the helix appears to play a key role in determining antimicrobial activity (A. Tossi, L. Sandri, A. Giangaspero, A. Biopolymers 2000, 55, 4-30). Gramicidin S is a backbone-cyclic peptide with a well defined β-hairpin structure (S. E. Hull, R. Karlsson, P. Main, M. M. Woolfson, E. J. Dodson, Nature 1978, 275, 206-275) that displays potent antimicrobial activity against gram-positive and gram-negative bacteria (L. H. Kondejewski, S. W. Farmer, D. S. Wishart, R. E. Hancock, R. S. Hodges, Int. J. Peptide Prot. Res. 1996, 47, 460-466). The high hemolytic activity of *gramicidin S* has, however, hindered its widespread use as an antibiotic. Recent structural studies by NMR have indicated that the high hemolytic activity apparently correlates with the highly amphipathic nature of this cyclic β-hairpin-like molecule, but that it is possible to dissociate antimicrobial and hemolytic activities by modulating the conformation and amphiphilicity (L. H. Kondejewski, M. Jelokhani-Niaraki, S. W. Farmer, B. Lix, M. Kay, B. D. Sykes, R. E. Hancock, R. S. Hodges, J. Biol. Chem. 1999, 274, 13181-13192; C. McInnesL. H. Kondejewski, R. S. Hodges, B. D. Sykes, J. Biol. Chem. 2000, 275, 14287-14294).

A new cyclic antimicrobial peptide RTD-1 was reported recently from primate leukocytes (Y.-Q. Tang, J. Yuan, G. Ösapay, K. Ösapay, D. Tran, C. J. Miller, A. J. Oellette, M. E. Selsted, Science 1999, 286, 498-502. This peptide contains three disulfide bridges, which act to constrain the cyclic peptide backbone into a hairpin geometry. Cleavage of the three disulfide bonds leads to a significant loss of antimicrobial activity. Analogues of protegrins (J. P. Tam, C. Wu, J.-L. Yang, Eur. J. Biochem. 2000, 267, 3289-3300) and tachyplesins (J.-P. Tam, Y.-A. Lu, J.-L. Yang, Biochemistry 2000, 39, 7159-7169; N. Sitaram, R. Nagaraij, Biochem. Biophys. Res. Comm. 2000, 267, 783-790) containing a cyclic peptide backbone, as well as multiple disulfide bridges to enforce a amphiphilic hairpin structure, have also been reported. In these cases, removal of all the cystine constraints does not always lead to a large loss of antimicrobial activity, but does modulate the membranolytic selectivity (J. P. Tam, C. Wu, J.-L. Yang, Eur. J. Biochem. 2000, 267, 3289-3300).
A key issue in the design of new cationic antimicrobial peptides is selectivity. The naturally occurring protegrins and tachyplesins exert a significant hemolytic activity against human red blood cells. This is also the case for protegrin analogues such as IB367 (J. Chen, T. J. Falla, H. J. Liu, M. A. Hurst, C. A. Fujii, D. A. Mosca, J. R. Embree, D. J. Loury, P. A. Radel, C. C. Chang, L. Gu, J. C. Fiddes, Biopolymers 2000, 55, 88-98; C. Chang, L. Gu, J. Chen, US-Pat: 5,916,872, 1999). This high hemolytic activity essentially obviates its use in vivo, and represents a serious disadvantage in clinical applications. Also, the antibiotic activity of analogues often decreases significantly with increasing salt concentration, such that under in vivo conditions (ca. 100-150 mM NaCI) the antimicrobial activity may be severely reduced. Before intravenous use can be considered, the general toxicity, protein-binding activity in blood serum, as well as protease stability become serious issues which must be adequately addressed.

*Protegrin I* exhibits potent and similar activity against gram-positive and gram-negative bacteria as well as fungi in both low- and high-salt assays. This broad antimicrobial activity combined with a rapid mode of action, and their ability to kill bacteria resistant to other classes of antibiotics, make them attractive targets for development of clinically useful antibiotics. The activity against gram-positive bacteria is typically higher than against gram-negative bacteria. However, protegrin 1 also exhibits a high hemolytic activity against human red blood cells, and hence a low selectivity towards microbial cells. Oriented CD experiments (W. T. Heller, A. J. Waring, R. I. Lehrer, H. W. Huang, Biochemistry 1998, 37, 17331-17338) indicate that protegrin 1 may exist in two different states as it interacts with membranes, and these states are strongly influenced by lipid composition. Studies of cyclic protegrin analogues (J.-P. Tam, C. Wu, J.-L. Yang, Eur. J. Biochem. 2000, 267, 3289-3300) have revealed, that an increase in the conformational rigidity, resulting from backbone cyclization and multiple disulfide bridges, may confer membranolytic selectivity that dissociates antimicrobial activity from hemolytic activity, at least in the series of compounds studied.
*Protegrin 1* is an 18 residues linear peptide, with an amidated carboxyl terminus and two disulfide bridges. *Tachyplesin I* contains 17 residues, also has an amidated carboxyl terminus and contains two disulfide bridges. Recently described backbone-cyclic protegrin and tachyplesin analogues typically contain 18 residues and up to three disulfide bridges (J. P. Tam, C. Wu, J.-L. Yang, Eur. J. Biochem. 2000, 267, 3289-3300; J. P. Tam, Y.-A. Lu, J.-L. Yang, Biochemistry 2000, 39, 7159-7169; N. Sitaram, R. Nagaraij, Biochem. Biophys. Res. Comm. 2000, 267, 783-790).
*Cathelicidin,* a 37-residue linear helical-type cationic peptide, and analogues are currently under investigation as inhaled therapeutic agents for *cystic fibrosis(CF) lung disease* (L. Saiman, S. Tabibi, T. D. Starner, P. San Gabriel, P. L. Winokur, H. P. Jia, P. B. McGray, Jr., B. F. Tack, Antimicrob. Agents and Chemother. 2001, 45, 2838-2844; R. E. W. Hancock, R. Lehrer, Trends Biotechnol. 1998, 16, 82-88). Over 80% of CF patients become chronically infected with *pseudomonas aeruginosa* (C. A. Demko, P. J. Biard, P. B. Davies, J. Clin. Epidemiol. 1995, 48, 1041-1049; E. M. Kerem, R. Gold, H. Levinson, J. Pediatr. 1990, 116, 714-719).
In the compounds described below, a new strategy is introduced to stabilize β-hairpin conformations in backbone-cyclic cationic peptide mimetic exhibiting antimicrobial activity. This involves transplanting the cationic and hydrophobic hairpin sequence onto a template, whose function is to restrain the peptide loop backbone into a hairpin geometry. The rigidity of the hairpin may be further influenced by introducing a disulfide bridge. Template-bound hairpin mimetic peptides have been described in the literature (D, Obrecht, M. Altorfer, J. A. Robinson, Adv. Med Chem. 1999, 4, 1-68; J. A. Robinson, Syn. Lett. 2000, 4, 429-441), but such molecules have not previously been evaluated for development of antimicrobial peptides. However, the ability to generate β-hairpin peptidomimetics using combinatorial and parallel synthesis methods has now been established (L. Jiang, K. Moehle, B. Dhanapal, D. Obrecht, J. A. Robinson, Helv. Chim. Acta. 2000, 83, 3097-3112). In addition incorporation of designated peptoid structure elements into template-bound hairpin mimetics have not previously been evaluated for development of antimicrobial peptides.

These methods allow the synthesis and screening of large hairpin mimetic libraries, which in turn considerably facilitates structure-activity studies, and hence the discovery of new molecules with potent antimicrobial and low hemolytic activity to human red blood cells. Furthermore, the present strategy allows to synthesize β-hairpin peptidomimetics with novel selectivities towards different types of pathogens, e.g. towards various multi-drug resistant *pseudomonas* strains. β-Hairpin peptidomimetics obtained by the approach described here can be used amongst other applications, e.g. as broad spectrum antibiotics.
The β-hairpin peptidomimetics of the present invention are compounds of the general formula
wherein 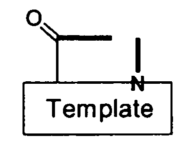 is a group of one of the formulae ^{D}Pro-^{L}Pro and ^{L}Pro-^{D}Pro
- R²⁰: is H; alkyl; alkenyl; or aryl-lower alkyl;
- R³³: is H; alkyl, alkenyl; -(CH₂)ₘ(CHR⁶¹)ₛOR⁵⁵; -(CH₂)ₘ(CHR⁶¹)ₛNR³⁴R⁶³;
-(CH₂)ₘ(CHR⁶¹)ₛOCONR⁷⁵R⁸²; -(CH₂)ₘ(CHR⁶¹)ₛNR²⁰CONR⁷⁸R⁸²;
-(CH₂)ₒ(CHR⁶¹)ₛCOR⁶⁴; -(CH₂)ₒ(CHR⁶¹)ₛ-CONR⁵⁸R⁵⁹, -(CH₂)ₒ(CHR⁶¹)ₛPO(OR⁶⁰)₂;
-(CH₂)ₒ(CHR⁶¹)ₛSO₂R⁶²; or -(CH₂)ₒ(CHR⁶¹)ₛC₆H₄R⁸;
- R³⁴: is H; lower alkyl; aryl, or aryl-lower alkyl;
- R³³ and R³⁴: taken together can form: -(CH₂)₂₋₆-; -(CH₂)₂O(CH₂)₂-; -(CH₂)₂S(CH₂)₂-; or -(CH₂)₂NR⁵⁷(CH₂)₂-;
- R³⁷: is H; F; Br; Cl; NO₂; CF₃; lower alkyl; -(CH₂)ₚ(CHR⁶¹)ₛOR⁵⁵; -(CH₂)ₚ(CHR⁶¹)ₛNR³³R³⁴;
-(CH₂)ₚ(CHR⁶¹)sOCONR³³R⁷⁵; -(CH₂)ₚ(CHR⁶¹)ₛNR²⁰CONR³³R⁸²;
-(CH₂)ₒ(CHR⁶¹)ₛCOOR⁵⁷; -(CH₂)ₒ(CHR⁶¹)ₛCONR⁵⁸R⁵⁹; -(CH₂)ₒ(CHR⁶¹)ₛPO(OR⁶⁰)₂;
-(CH₂)ₒ(CHR⁶¹)ₛSO₂R⁶²; or -(CH₂)ₒ(CHR⁶¹)ₛ C₆H₄R⁸;
- R⁵⁰: is H; lower alkyl; or aryl-lower alkyl;
- R⁵⁵: is H; lower alkyl; lower alkenyl; aryl-lower alkyl; -(CH₂)ₘ(CHR⁶¹)ₛOR⁵⁷;
-(CH₂)ₘ(CHR⁶¹)ₛNR³⁴R⁶³; -(CH₂)ₘ(CHR⁶¹)ₛOCONR⁷⁵R⁸²;
-(CH₂)ₘ(CHR⁶¹)ₛNR²⁰CONR⁷⁸R⁸²; -(CH₂)ₒ(CHR⁶¹)ₛ-COR⁶⁴;
-(CH₂)ₒ(CHR⁶¹)COOR⁵⁷; or
-(CH₂)ₒ(CHR⁶¹)ₛCONR⁵⁸R⁵⁹;
- R⁵⁶: is H; lower alkyl; lower alkenyl; aryl-lower alkyl; -(CH₂)ₘ(CHR⁶¹)ₛOR⁵⁷;
-(CH₂)ₘ(CHR⁶¹)ₛNR³⁴R⁶³; -(CH₂)ₘ(CHR⁶¹)ₛOCONR⁷⁵R⁸²;
-(CH₂)ₘ(CHR⁶¹)ₛNR²⁰CONR⁷⁸R⁸²; -(CH₂)ₒ(CHR⁶¹)ₛ-COR⁶⁴; or
-(CH₂)ₒ(CHR⁶¹)ₛCONR⁵⁸R⁵⁹;
- R⁵⁷: is H; lower alkyl; lower alkenyl; aryl lower alkyl; or heteroaryl lower alkyl;
- R⁵⁸: is H; lower alkyl; lower alkenyl; aryl; heteroaryl; aryl-lower alkyl; or heteroaryl-lower alkyl;
- R⁵⁹: is H; lower alkyl; lower alkenyl; aryl; heteroaryl; aryl-lower alkyl; or heteroaryl-lower alkyl; or
- R⁵⁸ and R⁵⁹: taken together can form: -(CH₂)₂₋₆-; -(CH₂)₂O(CH₂)₂-; -(CH₂)₂S(CH₂)₂-; or -(CH₂)₂NR⁵⁷(CH₂)₂-;
- R⁶⁰: is H; lower alkyl; lower alkenyl; aryl; or aryl-lower alkyl;
- R⁶¹: is alkyl; alkenyl; aryl; heteroaryl; aryl-lower alkyl; heteroaryl-lower alkyl; -(CH₂)ₘOR⁵⁵;
-(CH₂)ₘNR³³R³⁴; -(CH₂)ₘOCONR⁷⁵R⁸²; -(CH₂)ₘNR²⁰CONR⁷⁸R⁸²; -(CH₂)ₒCOOR³⁷;
-(CH2)ₒNR⁵⁸R⁵⁹; or -(CH₂)ₒPO(COR⁶⁰)₂;
- R⁶²: is lower alkyl; lower alkenyl; aryl, heteroaryl; or aryl-lower alkyl;
- R⁶³: is H; lower alkyl; lower alkenyl; aryl, heteroaryl; aryl-lower alkyl; heteroaryl-lower alkyl;
-COR⁶⁴; -COOR⁵⁷; -CONR⁵⁸R⁵⁹; -SO₂R⁶²; or -PO(OR⁶⁰)₂; or
- R³⁴ and R⁶³: taken together can form: -(CH₂)₂₋₆-; -(CH₂)₂O(CH₂)₂-; -(CH₂)₂S(CH₂)₂-; or -(CH₂)₂NR⁵⁷(CH₂)₂-;
- R⁶⁴: is H; lower alkyl; lower alkenyl; aryl; heteroaryl; aryl-lower alkyl; heteroaryl-lower alkyl;
-₍CH²)ₚ(CHR⁶¹)ₛOR⁶⁵; -(CH₂)ₚ(CHR⁶¹)ₛSR⁶⁶; or -(CH₂)ₚ(CHR⁶¹)ₛNR³⁴R⁶³;
-(CH₂)ₚ(CHR⁶¹)ₛOCONR⁷⁵R⁸²; -(CH₂)ₚ(CHR⁶¹)ₛNR²⁰CONR⁷⁸R⁸²
- R⁶⁵: is H; lower alkyl; lower alkenyl; aryl, aryl-lower alkyl; heteroaryl-lower alkyl; -COR⁵⁷; -COOR⁵⁷; or -CONR⁵⁸R⁵⁹;
- R⁶⁶: is H; lower alkyl; lower alkenyl; aryl; aryl-lower alkyl; heteroaryl-lower alkyl; or -CONR⁵⁸R⁵⁹;
- m: is 2-4; o is 0-4; p is 1-4; q is 0-2; r is 1 or 2; s is 0 or 1;
Z is a chain of 12 α-amino acid residues, the positions of said amino acid residues in said chain being counted starting from the N-terminal amino acid, whereby these amino acid residues are, depending on their position in the chains, Gly, or Pro, or of one of the types
- C:: -NR²⁰CH(R⁷²)CO-;
- D:: -NR²⁰CH(R⁷³)CO-;
- E:: -NR²⁰CH(R⁷⁴)CO-;
- F:: -NR²⁰CH(R⁸⁴)CO-; and
- H:: -NR²⁰-CH(CO-)-(CH₂)₄₋₇-CH(CO-)-NR²⁰-;
-NR²⁰-CH(CO-)-(CH₂)ₚSS(CH₂)ₚ-CH(CO-)-NR²⁰-;
-NR²⁰-CH(CO-)-(-(CH₂)ₚNR²⁰CO(CH₂)ₚ-CH(CO-)-NR²⁰-; and
-NR²⁰-CH(CO-)-(-(CH₂)ₚNR²⁰CONR²⁰(CH₂)ₚ-CH(CO-)-NR²⁰-;
- I:: -NR⁸⁶CH₂CO-;
- K:: -NR⁸⁷CH₂CO-;
- R⁷²: is H, lower alkyl; lower alkenyl; -(CH₂)ₚ(CHR⁶¹)ₛOR⁸⁵; or -(CH₂)ₚ(CHR⁶¹)ₛSR⁸⁵;
- R⁷³: is -(CH₂)ₒR⁷⁷; -(CH₂)ᵣO(CH₂)ₒR⁷⁷; -(CH₂)ᵣS(CH₂)ₒR⁷⁷; or -(CH₂)ᵣNR²⁰(CH₂)ₒR⁷⁷;
- R⁷⁴: is -(CH₂)ₚNR⁷⁸R⁷⁹; -(CH₂)ₚNR⁷⁷R⁸⁰; -(CH₂)ₚC(=NR⁸⁰)NR⁷⁸R⁷⁹;
-(CH₂)ₚC(=NOR⁵⁰)NR⁷⁸R⁷⁹;
-(CH₂)ₚC(=NNR⁷⁸R⁷⁹)NR⁷⁸R⁷⁹; -(CH₂)ₚNR⁸⁰C(=NR⁸⁰)NR⁷⁸R⁷⁹;
-(CH₂)ₚN=C(NR⁷⁸R⁸⁰)NR⁷⁹R⁸⁰;-(CH₂)ₚC₆H₄NR⁷⁸R⁷⁹; -(CH₂)ₚC₆H₄NR⁷⁷R⁸⁰;
-(CH₂)ₚC₆H₄(=NR⁸⁰)NR⁷⁸R⁷⁹; -(CH₂)ₚC₆H₄C(=NOR⁵⁰)NR⁷⁸R⁷⁹;
-(CH₂)ₚC₆H₄C(=NNR⁷⁸R⁷⁹)NR⁷⁸R⁷⁹; -(CH₂)ₚC₆H₄NR⁸⁰C(=NR⁸⁰)NR⁷⁸R7⁹;
-(CH₂)ₚC₆H₄N=C(NR⁷⁸R⁸⁰)NR⁷⁹R⁸⁰; -(CH₂)ᵣO(CH₂)ₘNR⁷⁸R⁷⁹; -(CH₂)ᵣO(CH₂)ₘNR⁷⁷R⁸⁰;
-(CH₂)ᵣO(CH₂)ₚC(=NR⁸⁰)NR⁷⁸R⁷⁹; -(CH₂)ᵣO(CH₂)ₚC(=NOR⁵⁰)NR⁷⁸R⁷⁹;
-(CH₂)ᵣO(CH₂)ₚC(=NNR⁷⁸R⁷⁹)NR⁷⁸R⁷⁹; -(CH₂)ᵣO(CH₂)ₘNR⁸⁰C(=NR⁸⁰)NR⁷⁸R⁷⁹;
-(CH₂)ᵣO(CH₂)ₘN=C(NR⁷⁸R⁸⁰)NR⁷⁹R⁸⁰; -(CH₂)ᵣO(CH₂)ₚC₆H₄CNR⁷⁸R⁷⁹;
-(CH₂)ᵣO(CH₂)ₚC₆H₄C(=NR⁸⁰)NR⁷⁸R⁷⁹; -(CH₂)ᵣO(CH₂)ₚC₆H₄C(=NOR⁵⁰)NR⁷⁸R⁷⁹;
-(CH₂)ᵣO(CH₂)ₚC₆H₄C(=NNR⁷⁸R⁷⁹)NR⁷⁸R⁷⁹;
-(CH₂)ᵣO(CH₂)ₚC₆H₄NR⁸⁰C(=NR⁸⁰)NR⁷⁸R⁷⁹; -(CH₂)ᵣS(CH₂)ₘNR⁷⁸R⁷⁹;
-(CH₂)ᵣS(CH₂)ₘNR⁷⁷R⁸⁰;-(CH₂)ᵣS(CH₂)ₚC(=NR⁸⁰)NR⁷⁸R⁷⁹;
-(CH₂)ᵣS(CH₂)ₚC(=NOR⁵⁰)NR⁷⁸R⁷⁹; -(CH₂)ᵣS(CH₂)ₚC(=NNR⁷⁸R⁷⁹)NR⁷⁸R⁷⁹;
-(CH₂)ᵣS(CH₂)ₘNR⁸⁰C(=NR⁸⁰)NR⁷⁸R⁷⁹; -(CH₂)ᵣS(CH₂)ₘN=C(NR⁷⁸R⁸⁰)NR⁷⁹R⁸⁰;
-(CH₂)ᵣS(CH₂)ₚC₆H₄CNR⁷⁸R⁷⁹; -(CH₂)ᵣS(CH₂)ₚC₆H₄C(=NR⁸⁰)NR⁷⁸R⁷⁹
-(CH₂)ᵣS(CH₂)ₚC₆H₄C(=NOR⁵⁰)NR⁷⁸R⁷⁹; -(CH₂)ᵣS(CH₂)ₚC₆H₄C(=NNR⁷⁸R⁷⁹)NR⁷⁸R⁷⁹;
-(CH₂)ᵣS(CH₂)ₚC₆H₄NR⁸⁰C(=NR⁸⁰)NR⁷⁸R⁷⁹; -(CH₂)ₚNR⁸⁰COR⁶⁴; -(CH₂)ₚNR⁸⁰COR⁷⁷;
-(CH₂)ₚNR⁸⁰CONR⁷⁸R⁷⁹; or -(CH₂)ₚC₆H₄NR⁸⁰CONR⁷⁸R⁷⁹;
- R⁷⁵: is lower alkyl; lower alkenyl; or aryl-lower alkyl; or
- R³³ and R⁷⁵: taken together can form: -(CH₂)₂₋₆-; -(CH₂)₂O(CH₂)₂-; -(CH₂)₂S(CH₂)₂-; or -(CH₂)₂NR⁵⁷(CH₂)₂-; or
- R⁷⁵ and R⁸²: taken together can form: -(CH₂)₂₋₆-; -(CH₂)₂O(CH₂)₂-; -(CH₂)₂S(CH₂)₂-; or -(CH₂)₂NR⁵⁷(CH₂)₂-;
- R⁷⁷: is R⁸⁸; or a heteroaryl group of one of the formulae 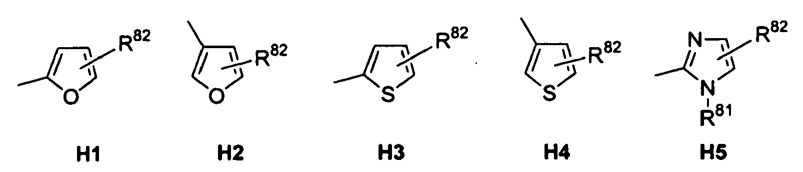 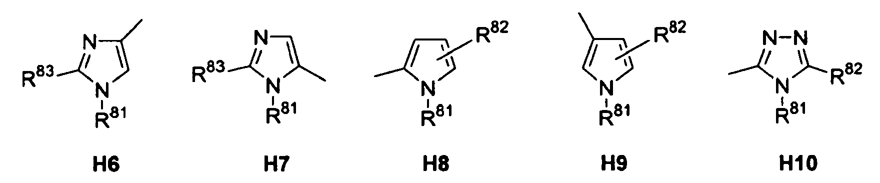 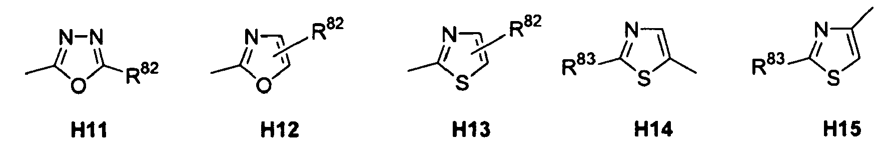 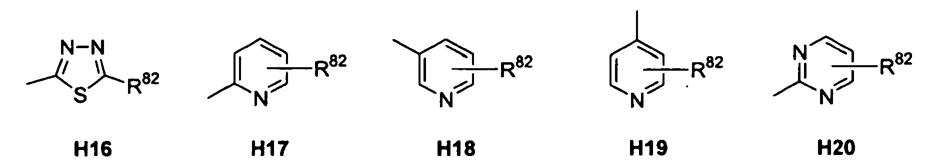 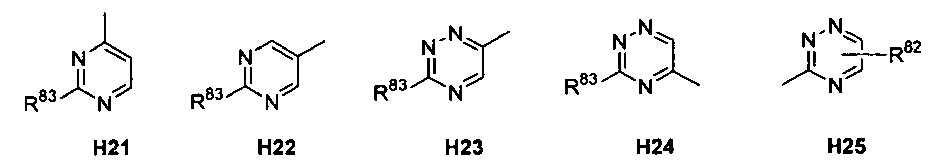 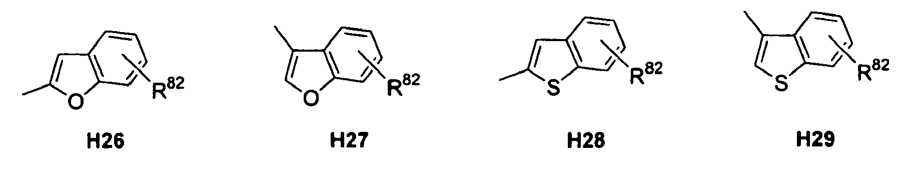 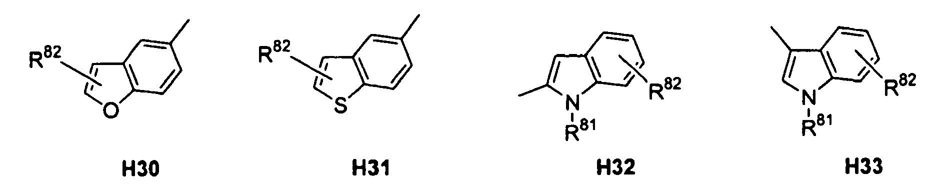 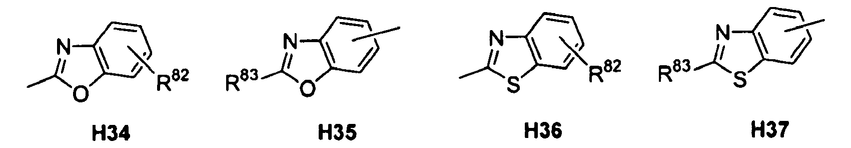 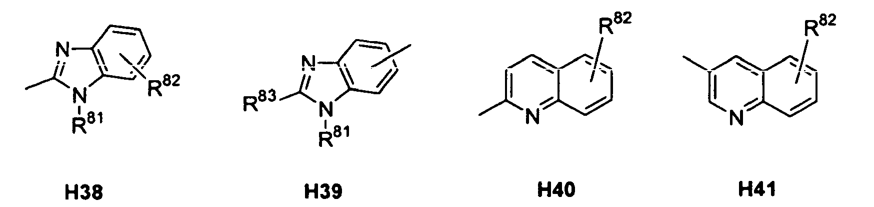 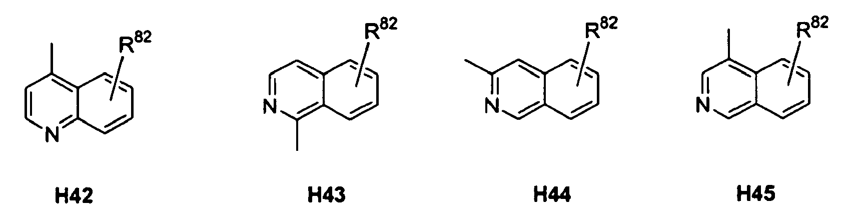 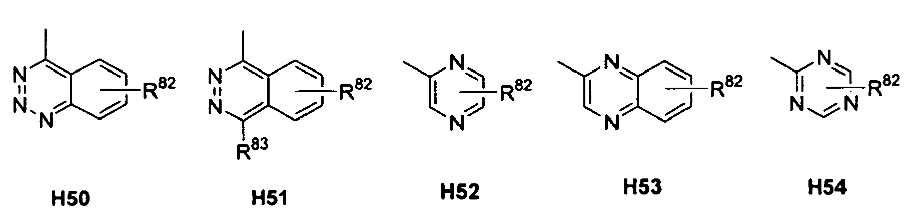 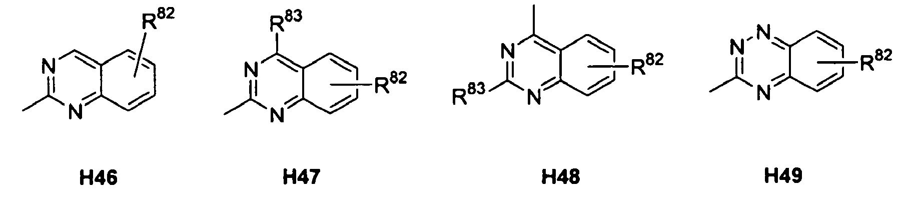
- R⁷⁸: is H; lower alkyl; aryl; or aryl-lower alkyl;
- R⁷⁸ and R⁸²: taken together can form: -(CH₂)₂₋₆-; -(CH₂)₂O(CH₂)₂-; -(CH₂)₂S(CH₂)₂-; or -(CH₂)₂NR⁵⁷(CH₂)₂-;
- R⁷⁹: is H; lower alkyl; aryl; or aryl-lower alkyl; or
- R⁷⁸ and R⁷⁹,: taken together, can be -(CH₂)₂₋₇-; -(CH₂)₂O(CH₂)₂-; or -(CH₂)₂NR⁵⁷(CH₂)₂-;
- R⁸⁰: is H; or lower alkyl;
- R⁸¹: is H; lower alkyl; or aryl-lower alkyl;
- R⁸²: is H; lower alkyl; aryl; heteroaryl; or aryl-lower alkyl;
- R³³ and R⁸²: taken together can form: -(CH₂)₂₋₆-; -(CH₂)₂O(CH₂)₂-; -(CH₂)₂S(CH₂)₂-; or -(CH₂)₂NR⁵⁷(CH₂)₂-;
- R⁸³: is H; lower alkyl; aryl; or -NR⁷⁸R⁷⁹;
- R⁸⁴: is -(CH₂)ₘ(CHR⁶¹)ₛOR⁷⁸; -(CH₂)ₘ(CHR⁶¹)ₛSR⁷⁸; -(CH₂)ₚCONR⁷⁸R⁷⁹; -(CH₂)ₚNR⁸⁰CONR⁷⁸R⁷⁹;
-(CH₂)ₚC₆H₄CONR⁷⁸R⁷⁹; or -(CH₂)ₚC₆H₄NR⁸⁰CONR⁷⁸R⁷⁹;
- R⁸⁵: is lower alkyl; or lower alkenyl;
- R⁸⁶: is R⁷⁴; -[(CH₂)ᵤ-X]ₜ-(CH₂)ᵥNR⁷⁸R⁷⁹; -[(CH₂)ᵤ₋X]ₜ-(CH₂)ᵥ-C(=NR⁸⁰)NR⁷⁸R⁷⁹; X is -O-, -NR²⁰-, -S-,
-OCOO-, u is 1-3, t is 1-6, v is 1-3;
- R⁸⁷: is R⁸⁴; -[(CH₂)ᵤ-X]ₜ-(CH₂)ᵥOR⁷⁸, -[(CH₂)ᵤX]ₜ-(CH₂)ᵥ-CONR⁷⁸R⁷⁹, -[(CH₂)ᵤ-X]ₜ-(CH₂)ᵥ NR⁸⁰CONR⁷⁸R⁷⁹, -[(CH₂)ᵤ-X]ₜ-(CH₂)ᵥ SR⁷⁸; X is -O-, -NR²⁰-, -S-, -OCOO-, u is 1-3, t is 1-6, v is 1-3;
- R⁸⁸: is phenyl, p-hydrxyphenyl, 2-naphthyl, 1-naphthyl, 4-chlorophenyl, 3-chlorophenyl, 2-chlorophenyl, 3,4-dichlorophenyl, 4-fluorophenyl, 3-fluorophenyl, 2-fluorophenyl, p-benzyloxyphenyl, p-biphenyl or p-benzoylphenyl.
with the proviso that in said chain of 12 α-amino acid residues Z the amino acid residues in positions 1 to 12 are:
- P1: of type C or of type D or of type E or of type F, or the residue is Pro;
- P2: of type E or of type D;
- P3: of type C, or the residue is Pro;
- P4: of type E or of type F or of type I or of type K;
- P5: of type E or of type D or or of type C or of type I or of type K or of type F, or the residue is Gly or Pro;
- P6: of type E or of type F, or of type I or of type K or of type D, or the residue is Gly;
- P7: of type E or of type F or of type I or of type C;
- P8: of type D or of type C, or the residue is Pro;
- P9: of type E or of type D or of type F ;
- P10: of type D or of type C or the residue is Pro;
- P11: of type E or of type D or of type C; and
- P12: of type C or of type D or of type E or of type F, or the residue is Pro; or
- P4 and P9 and/or P2 and P11, taken together, can form a group of type H; and at P6 and P7 also D-isomers being possible;
with the further proviso that said chain of 12 α-amino acid residues contains at least one residue of type I or of type K;
and pharmaceutically acceptable salts thereof.

In accordance with the present invention these β-hairpin peptidomimetics can be prepared by a process which comprises
(a) coupling an appropriately functionalized solid support with an appropriately N-protected derivative of that amino acid which in the desired end-product is in position 5, 6 or 7, any functional group which may be present in said N-protected amino acid derivative being likewise appropriately protected;
(b) removing the N-protecting group from the product thus obtained;
(c) coupling the product thus obtained with an appropriately N-protected derivative of that amino acid which in the desired end-product is one position nearer the N-terminal amino acid residue, any functional group which may be present in said N-protected amino acid derivative being likewise appropriately protected;
(d) removing the N-protecting group from the product thus obtained;
(e) repeating steps (c) and (d) until the N-terminal amino acid residue has been introduced;
(f) coupling the product thus obtained with
   (fa) an appropiatly N-protected derivative of ^{L}Pro or ^{D}Pro
   (fb) removing the N-protecting group from the product thus obtained; anf
   (fc) coupling the product thus obtained with an appropiatly N-protected derivative of ^{D}Pro and, respectively ^{L}Pro;
(g) removing the N-protecting group from the product obtained in step (fc);
(h) coupling the product thus obtained with an appropriately N-protected derivative of that amino acid which in the desired end-product is in position 12, any functional group which may be present in said N-protected amino acid derivative being likewise appropriately protected;
(i) removing the N-protecting group from the product thus obtained;
(j) coupling the product thus obtained with an appropriately N-protected derivative of that amino acid which in the desired end-product is one position farther away from position 12, any functional group which may be present in said N-protected amino acid derivative being likewise appropriately protected;
(k) removing the N-protecting group from the product thus obtained;
(l) repeating steps (j) and (k) until all amino acid residues have been introduced;
(m) if desired, selectively deprotecting one or several protected functional group(s) present in the molecule and appropriately substituting the reactive group(s) thus liberated;
(o) detaching the product thus obtained from the solid support;
(p) cyclizing the product cleaved from the solid support;
(q) if desired, forming one or two interstrand linkage(s) between side-chains of appropriate amino acid residues at opposite positions of the β-strand region;
(r) removing any protecting groups present on functional groups of any members of the chain of amino acid residues and, if desired, any protecting group(s) which may in addition be present in the molecule;
(s) if desired guanidinylating any side-chain amino group present in the chain of amino acid residues; and
(t) if desired, converting the product thus obtained into a pharmaceutically acceptable salt or converting a pharmaceutically acceptable, or unacceptable, salt thus obtained into the corresponding free compound of formula I or into a different, pharmaceutically acceptable, salt.

Alternatively, the peptidomimetics of the present invention can be prepared by
(a') coupling an appropriately functionalized solid support
   (a'a) coupling said appropriately functionalized solid support with an appropriately N-protected derivative of ^{L}Pro or ^{D}Pro
   (a'b) removing the N-protecting group from the product thus obtained; and
   (a'c) coupling the product thus obtained with an appropriately N-protected derivative of ^{D}Pro and, respectively, ^{L}Pro;
(b') removing the N-protecting group from the product obtained in step (a'c);
(c') coupling the product thus obtained with an appropriately N-protected derivative of that amino acid which in the desired end-product is one position nearer the N-terminal amino acid residue, any functional group which may be present in said N-protected amino acid derivative being likewise appropriately protected;
(d') removing the N-protecting group from the product thus obtained;
(e') coupling the product thus obtained with an appropriately N-protected derivative of that amino acid which in the desired end-product is one position farther away from position 12, any functional group which may be present in said N-protected amino acid derivative being likewise appropriately protected;
(f) removing the N-protecting group from the product thus obtained;
(g') repeating steps (e') and (f) until all amino acid residues have been introduced;
(h') if desired, selectively deprotecting one or several protected functional group(s) present in the molecule and appropriately substituting the reactive group(s) thus liberated;
(i') detaching the product thus obtained from the solid support;
(j') cyclizing the product cleaved from the solid support;
(k') if desired forming one or two interstrand linkage(s) between side-chains of appropriate amino acid residues at opposite positions of the β-strand region;
(l') removing any protecting groups present on functional groups of any members of the chain of amino acid residues and, if desired, any protecting group(s) which may in addition be present in the molecule;
(m') if desired guanidinylating any side-chain amino group present in the chain of amino acid residues; and
(n') if desired, converting the product thus obtained into a pharmaceutically acceptable salt or converting a pharmaceutically acceptable, or unacceptable, salt thus obtained into the corresponding free compound of formula I or into a different, pharmaceutically acceptable, salt.

Introducing an amino acid residue of type I or K can, alternatively, be effected by coupling with a leaving group-containing acetylating agent, such as bromo, chloro or iodo acetic acid, followed by nucleophilic displacement with an amine of the formula H₂NR⁸⁷ and, respectively, H₂NR⁸⁷ which, if necessary, is appropriately protected.

The peptidomimetics of the present invention can also be enantiomers of the compounds of formula I. These enantiomers can be prepared by a modification of the above processes in which enantiomers of all chiral starting materials are used.
As used in this description, the term "alkyl", taken alone or in combinations, designates saturated, straight-chain or branched hydrocarbon radicals having up to 24, preferably up to 12, carbon atoms. Similarly, the term "alkenyl" designates straight chain or branched hydrocarbon radicals having up to 24, preferably up to 12, carbon atoms and containing at least one or, depending on the chain length, up to four olefinic double bonds. The term "lower" designates radicals and compounds having up to 6 carbon atoms. Thus, for example, the term "lower alkyl" designates saturated, straight-chain or branched hydrocarbon radicals having up to 6 carbon atoms, such as methyl, ethyl, n-propyl, isopropyl, n-butyl, sec.-butyl, isobutyl, tert.-butyl and the like.
The term "aryl" designates aromatic carbocyclic hydrocarbon radicals containing one or two six-membered rings, such as phenyl or naphthyl, which may be substituted by up to three substituents such as Br, Cl, F, CF₃, NO₂ OH, NH₂, lower alkyl or lower alkenyl. The term "heteroaryl" designates aromatic heterocyclic radicals containing one or two five- and/or six-membered rings, at least one of them containing up to three heteroatoms selected from the group consisting of O, S and N and said ring(s) being optionally substituted; representative examples of such optionally substituted heteroaryl radicals are indicated hereinabove in connection with the definition of R⁷⁷.

The templates constitute building blocks which have an N-terminus and a C-terminus oriented in space in such a way that the distance between those two groups may lie between 4.0-5.5A. A peptide chain **Z** is linked to the C-terminus and the N-terminus of the template via the corresponding N- and C-termini so that the template and the chain form a cyclic structure such as that depicted in formula **I**. In a case as here where the distance between the N- and C- termini of the template lies between 4.0-5.5A the template will induce the H-bond network necessary for the formation of a β-hairpin conformation in the peptide chain Z. Thus template and peptide chain form a β*-hairpin mimetic.*

The β-hairpin conformation is highly relevant for the antibiotic activity of the β-hairpin mimetics of the present invention. The β-hairpin stabilizing conformational properties of the templates (a) through (p) play a key role not only for antibiotic activity but also for the synthesis process defined hereinabove, as incorporation of the templates near the middle or at the beginning of the linear protected peptide precursors enhance cyclization yields.

The peptidic chain Z of the β-hairpin mimetics described herein are generally defined in terms of amino acid residues belonging to one of the following groups:

| | | |
|---|---|---|
| - | Group C | -NR²⁰CH(R⁷²)CO-; "hydrophobic: small to medium-sized" |
| | Group D | -NR²⁰CH(R⁷³)CO-; "hydrophobic: large aromatic or heteroaromatic" |
| | Group E | -NR²⁰CH(R⁷⁴)CO-; "polar-cationic" and "urea-derived" |
| - | Group F | -NR²⁰CH(R⁸⁴)CO-; "polar-non-charged" |
| | Group H | -NR²⁰-CH(CO-)-(CH₂)₄₋₇-CH(CO-)-NR²⁰-; |
| | | -NR²⁰-CH(CO-)-(CH₂)ₚSS(CH₂)ₚ-CH(CO-)-NR²⁰-; |
| | | -NR²⁰-CH(CO-)-(-(CH₂)ₚNR²⁰CO(CH₂)ₚ-CH(CO-)-NR²⁰-; and |
| | | -NR²⁰-CH(CO-)-(-(CH₂)ₚNR²⁰CONR²⁰(CH₂)ₚ-CH(CO-)-NR²⁰-; |
| | | "interstrand linkage" |
| | Group I | -NR⁸⁶CH₂CO-; "polar-cationic" |
| | Group K | -NR⁸⁷CH₂CO-; "polar non charged" |

Furthermore Pro can be an amino acid residue in chain **Z,** too, with the exception of positions where interstrand linkages **(H)** are possible.

**Group C** comprises amino acid residues with small to medium-sized *hydrophobic* side chain groups according to the general definition for substituent R⁷². A hydrophobic residue refers to an amino acid side chain that is uncharged at physiological pH and that is repelled by aqueous solution. Furthermore these side chains generally do *not* contain hydrogen bond donor groups, such as (but not limited to) primary and secondary amides, primary and secondary amines and the corresponding protonated salts thereof, thiols, alcohols, phosphonates, phosphates, ureas or thioureas. However, they may contain hydrogen bond acceptor groups such as ethers, thioethers, esters, tertiary amides, alkyl- or aryl phosphonates and phosphates or tertiary amines. Genetically encoded small-to-medium-sized amino acids include alanine, isoleucine, leucine, methionine and valine.

**Group D** comprises amino acid residues with *aromatic* and *heteroaromatic* side chain groups according to the general definition for substituent R⁷³. An aromatic amino acid residue refers to a hydrophobic amino acid having a side chain containing at least one ring having a conjugated π-electron system (aromatic group). In addition they may contain hydrogen bond donor groups such as (but not limited to) primary and secondary amides, primary and secondary amines and the corresponding protonated salts thereof, thiols, alcohols, phosphonates, phosphates, ureas or thioureas, and hydrogen bond acceptor groups such as (but not limited to) ethers, thioethers, esters, tetriary amides, alkyl- or aryl phosphonates -and phosphates or tertiary amines. Genetically encoded aromatic amino acids include phenylalanine and tyrosine.

A heteroaromatic amino acid residue refers to a hydrophobic amino acid having a side chain containing at least one ring having a conjugated π-system incorporating at least one heteroatom such as (but not limited to) O, S and N according to the general definition for substituent R⁷⁷. In addition such residues may contain hydrogen bond donor groups such as (but not limited to) primary and secondary amides, primary and secondary amines and the corresponding protonated salts thereof, thiols, alcohols, phosphonates, phosphates, ureas or thioureas, and hydrogen bond acceptor groups such as (but not limited to) ethers, thioethers, esters, tetriary amides, alkyl- or aryl phosphonates -and phosphates or tertiary amines. Genetically encoded heteroaromatic amino acids include tryptophan and histidine.

**Group E** comprises amino acids containing side chains with polar-cationic, acylamino- and urea-derived residues according to the general definition for substituent R⁷⁴. Polar-cationic refers to a basic side chain which is protonated at physiological pH. Genetically encoded polar-cationic amino acids include arginine, lysine and histidine. Citrulline is an example for an urea derived amino acid residue.

**Group F** comprises amino acids containing side chains with polar-non-charged residues according to the general definition for substituent R⁸⁴. A polar-non-charged residue refers to a hydrophilic side chain that is uncharged at physiological pH, but that is not repelled by aqueous solutions. Such side chains typically contain hydrogen bond donor groups such as (but not limited to) primary and secondary amides, primary and secondary amines, thiols, alcohols, phosphonates, phosphates, ureas or thioureas. These groups can form hydrogen bond networks with water molecules. In addition they may also contain hydrogen bond acceptor groups such as (but not limited to) ethers, thioethers, esters, tetriary amides, alkyl- or aryl phosphonates -and phosphates or tertiary amines. Genetically encoded polar-non-charged amino acids include asparagine, cysteine, glutamine, serine and threonine.

**Group H** comprises side chains of preferably (L)-amino acids at opposite positions of the β-strand region that can form an interstrand linkage. The most widely known linkage is the disulfide bridge formed by cysteines and homo-cysteines positioned at opposite positions of the β-strand. Various methods are known to form disulfide linkages including those described by: J. P. Tam et al. Synthesis 1979, 955-957; Stewart et al., Solid Phase Peptide Synthesis, 2d Ed., Pierce Chemical Company, III., 1984**;** Ahmed et al. J. Biol. Chem. 1975, 250, 8477-8482 ; and Pennington et al., Peptides, pages 164-166, Giralt and Andreu, Eds., ESCOM Leiden, The Netherlands, 1990**.** Most advantageously, for the scope of the present invention, disulfide linkages can be prepared using acetamidomethyl (Acm)- protective groups for cysteine. A well established interstrand linkage consists in linking ornithines and lysines, respectively, with glutamic and aspartic acid residues located at opposite β-strand positions by means of an amide bond formation. Preferred protective groups for the side chain amino-groups of ornithine and lysine are allyloxycarbonyl (Alloc) and allylesters for aspartic and glutamic acid. Finally, interstrand linkages can also be established by linking the amino groups of lysine and ornithine located at opposite β-strand positions with reagents such as N,N-carbonylimidazole to form cyclic ureas.

**Group I** comprises glycine having the amino group substituted by chains containing polar-cationic residues according to the general definition for substituent R⁸⁶. Polar-cationic refers to a basic side chain which is protonated at physiological pH.

**Group K** comprises glycine having the amino group substituted by chains containing polar-non-charged residues according to the general definition for substituent R⁸⁷. A polar-non-charged residue refers to a hydrophilic side chain that is uncharged at physiological pH, but that is not repelled by aqueous solutions. Such side chains typically contain hydrogen bond donor groups such as (but not limited to) primary and secondary amides, thiols, alcohols, or ureas. These groups can form hydrogen bond networks with water molecules. In addition they may also contain hydrogen bond acceptor groups such as (but not limited to) ether, thioether, ester or tertiary amino groups.

As mentioned earlier, positions for interstrand linkages are positions P4 and P9 and/or P2 and P11 taken together.
Such interstrand linkages are known to stabilize the β-hairpin conformations and thus constitute an important structural element for the design of β-hairpin mimetics.

Preferred amino acid residues (other than of types I and K) in chain Z are those derived from natural α-amino acids. Hereinafter follows a list of amino acids which, or the residues of which, are suitable for the purposes of the present invention, the abbreviations corresponding to generally adopted usual practice:

| three letter code | | one letter code |
|---|---|---|
| Ala | L-Alanine | A |
| Arg | L-Arginine | R |
| Asn | L-Asparagine | N |
| Asp | L-Aspartic acid | D |
| Cys | L-Cysteine | C |
| Glu | L-Glutamic acid | E |
| Gln | L-Glutamine | Q |
| Gly | Glycine | G |
| His | L-Histidine | H |
| Ile | L-Isoleucine | I |
| Leu | L-Leucine | L |
| Lys | L-Lysine | K |
| Met | L-Methionine | M |
| Phe | L-Phenylalanine | F |
| Pro | L-Proline | P |
| ^{D}Pro | D-Proline | ^{D}P |
| Ser | L-Serine | S |
| Thr | L-Threonine | T |
| Trp | L-Tryptophan | W |
| Tyr | L-Tyrosine | Y |
| Val | L-Valine | V |

Other α-amino acids which, or the residues of which, are suitable for the purposes of the present invention include:

| | |
|---|---|
| Cit | L-Citrulline |
| Orn | L-Ornithine |
| tBuA | L-t-Butylalanine |
| Sar | Sarcosine |
| Pen | L-Penicillamine |
| t-BuG | L-tert.-Butylglycine |
| 4AmPhe | L-para-Aminophenylalanine |
| 3AmPhe | L-meta-Aminophenylalanine |
| 2AmPhe | L-ortho-Aminophenylalanine |
| Phe(mC(NH₂)=NH) | L-meta-Amidinophenylalanine |
| Phe(pC(NH₂)=NH) | L-para-Amidinophenylalanine |
| Phe(mNHC (NH₂)=NH) | L-meta-Guanidinophenylalanine |
| Phe(pNHC (NH₂)=NH) | L-para-Guanidinophenylalanine |
| Phg | L-Phenylglycine |
| Cha | L-Cyclohexylalanine |
| C₄al | L-3-Cyclobutylalanine |
| C₅al | L-3-Cyclopentylalanine |
| Nle | L-Norleucine |
| 2-Nal | L-2-Naphthylalanine |
| 1-Nal | L-1-Naphthylalanine |
| 4Cl-Phe | L-4-Chlorophenylalanine |
| 3Cl-Phe | L-3-Chlorophenylalanine |
| 2Cl-Phe | L-2-Chlorophenylalanine |
| 3,4Cl₂-Phe | L-3,4-Dichlorophenylalanine |
| 4F-Phe | L-4-Fluorophenylalanine |
| 3F-Phe | L-3-Fluorophenylalanine |
| 2F-Phe | L-2-Fluorophenylalanine |
| Tic | 1,2,3,4-Tetrahydroisoquinoline-3-carboxylic acid |
| Thi | L-β-2-Thienylalanine |
| Tza | L-2-Thiazolylalanine |
| Mso | L-Methionine sulfoxide |
| AcLys | N-Acetyllysine |
| Dpr | 2,3-Diaminopropionic acid |
| A₂Bu | 2,4-Diaminobutyric acid |
| Dbu | (S)-2,3-Diaminobutyric acid |
| Abu | γ-Aminobutyric acid (GABA) |
| Aha | ε-Aminohexanoic acid |
| Aib | α-Aminoisobutyric acid |
| Y(Bzl) | L-O-Benzyltyrosine |
| Bip | L-(4-phenyl)phenylalanine |
| S(Bzl) | L-O-Benzylserine |
| T(Bzl) | L-O-Benzylthreonine |
| hCha | L-Homo-cyclohexylalanine |
| hCys | L-Homo-cysteine |
| hSer | L-Homo-serine |
| hArg | L-Homo-arginine |
| hPhe | L-Homo-phenylalanine |
| Bpa | L-4-Benzoylphenylalanine |
| 4-AmPyrrl | (2S,4S)-4-Amino-pyrrolidine-L-carboxylic acid |
| 4-AmPyrr2 | (2S,4R)-4-Amino-pyrrolidine-L-carboxylic acid |
| 4-PhePyrrl | (2S,5R)-4-Phenyl-pyrrolidine-L-carboxylic acid |
| 4-PhePyrr2 | (2S,5S)-4-Phenyl-pyrrolidine-L-carboxylic acid |
| 5-PhePyrrl | (2S,5R)-5-Phenyl-pyrrolidine-L-carboxylic acid |
| 5-PhePyrr2 | (2S,SS)-5-Phenyl-pyrrolidine-L-carboxylic acid |
| Pro(4-OH) 1 | (4S)-L-Hydroxyproline |
| Pro(4-OH)2 | (4R)-L-Hydroxyproline |
| Pip | L-Pipecolic acid |
| ^{D}Pip | D-Pipecolic acid |
| OctG | L-Octylglycine |
| MePhe | L-N-Methylphenylalanine |
| MeNle | L-N-Methylnorleucine |
| MeAla | L-N-Methylalanine |
| Melle | L-N-Methylisoleucine |
| MeVal | L-N-Methylvaline |
| MeLeu | L-N-Methylleucine |
| BnG | N-Benzylglycine |
| (4-OH)BnG | N-4-Hydroxy-benzylglycine |
| IaG | N-Isoamylglycine |
| IbG | N-Isobutlyglycine |
| (EA)G | N-(2-Aminoethyl)glycine |
| (PrA)G | N-(3-Amino-n-propyl)glycine |
| (BA)G | N-(4-Amino-n-butyl)glycine |
| (PeA)G | N-(5-Amino-n-pentyl)glycine |
| (EGU)G | N-(2-Guanidinoethyl)glycine |
| (PrGU)G | N-(3-Guanidino-n-propyl)glycine |
| (BGU)G | N-(4-Guanidino-n-butyl)glycine |
| (PeGU)G | N-(5-Guanidino-n-pentyl)glycine |
| (PEG₃-NH₂)G | N-[(CH₂)₃O-(CH₂-CH₂O)₂-(CH₂)₃-NH₂]glycine |
| (Et-CONH₂)G | N-(2-Carbamoylethyl)glycine |
| (Et-OH)G | N-(2-Hydroxyethyl)glycine |
| (CH₂-CONH₂)G | N-(Carbamoylmethyl)glycine |
| (n-Pr-NHCONH₂)G | N-(3-Ureyl-n-propyl)glycine |
| (Et-SH)G | N-(2-Mercaptoethyl)glycine |

Particularly preferred residues for **group C** are:

| | |
|---|---|
| Ala | L-Alanine |
| Ile | L-Isoleucine |
| Leu | L-Leucine |
| Met | L-Methionine |
| Val | L-Valine |
| tBuA | L-t-Butylalanine |
| t-BuG | L-tert.-Butylglycine |
| Cha | L-Cyclohexylalanine |
| C₄al | L-3-Cyclobutylalanine |
| C₅al | L-3-Cyclopentylalanine |
| Nle | L-Norleucine |
| hCha | L-Homo-cyclohexylalanine |
| OctG | L-Octylglycine |
| MePhe | L-N-Methylphenylalanine |
| MeNle | L-N-Methylnorleucine |
| MeAla | L-N-Methylalanine |
| Melle | L-N-Methylisoleucine |
| MeVal | L-N-Methylvaline |
| MeLeu | L-N-Methylleucine |
| BnG | N-Benzylglycine |
| (4-OH)BnG | N-4-Hydroxy-benzylglycine |
| IaG | N-Isoamylglycine |
| IbG | N-Isobutlyglycine |

Particularly preferred residues for **group D** are:

| | |
|---|---|
| His | L-Histidine |
| Phe | L-Phenylalanine |
| Trp | L-Tryptophan |
| Tyr | L-Tyrosine |
| Phg | L-Phenylglycine |
| 2-Nal | L-2-Naphthylalanine |
| 1-Nal | L-1-Naphthylalanine |
| 4Cl-Phe | L-4-Chlorophenylalanine |
| 3Cl-Phe | L-3-Chlorophenylalanine |
| 2Cl-Phe | L-2-Chlorophenylalanine |
| 3,4Cl₂-Phe | L-3,4-Dichlorophenylalanine |
| 4F-Phe | L-4-Fluorophenylalanine |
| 3F-Phe | L-3-Fluorophenylalanine |
| 2F-Phe | L-2-Fluorophenylalanine |
| Thi | L-β-2-Thienylalanine |
| Tza | L-2-Thiazolylalanine |
| Y(Bzl) | L-O-Benzyltyrosine |
| Bip | L-Biphenylalanine |
| S(Bzl) | L-O-Benzylserine |
| T(Bzl) | L-O-Benzylthreonine |
| hPhe | L-Homo-phenylalanine |
| Bpa | L-4-Benzoylphenylalanine |

Particularly preferred residues for **group E** are

| | |
|---|---|
| Arg | L-Arginine |
| Lys | L-Lysine |
| Orn | L-Omithine |
| Dpr | L-2,3-Diaminopropionic acid |
| A₂Bu | L-2,4-Diaminobutyric acid |
| Dbu | (S)-2,3-Diaminobutyric acid |
| Phe(pNH₂) | L-para-Aminophenylalanine |
| Phe(mNH₂) | L-meta-Aminophenylalanine |
| Phe(oNH₂) | L-ortho-Aminophenylalanine |
| hArg | L-Homo-arginine |
| Phe(mC(NH₂)=NH) | L-meta-Amidinophenylalanine |
| Phe(pC(NH₂)=NH) | L-para-Amidinophenylalanine |
| Phe(mNHC (NH₂)=NH) | L-meta-Guanidinophenylalanine |
| Phe(pNHC (NH₂)=NH) | L-para-Guanidinophenylalanine |
| Cit | L-Citrulline |

Particularly preferred residues for **group F** are

| | |
|---|---|
| Asn | L-Asparagine |
| Cys | L-Cysteine |
| Gln | L-Glutamine |
| Ser | L-Serine |
| Thr | L-Threonine |
| Cit | L-Citrulline |
| Pen | L-Penicillamine |
| AcLys | L-N^{ε}-Acetyllysine |
| hCys | L-Homo-cysteine |
| hSer | L-Homo-serine |

Particularly preferred residues for **group I** are

| | |
|---|---|
| (EA)G | N-(2-Aminoethyl)glycine |
| (PrA)G | N-(3-Amino-n-propyl)glycine |
| (BA)G | N-(4-Amino-n-butyl)glycine |
| (PeA)G | N-(5-Amino-n-pentyl)glycine |
| (EGU)G | N-(2-Guanidinoethyl)glycine |
| (PrGU)G | N-(3-Guanidino-n-propyl)glycine |
| (BGU)G | N-(4-Guanidino-n-butyl)glycine |
| (PeGU)G | N-(5-Guanidino-n-pentyl)glycine |
| (PEG₃-NH₂)G | N-[(CH₂)₃)O-(CH₂-CH₂O)₂-(CH₂)₃-NH₂]glycine |

Particularly preferred residues for **group K** are

| | |
|---|---|
| (Et-CONH₂)G | N-(2-Carbamoylethyl)glycine |
| (CH₂-CONH₂)G | N-(Carbamoylmethyl)glycine |
| (n-Pr-NHCONH₂)G | N-(3-Ureyl-n-propyl)glycine |
| (Et-SH)G | N-(2-Mercaptoethyl)glycine |
| (Et-OH)G | N-(2-Hydroxyethyl)glycine |

Generally, the peptidic chain **Z** within the β-hairpin mimetics of the invention comprises 12 amino acid residues. The positions P1 to P12 of each amino acid residue in the chain **Z** are unequivocally defined as follows: P1 represents the first amino acid in the chain **Z** that is coupled with its N-terminus to the C-terminus of the template and P12 represents the last amino acid in the chain **Z** that is coupled with its C-terminus to the N-terminus of the template. Each of the positions P1 to P12 will preferably contain an amino acid residue belonging to one of the above types **C, D, E, F** or **I,** as follows:
- P1: of type C or of type D or of type E or of type F,
- P2: of type D or of type E;
- P3: of type C;
- P4: of type E or of type I or of type F;
- P5: of type E or of type I or of type F;
- P6: of type E or of type I or of type D;
- P7: of type E or of type I or of type C;
- P8: of type D;
- P9: of type E;
- P10: of type D or of type C,
- P11: of type E or of type D; or of type C and
- P12: of type C or of type D or of type E or of type F;
- at P6 and P7 also D-isomers being possible;
with the proviso that at least one of the amino acid residues is of type I.

Most preferably the amino acid residues in position 1-12 are:
- P1: Leu; Thr; or Arg;
- P2: Arg; or Trp;
- P3: Leu;
- P4: Lys; hArg; (BA)G; or Gln;
- P5: Lys; Gln; hArg; or (PeA)G;
- P6: Arg, Trp, hArg; (EGU)G;
   - (EA)G; (PrA)G; (PeA)G or (BA)G;
- P7: Arg; (PeA)G; or Val
- P8: Trp; or Bip;
- P9: Lys; Arg; or hArg;
- P10: Tyr;
- P11: Arg; or Tyr; and
- P12: Val; or Arg;
with the proviso that
- the amino acid residue in P4 is (BA)G; and/or
- the amino acid residue in P5 is (PeA)G; and/or
- the amino acid residue in P6 is (EGU)G or (EA)G or (PrA)G or (PeA)G or (BA)G; and/or
- the amino acid residue in P7 is (PeA)G.

Particularly preferred β-peptidomimetics of the invention include those described in examples 1 to 12.

The process of the invention can advantageously be carried out as parallel array synthesis to yield libraries of template-fixed β-hairpin peptidomimetics of the above general formula I. Such parallel synthesis allows one to obtain arrays of numerous (normally 24 to 192, typically 96) compounds of general formula I in high yields and defined purities, minimizing the formation of dimeric and polymeric by-products. The proper choice of the functionalized solid-support (i.e. solid support plus linker molecule), templates and site of cyclization play thereby key roles.

The functionalized solid support is conveniently derived from polystyrene cross linked with, preferably 1-5%, divinylbenzene; polystyrene coated with polyethyleneglycol spacers (Tentagel^{R}); and polyacrylamide resins (see also Obrecht, D.; Villalgordo, J.-M, "Solid-Supported Combinatorial and Parallel Synthesis of Small-Molecular-Weight Compound Libraries", Tetrahedron Organic Chemistry Series, Vol. 17, Pergamon, Elsevier Science, 1998).

The solid support is functionalized by means of a linker, i.e. a bifunctional spacer molecule which contains on one end an anchoring group for attachment to the solid support and on the other end a selectively cleavable functional group used for the subsequent chemical transformations and cleavage procedures. For the purposes of the present invention the linker must be designed to eventually release the carboxyl group under mild acidic conditions which do not affect protecting groups present on any functional group in the side-chains of the various amino acids. Linkers which are suitable for the purposes of the present invention form acid-labile esters with the carboxyl group of the amino acids, usually acid-labile benzyl, benzhydryl and trityl esters; examples of linker structures of this kind include 2-methoxy-4-hydroxymethylphenoxy (Sasrin^{R} linker), 4-(2,4-dimethoxyphenyl-hydroxymethyl)-phenoxy (Rink linker), 4-(4-hydroxymethyl-3-methoxyphenoxy)butyric acid (HMPB linker), trityl and 2-chlorotrityl.

Preferably, the support is derived from polystyrene crosslinked with, most preferably 1-5%, divinylbenzene and functionalized by means of the 2-chlorotrityl linker.

When carried out as a parallel array synthesis the process of the invention can be advantageously carried out as described herein below but it will be immediately apparent to those skilled in the art how this procedure will have to be modified in case it is desired to synthesize one single compound of the above formula I.

A number of reaction vessels (normally 24 to 192, typically 96) equal to the total number of compounds to be synthesized by the parallel method are loaded with 25 to 1000 mg, preferably 100 mg, of the appropriate functionalized solid support, preferably 1 to 3% cross linked polystyrene or tentagel resin.

The solvent to be used must be capable of swelling the resin and includes, but is not limited to, dichloromethane (DCM), dimethylformamide (DMF), N-methylpyrrolidone (NMP), dioxane, toluene, tetrahydrofuran (THF), ethanol (EtOH), trifluoroethanol (TFE), isopropylalcohol and the like. Solvent mixtures containing as at least one component a polar solvent (e. g. 20% TFE/DCM, 35% THF/NMP) are beneficial for ensuring high reactivity and solvation of the resin-bound peptide chains ( Fields, G. B., Fields, C. G., J. Am. Chem. Soc. 1991, 113, 4202-4207).

With the development of various linkers that release the C-terminal carboxylic acid group under mild acidic conditions, not affecting acid-labile groups protecting functional groups in the side chain(s), considerable progresses have been made in the synthesis of protected peptide fragments. The 2-methoxy-4-hydroxybenzylalcohol-derived linker (SasrinR linker, Mergler et al., Tetrahedron Lett. 1988, 29 4005-4008) is cleavable with diluted trifluoroacetic acid (0.5-1% TFA in DCM) and is stable to Fmoc deprotection conditions during the peptide synthesis, Boc/tBu-based additional protecting groups being compatible with this protection scheme. Other linkers which are suitable for the process of the invention include the super acid labile 4-(2,4-dimethoxyphenyl-hydroxymethyl)-phenoxy linker (Rink linker, Rink, H. Tetrahedron Lett. 1987, 28, 3787-3790), where the removal of the peptide requires 10% acetic acid in DCM or 0.2% trifluoroacetic acid in DCM; the 4-(4-hydroxymethyl-3-methoxyphenoxy)butyric acid-derived linker (HMPB-linker, Flörsheimer & Riniker, Peptides 1991,1990 131) which is also cleaved with 1%TFA/DCM in order to yield a peptide fragment containing all acid labile side- chain protective groups; and, in addition, the 2-chlorotritylchloride linker (Barlos et al., Tetrahedron Lett. 1989, 30, 3943-3946), which allows the peptide detachment using a mixture of glacial acetic acid/trifluoroethanol/DCM (1:2:7) for 30 min.

Suitable protecting groups for amino acids and, respectively, for their residues are, for example,
- for the amino group (as is present e. g. also in the side-chain of lysine)

| | |
|---|---|
| Cbz | benzyloxycarbonyl |
| Boc | tert.-butyloxycarbonyl |
| Fmoc | 9-fluorenylmethoxycarbonyl |
| Alloc | allyloxycarbonyl |
| Teoc | trimethylsilylethoxycarbonyl |
| Tcc | trichloroethoxycarbonyl |
| Nps | o-nitrophenylsulfonyl; |
| Trt | triphenymethyl or trityl |

- for the carboxyl group (as is present e. g. also in the side-chain of aspartic and glutamic acid) by conversion into esters with the alcohol components

| | |
|---|---|
| tBu | tert.-butyl |
| Bn | benzyl |
| Me | methyl |
| Ph | phenyl |
| Pac | Phenacyl |
| | Allyl |
| Tse | trimethylsilylethyl |
| Tce | trichloroethyl; |

- for the guanidino group (as is present e. g. in the side-chain of arginine)

| | |
|---|---|
| Pmc | 2,2,5,7,8-pentamethylchroman-6-sulfonyl |
| Ts | tosyl (i. e. p-toluenesulfonyl) |
| Cbz | benzyloxycarbonyl |
| Pbf | pentamethyldihydrobenzofuran-5-sulfonyl |

- for the hydroxy group (as is present e. g. in the side-chain of threonine and serine)

| | |
|---|---|
| tBu | tert.-butyl |
| Bn | benzyl |
| Trt | trityl |

- and for the mercapto group (as is present e. g. in the side-chain of cysteine)

| | |
|---|---|
| Acm | acetamidomethyl |
| tBu | tert.-butyl |
| Bn | benzyl |
| Trt | trityl |
| Mtr | 4-methoxytrityl. |

The 9-fluorenylmethoxycarbonyl- (Fmoc)-protected amino acid derivatives are preferably used as the building blocks for the construction of the template-fixed β-hairpin loop mimetics of formula I. For the deprotection, i. e. cleaving off of the Fmoc group, 20% piperidine in DMF or 2% DBU/2% piperidine in DMF can be used.

The N-substituted glycine derivatives (types I and K) used as building blocks for the construction of the template-fixed β-hairpin loop mimetics of formula I are derived from 9-fluorenylmethoxycarbonyl- (Fmoc)-protected amino acid derivatives or preferably built up in two steps from leaving group-containing glycine precursors, such as bromo, chloro or iodo acetic acid, and suitable primary amine building blocks NH₂-R⁸⁶ or NH₂-R⁸⁷ according to definition of R⁸⁶ or R⁸⁷. The first synthesis step consists of the attachment of the leaving group-containing acetylating agent, such as bromo acetic acid, to the resin bound intermediate through formation of the amide bond. The second reaction step - the nucleophilic displacement - is accomplished using the primary amine building blocks, wherein the residues are, if necessary, suitably protected with groups as described above for side chains of amino acids.
For the incorporation of the N-substituted glycine derivatives as building blocks into the template-fixed β-hairpin loop mimetics the general synthesis procedure for assembling the hairpin mimetics is used as described herein.

The quantity of the reactant, i. e. of the amino acid derivative or leaving group-containing glycine precursor, is usually 1 to 20 equivalents based on the milliequivalents per gram (meq/g) loading of the functionalized solid support (typically 0.1 to 2.85 meq/g for polystyrene resins) originally weighed into the reaction tube. Additional equivalents of reactants can be used if required to drive the reaction to completion in a reasonable time. The reaction tubes, in combination with the holder block and the manifold, are reinserted into the reservoir block and the apparatus is fastened together. Gas flow through the manifold is initiated to provide a controlled environment, for example, nitrogen, argon, air and the like. The gas flow may also be heated or chilled prior to flow through the manifold. Heating or cooling of the reaction wells is achieved by heating the reaction block or cooling externally with isopropanol/dry ice and the like to bring about the desired synthetic reactions. Agitation is achieved by shaking or magnetic stirring (within the reaction tube). The preferred workstations (without, however, being limited thereto) are Labsource's Combi-chem station, ABI 433A and MultiSyn Tech's-Syro synthesizer.

Amide bond formation requires the activation of the α-carboxyl group for the acylation step. When this activation is being carried out by means of the commonly used carbodiimides such as dicyclohexylcarbodiimide (DCC, Sheehan & Hess, J. Am. Chem. Soc. 1955, 77, 1067-1068) or diisopropylcarbodiimide (DIC, Sarantakis et al Biochem. Biophys. Res. Commun. 1976, 73, 336-342), the resulting dicyclohexylurea is insoluble and, respectively, diisopropylurea is soluble in the solvents generally used. In a variation of the carbodiimide method 1-hydroxybenzotriazole (HOBt, König & Geiger, Chem. Ber 1970, 103, 788-798) is included as an additive to the coupling mixture. HOBt prevents dehydration, suppresses racemization of the activated amino acids and acts as a catalyst to improve the sluggish coupling reactions. Certain phosphonium reagents have been used as direct coupling reagents, such as benzotriazol-1-yl-oxy-tris-(dimethylamino)-phosphonium hexafluorophosphate (BOP) (Castro et al., Tetrahedron Lett. 1975, 14, 1219-1222; Synthesis, 1976, 751-752), or benzotriazol-1-yl-oxy-tris-pyrrolidino-phosphonium hexaflurophoshate (Py-BOP, Coste et al., Tetrahedron Lett. 1990, 31, 205-208), or 2-(1H-benzotriazol-1-yl-) 1,1,3,3-tetramethyluronium terafluoroborate (TBTU), or hexafluorophosphate (HBTU, Knorr et al., Tetrahedron Lett. 1989, 30, 1927-1930); these phosphonium reagents are also suitable for in situ formation of HOBt esters with the protected amino acid derivatives. More recently diphenoxyphosphoryl azide (DPPA) or O-(7-aza-benzotriazol-1-yl)-N,N,N',N'-tetramethyluronium tetrafluoroborate (TATU) or O-(7-aza-benzotriazol-1-yl)-N,N,N',N'-tetramethyluronium hexafluorophosphate (HATU)/7-aza-1-hydroxy benzotriazole (HOAt, Carpino et al., Tetrahedron Lett. 1994, 35, 2279-2281) have also been used as coupling reagents.

Due to the fact that near-quantitative coupling reactions are essential it is desirable to have experimental evidence for completion of the reactions. The ninhydrin test (Kaiser et al., Anal. Biochemistry 1970, 34, 595), where a positive colorimetric response to an aliquot of resin-bound peptide indicates qualitatively the presence of the primary amine, can easily and quickly be performed after each coupling step. Fmoc chemistry allows the spectrophotometric detection of the Fmoc chromophore when it is released with the base (Meienhofer et al., Int. J. Peptide Protein Res. 1979, 13, 35-42).

The nucleophilic displacement reaction substituting the leaving group of the glycine precursor is preferably performed in DMF. The typical amount of the primary amine building block used for the nucleophilic displacement reaction is between 1 and 12 eq based on the milliequivalents per gram (meq/g) loading of the functionalized solid support. The experimental evidence for completion of the acetylation- and the following nucleophilic displacement reaction of the two-step procedure for assembling N-substituted glycine derivatives used as building blocks is usually not monitored (R.N. Zuckermann, J. Am. Chem. Soc. 1992, 114, 10646-10647 and cited references).

The resin-bound intermediate within each reaction tube is washed free of excess of retained reagents, of solvents, and of by-products by repetitive exposure to pure solvent(s) by one of the two following methods:
1) The reaction wells are filled with solvent (preferably 5 ml), the reaction tubes, in combination with the holder block and manifold, are immersed and agitated for 5 to 300 minutes, preferably 15 minutes, and drained by gravity followed by gas pressure applied through the manifold inlet (while closing the outlet) to expel the solvent;
2) The manifold is removed from the holder block, aliquots of solvent (preferably 5 ml) are dispensed through the top of the reaction tubes and drained by gravity through a filter into a receiving vessel such as a test tube or vial.

Both of the above washing procedures are repeated up to about 50 times (preferably about 10 times), monitoring the efficiency of reagent, solvent, and byproduct removal by methods such as TLC, GC, or inspection of the washings.

The above described procedure of reacting the resin-bound compound with reagents within the reaction wells followed by removal of excess reagents, by-products, and solvents is repeated with each successive transformation until the final resin-bound fully protected linear peptide has been obtained.

Before this fully protected linear peptide is detached from the solid support, it is possible, if desired, to selectively deprotect one or several protected functional group(s) present in the molecule and to appropriately substitute the reactive group(s) thus liberated. To this effect, the functional group(s) in question must initially be protected by a protecting group which can be selectively removed without affecting the remaining protecting groups present. Alloc (allyloxycarbonyl) is an example for such a protecting group for amino which can be selectively removed, e.g. by means of Pd° and phenylsilane in CH₂Cl₂, without affecting the remaining protecting groups, such as Fmoc, present in the molecule. The reactive group thus liberated can then be treated with an agent suitable for introducing the desired substituent. Thus, for example, an amino group can be acylated by means of an acylating agent corresponding to the acyl substituent to be introduced.

Detachment of the fully protected linear peptide from the solid support is achieved by immersion of the reaction tubes, in combination with the holder block and manifold, in reaction wells containing a solution of the cleavage reagent (preferably 3 to 5 ml). Gas flow, temperature control, agitation, and reaction monitoring are implemented as described above and as desired to effect the detachment reaction. The reaction tubes, in combination with the holder block and manifold, are disassembled from the reservoir block and raised above the solution level but below the upper lip of the reaction wells, and gas pressure is applied through the manifold inlet (while closing the outlet) to efficiently expel the final product solution into the reservoir wells. The resin remaining in the reaction tubes is then washed 2 to 5 times as above with 3 to 5 ml of an appropriate solvent to extract (wash out) as much of the detached product as possible. The product solutions thus obtained are combined, taking care to avoid cross-mixing. The individual solutions/extracts are then manipulated as needed to isolate the final compounds. Typical manipulations include, but are not limited to, evaporation, concentration, liquid/liquid extraction, acidification, basification, neutralization or additional reactions in solution.

The solutions containing fully protected linear peptide derivatives which have been cleaved off from the solid support and neutralized with a base, are evaporated. Cyclization is then effected in solution using solvents such as DCM, DMF, dioxane, THF and the like. Various coupling reagents which were mentioned earlier can be used for the cyclization. The duration of the cyclization is about 6-48 hours, preferably about 24 hours. The progress of the reaction is followed, e. g. by RP-HPLC (Reverse Phase High Performance Liquid Chromatography). Then the solvent is removed by evaporation, the fully protected cyclic peptide derivative is dissolved in a solvent which is not miscible with water, such as DCM, and the solution is extracted with water or a mixture of water-miscible solvents, in order to remove any excess of the coupling reagent.

Before removing the protecting groups from the fully protected cyclic peptide, it is possible, if desired, to form an interstrand linkage between side-chains of appropriate amino acid residues at opposite positions of the β-strand region.

Interstrand linkages and their formation have been discussed above, in connection with the explanations made regarding groups of the type H which can, for example, be disulfide bridges formed by cysteines and homocysteines at opposite positions of the β-strand, or glutamic and aspartic acid residues linking ornithines and, respectively, lysines located at opposite β-strand positions by amide bond formation. The formation of such interstrand linkages can be effected by methods well known in the art.

The fully protected peptide derivative of type I is treated with 95% TFA, 2.5% H₂O, 2.5% TIS or another combination of scavengers for effecting the cleavage of protecting groups. The cleavage reaction time is commonly 30 minutes to 12 hours, preferably about 2 hours.

After the deprotection it is possible, if desired, to convert any amino group(s) present in the molecule into guanidino groups using appropriate guanidinylation reagents (K. Feichinger et al, J. Org. Chem. 1998, 63, 3804-2805). Suitable guanidinylation reagents include, but are not limited to, N,N-di-Boc-trifluoromethanesulfonylguanidine. Such guanidinylation will convert, for example, any (EA)G residue to (EGU)G and, simultaneously, any Lys residue to hArg.

Finally thereafter most of the solvent (such as TFA) which usually still is present is evaporated and the product is precipitated with ether/hexane (1:1) or other solvents which are suitable therefor. After careful removal of the solvent, the cyclic peptide derivative obtained as end-product can be isolated. Depending on its purity, this peptide derivative can be used directly for biological assays, or it has to be further purified, for example by preparative HPLC.

As mentioned earlier, it is thereafter possible, if desired, to convert a compound of formula I thus obtained into a pharmaceutically acceptable salt or to convert a pharmaceutically acceptable, or unacceptable, salt thus obtained into the corresponding free compound of formula I or into a different, pharmaceutically acceptable, salt. Any of these operations can be carried out by methods well known in the art.

The starting materials of formulae H₂NR⁸⁶ and H₂NR⁸⁷ are known or can be prepared by methods which are well known in the art.

The β-hairpin peptidomimetics of the invention can be used in a wide range of applications in order to inhibit the growth of or to kill microorganisms.

They can be used for example as disinfectants or as preservatives for materials such as foodstuffs, cosmetics, medicaments and other nutrient-containing materials. The β-hairpin peptidomimetics of the invention can also be used to treat or prevent diseases related to microbial infection in plants and animals.

For use as disinfectants or preservatives the β-hairpin peptidomimetics can be added to the desired material singly, as mixtures of several β-hairpin peptidomimetics or in combination with other antimicrobial agents. The β-hairpin peptidomimetics may be administered per se or may be applied as an appropriate formulation together with carriers, diluents or excipients well known in the art.

When used to treat or prevent infections or diseases related to such infections particularly infections related to respiratory diseases such as cystic fibrosis, the β-hairpin peptidomimetics can be administered singly, as mixtures of several β-hairpin peptidomimetics, in combination with other antimicrobial or antibiotic agents or in combination with antiviral (e.g. anti-HIV) or anti cancer agents or in combination with other pharmaceutically active agents. The β-hairpin peptidomimetics can be administered per se or as pharmaceutical compositions.

Pharmaceutical compositions comprising β-hairpin peptidomimetics of the invention may be manufactured by means of conventional mixing, dissolving, granulating, coated tablet-making, levigating, emulsifying, encapsulating, entrapping or lyophilizing processes. Pharmaceutical compositions may be formulated in conventional manner using one or more physiologically acceptable carriers, diluents, excipients or auxilliaries which facilitate processing of the active β-hairpin peptidomimetics into preparations which can be used pharmaceutically. Proper formulation depends upon the method of administration chosen.

For topical administration the β-hairpin peptidomimetics of the invention may be formulated as solutions, gels, ointments, creams, suspensions, etc. as are well-known in the art.

Systemic formulations include those designed for administration by injection, e.g. subcutaneous, intravenous, intramuscular, intrathecal or intraperitoneal injection, as well as those designed for transdermal, transmucosal, oral or pulmonary administration.

For injections, the β-hairpin peptidomimetics of the invention may be formulated in adequate solutions, preferably in physiologically compatible buffers such as Hink's solution, Ringer's solution, or physiological saline buffer. The solution may contain formulatory agents such as suspending, stabilizing and/or dispersing agents. Alternatively, the β-hairpin peptidomimetics of the invention may be in powder form for combination with a suitable vehicle, e.g., sterile pyrogen-free water, before use.

For transmucosal administration, penetrants appropriate to the barrier to be permeated are used in the formulation as known in the art.

For oral administration, the compounds can be readily formulated by combining the active β-hairpin peptidomimetics of the invention with pharmaceutically acceptable carriers well known in the art. Such carriers enable the β-hairpin peptidomimetics of the invention to be formulated as tablets, pills, dragees, capsules, liquids, gels, syrups, slurries, suspensions etc., for oral ingestion of a patient to be treated. For oral formulations such as, for example, powders, capsules and tablets, suitable excipients include fillers such as sugars, such as lactose, sucrose, mannitol and sorbitol; cellulose preparations such as maize starch, wheat starch, rice starch, potato starch, gelatin, gum tragacanth, methyl cellulose, hydroxypropylmethyl cellulose, sodium carboxymethylcellulose, and/or polyvinylpyrrolidone (PVP); granulating agents; and binding agents. If desired, desintegrating agents may be added, such as cross-linked polyvinylpyrrolidones, agar, or alginic acid or a salt thereof, such as sodium alginate. If desired, solid dosage forms may be sugar-coated or enteric-coated using standard techniques.

For oral liquid preparations such as, for example, suspensions, elixirs and solutions, suitable carriers, excipients or diluents include water, glycols, oils, alcohols, etc. In addition, flavoring agents, preservatives, coloring agents and the like may be added.

For buccal administration, the composition may take the form of tablets, lozenges, etc. formulated as usual.

For administration by inhalation, the β-hairpin peptidomimetics of the invention are conveniently delivered in form of an aeorosol spray from pressurized packs or a nebulizer, with the use of a suitable propellant, e.g. dichlorodifluoromethane, trichlorofluromethane, carbon dioxide or another suitable gas. In the case of a pressurized aerosol the dose unit may be determined by providing a valve to deliver a metered amount. Capsules and cartridges of e.g. gelatin for use in an inhaler or insufflator may be formulated containing a powder mix of the β-hairpin peptidomimetics of the invention and a suitable powder base such as lactose or starch.

The compounds may also be formulated in rectal or vaginal compositions such as suppositories together with appropriate suppository bases such as cocoa butter or other glycerides.

In addition to the formulation described previously, the β-hairpin peptidomimetics of the invention may also be formulated as depot preparations. Such long acting formulations may be administered by implantation (e.g. subcutaneously or intramuscularly) or by intramuscular injection. For the manufacture of such depot preparations the β-hairpin peptidomimetics of the invention may be formulated with suitable polymeric or hydrophobic materials (e.g. as an emulsion in an acceptable oil) or ion exchange resins, or as sparingly soluble salts.

In addition, other pharmaceutical delivery systems may be employed such as liposomes and emulsions well known in the art. Certain organic solvents such as dimethylsulfoxide also may be employed. Additionally, the β-hairpin peptidomimetics of the invention may be delivered using a sustained-release system, such as semipermeable matrices of solid polymers containing the therapeutic agent. Various sustained-release materials have been established and are well known by those skilled in the art. Sustained-release capsules may, depending on their chemical nature, release the compounds for a few weeks up to over 100 days. Depending on the chemical nature and the biological stability of the therapeutic agent, additional strategies for protein stabilization may be employed.

As the β-hairpin pepdidomimetics of the invention may contain charged residues, they may be included in any of the above-described formulations as free bases or as pharmaceutically acceptable salts. Pharmaceutically acceptable salts tend to be more soluble in aqueous and other protic solvents than are the corresponding free base forms.

The β-hairpin peptidomimetics of the invention, or compositions thereof, will generally be used in an amount effective to achieve the intended purpose. It is to be understood that the amount used will depend on a particular application.

For example, for use as a desinfectant or preservative, an antimicrobially effective amount of a β-hairpin peptidomimetic of the invention, or a composition thereof, is applied or added to the material to be desinfected or preserved. By antimicrobially effective amount is meant an amount of a β-hairpin peptidomimetic of the invention or composition that inhibits the growth of, or is lethal to, a target microbe population. While the antimicrobially effective amount will depend on a particular application, for use as desinfectants or preservatives the β-hairpin peptidomimetics of the invention, or compositions thereof, are usually added or applied to the material to be desinfected or preserved in relatively low amounts. Typically, the β-hairpin peptidomimetics of the invention comprise less than about 5% by weight of a desinfectant solution or material to be preserved, preferably less than 1% by weight and more preferably less than 0.1% by weight. An ordinary skilled expert will be able to determine antimicrobially effective amounts of particular β-hairpin pepdidomimetics of the invention for particular applications without undue experimentation using, for example, the in vitro assays provided in the examples.

For use to treat or prevent microbial infections or diseases related to such infections, the β-hairpin pepidomimetics of the invention, or compositions thereof, are administered or applied in a therapeutically effective amount. By therapeutically effective amount is meant an amount effective in ameliorating the symptoms of, or ameliorate, treat or prevent microbial infections or diseases related thereto. Determination of a therapeutically effective amount is well within the capacities of those skilled in the art, especially in view of the detailed disclosure provided herein.

As in the case of desinfectants and preservatives, for topical administration to treat or prevent bacterial infections a therapeutically effective dose can be determined using, for example, the in vitro assays provided in the examples. The treatment may be applied while the infection is visible, or even when it is not visible. An ordinary skilled expert will be able to determine therapeutically effective amounts to treat topical infections without undue experimentation.

For systemic administration, a therapeutically effective dose can be estimated initially from in vitro assays. For example, a dose can be formulated in animal models to achieve a circulating β-hairpin peptidomimetic concentration range that includes the IC₅₀ as determined in the cell culture (i.e. the concentration of a test compound that is lethal to 50% of a cell culture), the MIC, as determined in cell culture (i.e. the concentration of a test compound that is lethal to 100% of a cell culture). Such information can be used to more accurately determine useful doses in humans.

Initial dosages can also be determined from in vivo data, e.g. animal models, using techniques that are well known in the art. One having ordinary skills in the art could readily optimize administration to humans based on animal data.

Dosage amounts for applications as antimicrobial agents may be adjusted individually to provide plasma levels of the β-hairpin peptidomimetics of the invention which are sufficient to maintain the therapeutic effect. Therapeutically effective serum levels may be achieved by administering multiple doses each day.

In cases of local administration or selective uptake, the effective local concentration of the β-hairpin peptidomimetics of the invention may not be related to plasma concentration. One having the skills in the art will be able to optimize therapeutically effective local dosages without undue experimentation.

The amount of β-hairpin peptidomimetics administered will, of course, be dependent on the subject being treated, on the subject's weight, the severity of the affliction, the manner of administration and the judgement of the prescribing physician.

The antimicrobial therapy may be repeated intermittently while infections are detectable or even when they are not detectable. The therapy may be provided alone or in combination with other drugs, such as for example antibiotics or other antimicrobial agents.

Normally, a therapeutically effective dose of the β-hairpin peptidomimetics described herein will provide therapeutic benefit without causing substantial toxicity.

Hemolysis of red blood cells is often employed for assessment of toxicity of related compounds such as *protegrin* or *tachyplesin.* Values are given as %-lysis of red blood cells observed at a concentration of 100µg/ml. Typical values determined for cationic peptides such as *protegrin* and *tachyplesin* range between 30-40% with average MIC-values of 1-5 µg/ml over a wide range of pathogens. Normally, β-hairpin peptidomimetics of the invention will show hemolysis in a range of 0.5-10%, often in a range of 1-5%, at activity levels comparable to those mentioned above for *protegrin* and *tachyplesin.* Thus preferred compounds exhibit low MIC-values and low %-hemolysis of red blood cells observed at a concentration of 100µg/ml.

Toxicity of the β-hairpin peptidomimetics of the invention herein can be determined by standard pharmaceutical procedures in cell cultures or experimental animals, e.g., by determining the LD₅₀ (the dose lethal to 50% of the population) or the LD₁₀₀ (the dose lethal to 100% of the population). The dose ratio between toxic and therapeutic effect is the therapeutic index. Compounds which exhibit high therapeutic indices are preferred. The data obtained from these cell culture assays and animal studies can be used in formulating a dosage range that is not toxic for use in humans. The dosage of the β-hairpin peptidomimetics of the invention lies preferably within a range of circulating concentrations that include the effective dose with little or no toxicity. The dosage may vary within the range depending upon the dosage form employed and the route of administration utilized. The exact formulation, route of administration and dose can be chosen by the individual physician in view of the patient's condition (see, e.g. Fingl et al. 1975, In : The Pharmacological Basis of Therapeutics, Ch.1, p.1).

The following Examples illustrate the invention in more detail but are not intended to limit its scope in any way. The following abbreviations are used in these Examples:
HBTU : 1-benzotriazol-1-yl-tetramethylurounium hexafluorophosphate (Knorr et al. Tetrahedron Lett. 1989, 30, 1927-1930)
HOBt : 1-hydroxybenzotriazole
DIEA : diisopropylethylamine
HOAT: 7-aza-1-hydroxybenzotriazole
HATU: O-(7-aza-benzotriazole-1-yl)-N,N,N',N'-tetramethyluronoium hexafluorophosphate Carpino et al. Tetrahedron Left. 1994, 35, 2279-2281)

### Examples

### 1. Peptide synthesis

### Coupling of the first amino acid residue

1.0 g of 2-chlorotritylchloride resin (Barlos et al. Tetrahedron Lett. 1989, 30, 3943-3946) (1.08 mMol/g, 1.08 mmol) was filled into a dried flask. The resin was suspended in CH₂Cl₂ (10 ml) and allowed to swell at room temperature under constant stirring. The resin was treated two times with 0.18g (1.2eq.) bromo acid acid and 0.738 ml (4eq) of diisopropylethylamine (DIEA) in CH₂Cl₂ (10 ml), the mixture was shaken at 25°C for 2 h. The resin was washed extensively (CH₂Cl₂ /MeOH/DIEA : 17/2/1; CH₂Cl₂, DMF; CH₂Cl₂; Et₂O, 3 times each).
This was followed by nucleophilic substitution of bromine with a Boc-protected amine. Boc-protected amines used were Boc-NH-CH₂-CH₂-NH₂, Boc-NH-CH₂-CH₂-CH₂-NH₂ and Boc-NH-CH₂-CH₂-CH₂-CH₂-NH₂. The Boc-protected amine, dissolved (3eq) in DMSO:CH₂Cl₂ 1:1 v/v 5 ml was added to the resin. The resin was washed with CH₂Cl₂, DMF, CH₂Cl₂ 5 ml, 3 times).
The resin was dried overnight.
The following preloaded resins were prepared: (EA)G-O-chlorotritylresin; (BA)G-O-chlorotritylresin; (PrA)G-O-chlorotritylresin.

### Synthesis cycle

The synthesis was carried out using a Syro-peptide synthesizer (Multisyntech) or an ABI 433A using 24 to 96 reaction vessels. In each vessel was placed 60 mg weight of the resin before loading) of the above resin. The following reaction cycles were programmed and carried out:

| **Step** | **Reagent** | **Time** |
|---|---|---|
| 1 | CH₂Cl₂, wash and swell (manual) | 3 x 1 min. |
| 2 | DMF, wash and swell | 1 x 5 min |
| **3** | 40 % piperidine/DMF | 1 x 5 min. |
| **4** | DMF, wash | 5 x 2 min. |
| **5a** | 5 equiv. Fmoc amino acid/DMF | |
| | + 5 eq. HBTU | |
| | + 5 eq. HOBt | |
| | + 5 eq. DIEA | 1 x 120 min. |
| **6** | DMF, wash | 4 x 2 min. |
| 7 | CH₂Cl₂, wash (at the end of the synthesis) | 3 x 2 min. |

| | | |
|---|---|---|
| Steps **3** to **6** are repeated to add each amino-acid or N-substituted glycine building block. | | |

To introduce a N-substituted glycine building blocks into specific positions within the chain, the following steps 5b.1-5b.3 were used instead of step 5a.
- 5b.1: 11 equiv. BrCH₂COOH/DMF + 13 equiv. DIC, 90 min;
- 5b.2: DMF, wash 4 x 2 min;
- 5b.3: 20 equiv. amine building block with protected residue/DMF, 120 min.
Moreover, if a N-substituted glycine building block had been introduced in the previous cycle, step 5a was modified as follows: HBTU and HOBt were replaced by 3.5 eq. HATU, and 7 eq DIEA were used.

### Cleavage of the fully protected peptide fragment

After completion of the synthesis, the resin was suspended in 1 ml (0.39 mMol) of 1% TFA in CH₂Cl₂ (v/v) for 3 minutes, filtered and the filtrate was neutralized with 1 ml (1.17 mMol, 3eq.) of 20% DIEA in CH₂Cl₂ (v/v). This procedure was repeated twice to ensure completion of the cleavage. The filtrate was evaporated to dryness and a sample of the product was fully deprotected to be analyzed by reverse phase-HPLC (column C₁₈) to monitor the efficiency of the linear peptide synthesis.

### Cyclization of the linear peptide

100 mg of the fully protected linear peptide were dissolved in DMF (9 ml, conc. 10 mg/ml). Then 41.8 mg (0.110 mMol, 3 eq.) of HATU, 14.9 mg (0.110 mMol, 3 eq) of HOAt and 1 ml (0.584 mMol) of 10% DIEA in DMF (v/v) were added and the mixture was vortexed at 20°C for 16 hours and subsequently concentrated under high vacuum. The residue was partitioned between CH₂Cl₂ and H₂O/CH₃CN (90/10; v/v). The CH₂Cl₂ phase was evaporated to yield the fully protected cyclic peptide.

### Deprotection of the cyclic peptide

The cyclic peptide obtained was dissolved in 1 ml of the cleavage mixture containing 95% trifluoroacetic acid (TFA), 2.5% water and 2.5% triisopropylsilane (TIS). The mixture was left to stand at 20°C for 2.5 hours and then concentrated under vacuum. The residue was dissolved in a solution of H₂O/acetic acid (75/25: v/v) and the mixture extracted with diisopropylether.
The water phase was dried under vacuum and then the product purified by preparative reverse phase HPLC.

### Guanidinylation of the side chain amine functions with diprotected Triflyl-guanidines

8 mg of the deprotected cyclic peptide and N,N-di-Boc-trifluoromethanesulfonylguanidine (270 mg, 15 eq) wered dissolved in water and dioxane (3 ml, 1:5; v/v). Triethylamine (190µl, 15 eq) was added and the solution was stirred at room temperature for 72 h. The solution was concentrated under vacuum to remove dioxane. Water (1 ml) and CH₂Cl₂ (1 ml) was added. The extracted CH₂Cl₂ phase was econcentrated. The residue was redissolved in a mixture of TFA and CH₂Cl₂ (1:1, 2 ml) for 1 h at room temperature. The solution was then triturated with diethyl ether, the organic layer was removed and the residue dried in vacuo.

### Purification of the cyclic peptide

After lyophilisation the products were obtained as white powders and analysed by ESI-MS. The analytical data comprising HPLC retention times and ESI-MS are shown in table 1.

Analytical HPLC retention times (RT, in minutes) were determined using a VYDAC 218TP104 (length 25cm) column with *gradient A* (50% CH₃CN + 0.1% TFA and 50% H₂O + 0.1% TFA to 100% CH₃CN + 0.1% TFA and 0% H₂O + 0.1% TFA in 25minutes) or *gradient B* using solvents A (H₂O + 0.02% TFA) and B (CH₃CN) 0 min: 92%A, 8%B; 8 min: 62%A 38%B; 9-12 min: 0% A, 100%B.
Purification was done using preparative reverse phase HPLC with *gradient C*:
10% CH₃CN + 0.1 % TFA and 90% H₂O +0.1% TFA to 60% CH₃CN + 0.1% TFA and 40% H₂O + 0.1% TFA in 20 minutes.

**Examples 1 and 3-6** are shown in *table 1.* The peptides were synthesized starting with the amino acid at position P6 which was grafted to the resin. Starting resins were (EA)G-O-chlorotritylresin; (BA)G-O-chlorotritylresin; and (PrA)G-O-chlorotritylresin, which were prepared as described above. The linear peptides were synthesized on solid support according to the above procedure in the following sequence: P7-P8-P9-P10-P11-P12-^{D}Pro-Pro-P1-P2-P3-P4-P5-P6-resin, cleaved, cyclized, deprotected and purified as indicated.
HPLC-retention times (minutes) were determined using *gradient A.*

**Example 2** is also shown in *table 1.* The peptide was synthesized starting with a precursor of the amino acid at position P6 which was grafted to the resin. Starting resin was (EA)G-O chlorotritylresin, which was prepared as described above. The linear peptide was synthesized on solid support according to the above procedure in the following sequence: P7-P8-P9-P10-P11-P12-^{D}Pro-Pro-P1-P2-P3-P4-P5-precursor of P6-resin, Lys being introduced at positions P9, P4 and P5. The protected peptide was then cleaved, cyclized, deprotected, guanidinylated and purified as indicated, the guanidinylation transforming (EA)G into (EGU)G and Lys into hArg.
HPLC-retention times (minutes) were determined using *gradient A*.

**Examples 7-12** are shown in *table 1,* too.
0.5 g of 2-chlorotritylchloride resin (Barlos et al. Tetrahedron Lett. 1989, 30, 3943-3946) (0.83 mMol/g, 0.415 mmol) was filled into a dried flask. The resin was suspended in CH₂Cl₂ (2.5 ml) and allowed to swell at room temperature under constant stirring for 30 min. The resin was treated with 0.415 mMol (1eq) of the first suitably protected amino acid residue (see below) and 284 µl (4eq) of diisopropylethylamine (DIEA) in CH₂Cl₂ (2.5 ml), the mixture was shaken at 25°C for 4 hours. The resin colour changed to purple and the solution remained yellowish. The resin was shaken (CH₂Cl₂ /MeOH/DIEA : 17/2/1), 30 ml for 30 min; then washed in the following order with CH₂Cl₂ (1x), DMF (1x), CH₂Cl₂ (1x), MeOH (1x), CH₂Cl₂(1x), MeOH (1x), CH₂Cl₂ (2x), Et₂O (2x) and dried under vacuum for 6 hours. Loading was typically 0.6-0.7 mMol/g.
The following preloaded resin was prepared: Fmoc-ProO-chlorotritylresin.
The peptides were synthesized starting with the amino acid Pro which was grafted to the resin. Starting resins was Fmoc-ProO-chlorotritylresin, which was prepared as described above. The linear peptides were synthesized on solid support according to the above procedure in the following sequence: Resin-Pro- ^{D}Pro-P12-P11-P10-P9-P8-P7-P6- P5-P4-P3-P2-P1, cleaved, cyclized, deprotected and purified as indicated. HPLC-retension times (minutes) were determined using the *gradient B* as described above. The incorporation of (PeA)G in the relevant positions of examples 7-12 was accomplished using bromo acetic acid and Boc-NH-CH₂-CH₂-CH₂-CH₂-CH₂-NH₂.

**Table 1: Examples**

| Example | Sequ.ID | P1 | P2 | P3 | P4 | P5 | P6 | P7 | P8 | P9 | P10 | P11 | P12 | Template | RT(') | %^{B} | [MS] |
|---|---|---|---|---|---|---|---|---|---|---|---|---|---|---|---|---|---|
| 1 | SEQ ID NO:1 | Leu | Arg | Leu | Lys | Lys | (EA)G | Arg | Trp | Lys | Tyr | Arg | Val | ^{D}Pro^{L}Pro | 10.4 | 68 | 1823.9 |
| 2 | SEQ ID NO:2 | Leu | Arg | Leu | hArg | hArg | (EGU)G | Arg | Trp | hArg | Tyr | Arg | Val | ^{D}Pro^{L}Pro | 10.8 | 40 | 498.6* |
| 3 | SEQ ID NO:3 | Leu | Arg | Leu | Lys | Lys | (PrA)G | Arg | Trp | Lys | Tyr | Arg | Val | ^{D}Pro^{L}Pro | 10.0 | 51 | 933.2** |
| 4 | SEQ ID NO:4 | Leu | Arg | Leu | Lys | Lys | (BA)G | Arg | Bip | Lys | Tyr | Arg | Val | ^{D}Pro^{L}Pro | 10.5 | 63 | 1889.0 |
| 5 | SEQ ID NO:5 | Leu | Arg | Leu | (BA)G | Lys | (BA)G | Arg | Bip | Lys | Tyr | Arg | Val | ^{D}Pro^{L}Pro | 11.8 | 54 | 1890.0 |
| 6 | SEQ ID NO:6 | Leu | Arg | Leu | Lys | Lys | (PrA)G | Arg | Bip | Lys | Tyr | Arg | Val | ^{D}Pro^{L}Pro | 10.6 | 55 | 1902.8 |
| 7 | SeQ ID NO: 7 | Arg | Trp | Leu | Lys | Lys | Arg | (PeA)G | Trp | Lys | Tyr | Tyr | Val | ^{D}Pro^{L}Pro | 5.67 | 100 | 959.3** |
| 8 | SEQ ID NO: 8 | Arg | Trp | Leu | Gln | (PeA)G | Arg | Arg | Trp | Lys | Tyr | Tyr | Arg | ^{D}Pro^{L}Pro | 5.28/6.05 | 100 | 1015.2** |
| 9 | SEQ ID NO: 9 | Arg | Trp | Leu | Lys | (PeA)G | Arg | Arg | Trp | Lys | Tyr | Tyr | Val | ^{D}Pro^{L}Pro | 5.78 | 100 | 986.9** |
| 10 | SEQ ID NO: 10 | Thr | Trp | Leu | Lys | (PeA)G | Arg | Arg | Trp | Lys | Tyr | Tyr | Arg | ^{D}Pro^{L}Pro | 5.2 | 80 | 986.9** |
| 11 | SEQ ID NO: 11 | Arg | Trp | Leu | Gln | Lys | Arg | (PeA)G | Trp | Lys | Tyr | Tyr | Arg | ^{D}Pro^{L}Pro | 5.25 | 100 | 1001.3** |
| 12 | SEQ ID NO: 12 | Thr | Trp | Leu | Lys | (PeA)G | Arg | Arg | Trp | Lys | Tyr | Tyr | Arg | ^{D}Pro^{L}Pro | 5.27/5.93 | 70 | 987.7** |

| | | | | | | | | | | | | | | | | | |
|---|---|---|---|---|---|---|---|---|---|---|---|---|---|---|---|---|---|
| a) %-purity of compounds after prep. HPLC * M/z + 4H, z = 4 ** M/z, z = 2 | | | | | | | | | | | | | | | | | |

### 2. Biological methods

### 2.1. Preparation of the peptides.

Lyophilized peptides were weighed on a Microbalance (Mettler MT5) and dissolved in sterile water containing 0.01 % acetic acid.

### 2.2. Antimicrobial activity of the peptides.

The antimicrobial activities of the peptides were determined by the standard NCCLS broth microdilution method (see ref 1, below) examined in sterile 96-wells plates (Nunclon polystyrene microtiter plates) in a total volume of 100 µl. Innocula of the microorganisms were prepared with 0.5 Mcfarland standard and then diluted into Mueller-Hinton (MH) broth to give appr. 10⁶ colony forming units (CFU)/ml for bacteria Aliquots (50 µl) of the innocula were added to 50 µl of MH broth containing the peptide in serial twofold dilutions. The microorganisms used were *Escherichia coli* (ATCC 25922), *Pseudomonas aeruginosa (P. aeruginosa)* (ATCC 27853), *Pseudomonas putida* (ATCC 27853), *Staphylococcus aureus* (ATCC 29213 and ATCC 25923). Antimicrobial activities of the peptides were expressed as the minimal inhibitory concentration (MIC) in µg/ml at which no visible growth was observed after 18-20 hours of incubation of the microtiter plates at 37°C.

### 2.3. Hemolysis

The peptides were tested for their hemolytic activity against human red blood cells (hRBC). Fresh hRBC were washed three times with phosphate buffered saline (PBS) by centrifugation for 10 min at 2000 x g. Peptides at a concentration of 100 µg/ml were incubated with 20% v/v hRBC for 1 hour at 37°C. The final erythrocyte concentration was appr. 0.9 x 10⁹ /ml. A value of 0% resp. 100% cell lysis was determined by incubation of the hRBC in the presence of PBS alone and resp. 0.1% Triton X-100 in H₂O. The samples were centrifuged and the supernatant was 20 fold diluted in PBS buffer and the optical density (OD) of the sample at 540 nM was measured. The 100% lysis value (OD₅₄₀H₂0) gave an OD of approximately 1.6-2.0. Percent hemolysis was calculated as follows: (OD₅₄₀peptide/OD₅₄₀H₂0) x100%.

### 2.4. Results

The results of the experiments described above are indicated in Table 2, herein below.

### References

1. National Committee for Clinical Laboratory Standards. 1993. Methods for dilution antimicrobial susceptibility tests for bacteria that grow aerobically, 3rd ed. Approved standard M7-A3. National Committee for Clinical laboratory standards, Villanova, Pa.
2. Mossman T. J Immunol Meth 1983, 65, 55-63
3. Berridge MV, Tan AS. Archives of Biochemistry & Biophysics 1993, 303, 474-482

**Table 2. Minimal inhibitory concentrations (MIC in µg/ml) and percentage hemolyses at a concentration of 100 µg/ml of peptide**

| Ex. | *Escherichia coli* ATCC 25922 | *Pseudomonas putida* ATCC 27853 | *Staphylococcus aureus* ATCC 29213 | *Staphylococcus aureus* ATCC 25923 | P. Aeruginosa ATCC27853 | *Hemolyses hRBC* |
|---|---|---|---|---|---|---|
| 1 | 6.2 | 6.2 | 12.5 | 12.5 | n.d. | 1.7 |
| 2 | 12.5 | 6.2 | 12.5 | 12.5 | n.d. | 8.8 |
| 3 | 25 | 12.5 | 50 | 50 | n.d. | 1.6 |
| 4 | 12.5 | 12.5 | 12.5 | 12.5 | n.d. | 4.2 |
| 5 | 6.2 | 12.5 | 6.2 | 6.2 | n.d. | 2.1 |
| 6 | 12.5 | 6.2 | 6.2 | 6.2 | n.d. | 7.0 |
| 8 | 32 | n.d. | 128 | 128 | 8 | 1.1 |
| 9 | 32 | n.d. | 128 | l28 | 16 | 1.4 |
| 10 | 64 | n.d. | 128 | 128 | 8 | 1.0 |
| 11 | 32 | n.d. | 128 | 128 | 8 | 1.1 |
| 12 | 64 | n.d. | 128 | 128 | 8 | 2.2 |

| | | | | | | |
|---|---|---|---|---|---|---|
| n.d.: not determined | | | | | | |

## Claims

1. Compounds of the general formula 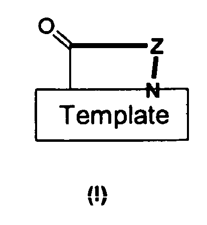 wherein 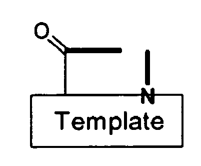 is a group of one of the formulae ^{D}Pro-^{L}Pro and ^{L}Pro-^{D}Pro
R⁸ is H; Cl; F; CF₃; NO₂; lower alkyl; lower alkenyl; aryl; aryl-lower alkyl,;
-(CH₂)ₒ(CHR⁶¹)ₛOR⁵⁵; -(CH₂)ₒ(CHR⁶¹)ₛSR⁵⁶; -(CH₂)ₒ(CHR⁶¹)NR³³R³⁴;
-(CH₂)ₒ(CHR⁶¹)ₛSOCONR³³R⁷⁵; -(CH₂)ₒ(CHR⁶¹)ₛNR²⁰CONR³³R⁸²;
-(CH₂)ₒ₍CHR⁶¹)ₛCOOR⁵⁷; -(CH₂)ₒ(CHR⁶¹)ₛCONR⁵⁸R⁵⁹; -(CH₂)ₒ(CHR⁶¹)ₛPO(OR⁶⁰)₂;
-(CH₂)ₒ(CHR⁶¹)ₛSO₂R⁶²; or -(CH₂)ₒ(CHR⁶¹)ₛSCOR⁶⁴;
R²⁰ is H; alkyl; alkenyl; or aryl-lower alkyl;
R³³ is H; alkyl, alkenyl; -(CH₂)ₘ(CHR⁶¹)ₛOR⁵⁵; -(CH₂)ₘ(CHR⁶¹)ₛNR³⁴R⁶³;
-(CH₂)ₘ(CHR⁶¹)ₛOCONR⁷⁵R⁸²; -(CH₂)ₘ(CHR⁶¹)ₛNR²⁰CONR⁷⁸R⁸²;
-(CH₂)ₒ(CHR⁶¹)ₛ,COR⁶⁴; -(CH₂)ₒ(CHR⁶¹)ₛ-CONR⁵⁸R⁵⁹, -(CH₂)ₒ(CHR⁶¹)ₛPO(OR⁶⁰)₂;
-(CH₂)ₒCHR⁶¹)ₛ SO₂R⁶²; or -(CH₂)ₒ(CHR⁶¹)ₛC₆H₄R⁸;
R³⁴ is H; lower alkyl; aryl, or aryl-lower alkyl;
R³³ and R³⁴ taken together can form: -(CH₂)₂₋₆-; -(CH₂)₂O(CH₂)₂-; -(CH₂)₂S(CH₂)₂-; or -(CH₂)₂NR⁵⁷(CH₂)₂-;
R³⁷ is H; F; Br; Cl; NO₂; CF₃; lower alkyl; -(CH₂)ₚ(CHR⁶¹)ₛOR⁵⁵; -(CH₂)ₚ(CHR⁶¹)ₛNR³³R³⁴;
-(CH₂)ₚ(CHR⁶¹)ₛOCONR³³R⁷⁵; -(CH₂)ₚ(CHR⁶¹)ₛNR²⁰CONR³³R¹²;
-(CH₂)ₒ(CHR⁶¹)ₛCOOR¹⁷; -(CH₂)ₒ(CHR⁶¹)ₛCONR⁵⁸R⁵⁹; -(CH₂)ₒ(CHR⁶¹)ₛPO(OR⁶⁰)₂;
-(CH₂)ₒ(CHR⁶¹)ₛSO₂R⁶²; or -(CH₂)ₒ(CHR⁶¹)ₛ C₆H₄R⁸;
R⁵⁰ is H; lower alkyl; or aryl-lower alkyl;
R⁵⁵ is H; lower alkyl; lower alkenyl; aryl-lower alkyl; -(CH₂)ₘ(CHR⁶¹)ₛOR⁵⁷;
-(CH₂)ₘ(CHR⁶¹)ₛNR³⁴R⁶³; -(CH₂)ₘ(CHR⁶¹)ₛOCONR⁷⁵R⁸²;
-(CH₂)ₘ(CHR⁶¹)ₛNR²⁰CONR⁷⁸R⁸²; -(CH₂)ₒ(CHR⁶¹)ₛ-COR⁶⁴;
-(CH₂)ₒ(CHR⁶¹)COOR⁵⁷; or
-(CH₂)ₒ(CHR⁶¹)ₛCONR⁵⁸R⁵⁹;
R⁵⁶ is H; lower alkyl; lower alkenyl; aryl-lower alkyl; -(CH₂)ₘ(CHR⁶¹)ₛOR⁵⁷;
-(CH₂)ₘ(CHR⁶¹)ₛNR³⁴R⁶³; -(CH₂)ₘ(CHR⁶¹)ₛOCONR⁷⁵R⁸²;
-(CH₂)ₘ(CHR⁶¹)ₛNR²⁰CONR⁷⁸R⁸²; -(CH²)ₒ(CHR⁶¹)ₛ-COR⁶⁴; or
-(CH₂)ₒ(CHR⁶¹)ₛCONR⁵⁸R⁵⁹;
R⁵⁷ is H; lower alkyl; lower alkenyl; aryl lower alkyl; or heteroaryl lower alkyl;
R⁵⁸ is H; lower alkyl; lower alkenyl; aryl; heteroaryl; aryl-lower alkyl; or heteroaryl-lower alkyl;
R⁵⁹ is H; lower alkyl; lower alkenyl; aryl; heteroaryl; aryl-lower alkyl; or heteroaryl-lower alkyl; or
R⁵⁸ and R⁵⁹ taken together can form: -(CH₂)₂-₆-; -(CH₂)₂O(CH₂)₂-; -(CH₂)₂S(CH₂)₂-; or -(CH₂)₂NR⁵⁷(CH₂)₂-;
R⁶⁰ is H; lower alkyl; lower alkenyl; aryl; or aryl-lower alkyl;
R⁶¹ is alkyl; alkenyl; aryl; heteroaryl; aryl-lower alkyl; heteroaryl-lower alkyl; -(CH₂)ₘOR⁵⁵;
-(CH₂)ₘNR³³R³⁴; -(CH₂)ₘOCONR⁷⁵R⁸²; -(CH₂)ₘNR²⁰CONR⁷⁸R⁸²; -(CH₂)ₒCOOR³⁷;
-(CH₂)ₒNR⁵⁸R⁵⁹; or -(CH₂)ₒPO(COR⁶⁰)₂;
R⁶² is lower alkyl; lower alkenyl; aryl, heteroaryl; or aryl-lower alkyl;
R⁶³ is H; lower alkyl; lower alkenyl; aryl, heteroaryl; aryl-lower alkyl; heteroaryl-lower alkyl;
-COR⁶⁴; -COOR⁵⁷; -CONR⁵⁸R⁵⁹; -SO₂R⁶²; or -PO(OR⁶¹)₂; or
R³⁴ and R⁶³ taken together can form: -(CH₂)₂₋₆-; -(CH₂)₂O(CH₂)₂-; -(CH₂)₂S(CH₂)₂-; or -(CH₂)₂NR¹⁷(CH₂)₂-;
R⁶⁴ is H; lower alkyl; lower alkenyl; aryl; heteroaryl; aryl-lower alkyl; heteroaryl-lower alkyl;
-(CH₂)ₚ(CHR⁶¹)ₛOR⁶⁵; -(CH₂)ₚ(CHR⁶¹)ₛSR⁶⁶; or -(CH₂)ₚ(CHR⁶¹)ₛNR³⁴R⁶³;
-(CH₂)ₚ(CHR⁶¹)ₛOCONR⁷⁵R⁸²; -(CH₂)ₚ(CHR⁶¹)ₛNR²⁰CONR⁷⁸R⁸²;
R⁶⁵ is H; lower alkyl; lower alkenyl; aryl, aryl-lower alkyl; heteroaryl-lower alkyl; -COR⁵⁷; -COOR⁵⁷; or -CONR⁵⁸R⁵⁹.
R⁶⁶ is H; lower alkyl; lower alkenyl; aryl; aryl-lower alkyl; heteroaryl-lower alkyl; or -CONR⁵⁸R⁵⁹;
m is 2-4; o is 0-4; p is 1-4; q is 0-2; r is 1 or 2; s is 0 or 1;
Z is a chain of 12 α-amino acid residues, the positions of said amino acid residues in said chain being counted starting from the N-terminal amino acid, whereby these amino acid residues are, depending on their position in the chains, Gly, or Pro, or of one of the types
C: -NR²⁰CH(R⁷²)CO-;
D: -NR²⁰CH(R⁷³)CO-;
E: -NR²⁰CH(R⁷⁴)CO-;
F: -NR²⁰CH(R⁸⁴)CO-; and
H: -NR²⁰-CH(CO-)-(CH₂)₄₋₇-CH(CO-)-NR²⁰-;
-NR²⁰-CH(CO-)-(CH₂)ₚSS(CH₂)ₚ-CH(CO-)-NR²⁰-;
-NR²⁰-CH(CO-)-(-(CH₂)_{P}NR²⁰CO(CH₂)_{P}CH(CO-)-NR²⁰-; and
-NR²⁰-CH(CO-)-(-(CH₂)_{P}NR²⁰CONR²⁰(CH₂)ₚCH(CO-)-NR²⁰-;
I: -NR⁸⁶CH₂CO-;
K: -NR⁸⁷CH₂CO-;
R⁷² is H, lower alkyl; lower alkenyl; -(CH₂)ₚ(CHR⁶¹)ₛOR⁸⁵; or -(CH₂)ₚ(CHR⁶¹)ₛSR⁸⁵;
R⁷³ is -(CH₂)ₒR⁷⁷; -(CH₂)ᵣO(CH₂)ₒR⁷⁷; -(CH₂)ᵣS(CH₂)ₒR⁷⁷; or -(CH₂)ᵣNR²⁰(CH₂)ₒR⁷⁷;
R⁷⁴ is -(CH₂)ₚNR⁷⁸R⁷⁹; -(CH₂)ₚNR⁷⁷R⁸⁰; -(CH₂)ₚC(=NR⁸⁰)NR⁷⁸R⁷⁹;
-(CH₂)ₚC(=NOR⁵⁰)NR⁷⁸R⁷⁹;
-(CH₂)ₚC(=NNR⁷⁸R⁷⁹)NR⁷⁸R⁷⁹; -(CH₂)ₚNR⁸⁰C(=NR⁸⁰)NR⁷⁸R⁷⁹;
-(CH₂)ₚN=C(NR⁷⁸R⁸⁰)NR⁷⁹R⁸⁰;-(CH₂)ₚC₆H₄NR⁷⁸R⁷⁹; -(CH₂)ₚC₆H₄NR⁷⁷R⁸⁰;
-(CH₂)ₚC₆H₄C(=NR⁸⁰)NR⁷⁹; -(CH₂)ₚC₆H₄C(=NOR⁵⁰)NR⁷⁸R⁷⁹;
-(CH₂)ₚC₆H₄C(=NNR⁷⁸R⁷⁹)NR⁷⁸R⁷⁹; -(CH₂)ₚC₆H₄NR⁸⁰C(=NR⁸⁰)NR⁷⁸R⁷⁹;
-(CH₂)ₚC₆H₄N=C(NR⁷⁸R⁸⁰)NR⁷⁹R⁸⁰; -(CH₂)ᵣO(CH₂)ₘNR⁷⁸R⁷⁹;
-(CH₂)ᵣO(CH₂)ₘNR⁷⁷R⁸⁰_{;}
-(CH₂)ᵣO(CH₂)ₚC(=NR⁸⁰)NR⁷⁸R⁷⁹; -(CH₂)ᵣO(CH₂)ₚC(=NOR⁵⁰)NR⁷⁸R⁷⁹;
-(CH₂)ᵣO(CH₂)ₚC(=NNR⁷⁸R⁷⁹)NR⁷⁸R⁷⁹; -(CH₂)ᵣO(CH₂)ₘNR⁸⁰C(=NR⁸⁰)NR⁷⁸R⁷⁹;
-(CH₂)ᵣO(CH₂)ₘN=C(NR⁷⁸R⁸⁰⁾NR⁷⁹R⁸⁰; -(CH₂)ᵣO(CH₂)ₚC₆H₄CNR⁷⁸N⁷⁹;
-(CH₂)ᵣO(CH₂)ₚC₆H₄C(=NR⁸⁰)NR⁷⁸R⁷⁹; -(CH₂)ᵣO(CH₂)ₚC₆H₄C(=NOR⁵⁰)NR⁷⁸R⁷⁹;
-(CH₂)ᵣO(CH₂)ₚC₆H₄C(=NNR⁷⁸R⁷⁹)NR⁷⁸R⁷⁹;
-(CH₂)ᵣO(CH₂)ₚC₆H₄NR⁸⁰C(=NR⁸⁰)NR⁷⁸R⁷⁹; -(CH₂)ᵣS(CH₂)ₘNR⁷⁸R⁷⁹;
-(CH₂)ᵣS(CH₂)ₘNR⁷⁷R⁸⁰;-(CH₂)ᵣS(CH₂)ₚC(=NR⁸⁰NR⁷⁸R⁷⁹;
-(CH₂)ᵣS(CH₂)ₚC(=NOR⁵⁰)NR⁷⁸R⁷⁹; -(CH₂)ᵣS(CH₂)ₚC(=NNR⁷⁸R⁷⁹)NR⁷⁸R⁷⁹;
-(CH₂)ᵣS(CH₂)ₘNR⁸⁰C(=NR⁸⁰)NR⁷⁸R⁷⁹; -(CH₂)ᵣS(CH₂)ₘN=C(NR⁷⁸R⁸⁰)NR⁷⁹R⁸⁰;
-(CH₂)ᵣS(CH₂)ₚC₆H₄CNR⁷⁸R⁷⁹; -(CH₂)ᵣS(CH₂)ₚC₆H₄C(=NR⁸⁰)NR⁷⁸R⁷⁹;
-(CH₂)ᵣS(CH₂)ₚC₆H₄(=NOR⁵⁰)NR⁷⁸R⁷⁹; -(CH₂)ᵣS(CH₂)ₚC₆H₄C(=NNR⁷⁸R⁷⁹)NR⁷⁸R⁷⁹;
-(CH₂)ᵣS(CH₂)ₚC₆H₄NR⁸⁰C(=NR⁸⁰)NR⁷⁸R⁷⁹; -(CH₂)ₚNR⁸⁰COR⁶⁴; -(CH₂)ₚNR⁸⁰COR⁷⁷;
-(CH₂)ₚNR⁸⁰CONR⁷⁸R⁷⁹; or -(CH₂)ₚC₆H₄NR⁸⁰CONR⁷⁸R⁷⁹;
R⁷⁵ is lower alkyl; lower alkenyl; or aryl-lower alkyl; or
R³³ and R⁷⁵ taken together can form: -(CH₂)₂₋₆-; -(CH₂)₂O(CH₂)₂-; -(CH₂)₂S(CH₂)₂-; or -(CH₂)₂NR¹⁷(CH₂)₂-; or
R⁷⁵ and R⁸² taken together can form: -(CH₂)₂₋₆-; -(CH₂)₂O(CH₂)₂-; -(CH₂)₂S(CH₂)₂-; or -(CH₂)₂NR¹⁷(CH₂)₂-;
R⁷⁷ is R⁸⁸; or a heteroaryl group of one of the formulae 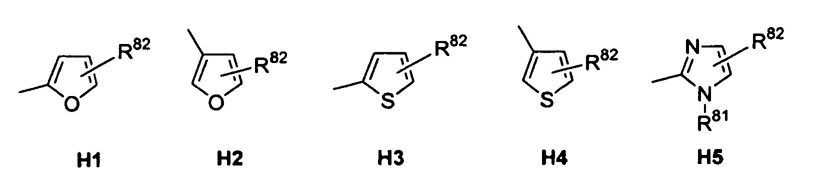 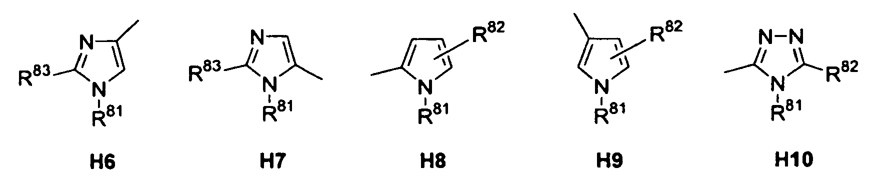 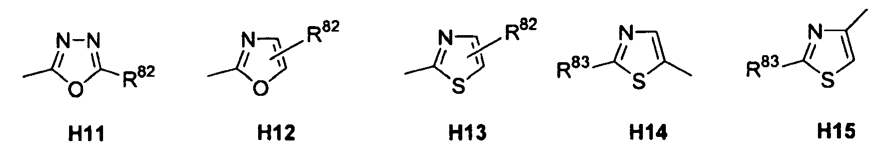 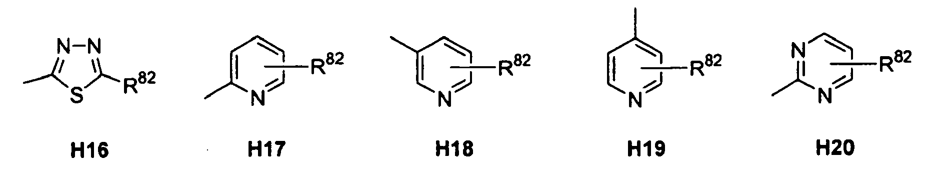 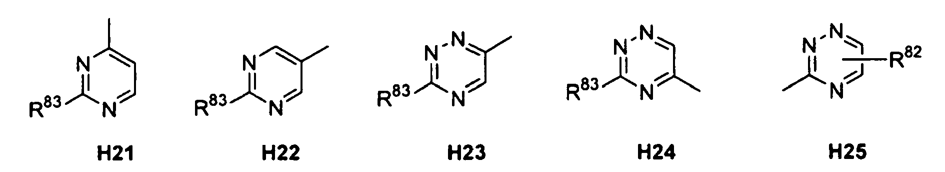 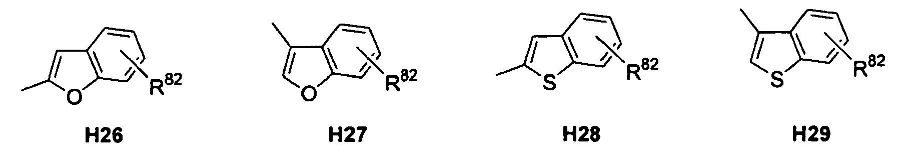 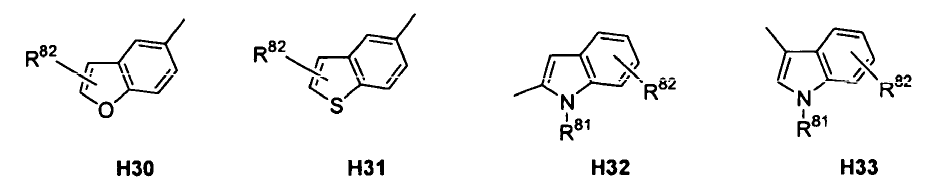 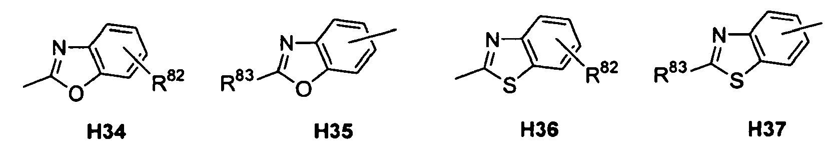 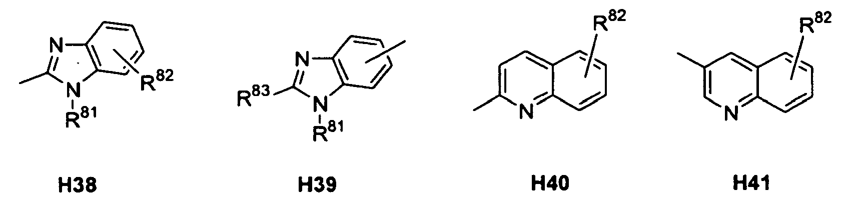 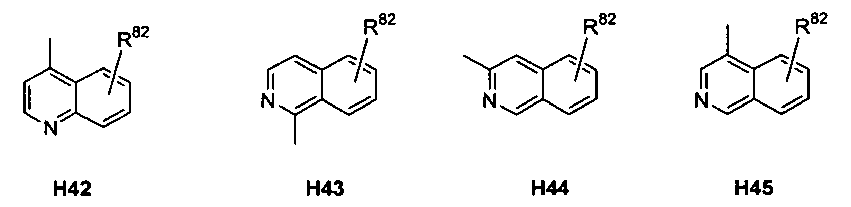 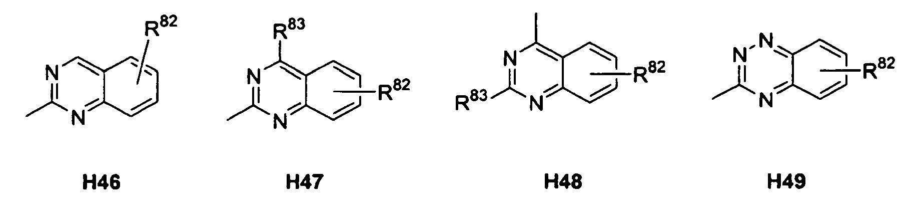 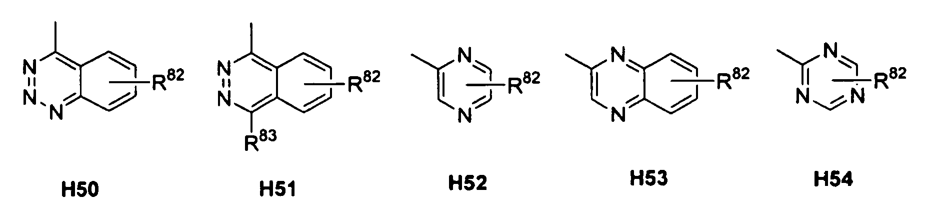
R⁷⁸ is H; lower alkyl; aryl; or aryl-lower alkyl;
R⁷⁸ and R⁸² taken together can form: -(CH₂)₂-₆-; -(CH₂)₂O(CH₂)₂-; -(CH₂)₂S(CH₂)₂-; or -(CH₂)₂NR⁵⁷(CH₂)₂-;
R⁷⁹ is H; lower alkyl; aryl; or aryl-lower alkyl,; or
R⁷⁸ and R⁷⁹, taken together, can be -(CH₂)₂₋₇-; -(CH₂)₂O(CH₂)₂-; or -(CH₂)₂NR⁵⁷(CH₂)₂-;
R⁸⁰ is H; or lower alkyl;
R⁸¹ is H; lower alkyl; or aryl-lower alkyl;
R⁸² is H; lower alkyl; aryl; heteroaryl; or aryl-lower alkyl,;
R³³ and R⁸² taken together can form: -(CH₂)₂-₆-; -(CH₂)₂O(CH₂)₂-; -(CH₂)₂S(CH₂)₂-; or -(CH₂)₂NR⁵⁷(CH₂)₂-;
R⁸³ is H; lower alkyl; aryl; or -NR⁷⁸R⁷⁹;
R⁸⁴ is -(CH₂)ₘ(CHR⁶¹)ₛOR⁷⁸; -(CH₂)ₘ(CHR⁶¹)ₛSR⁷⁸; -(CH₂)ₚCONR⁷⁸R⁷⁹; -(CH₂)ₚNR⁸⁰CONR⁷⁸R⁷⁹;
-(CH₂)ₚC₆H₄CONR⁷⁸R⁷⁹; or -(CH₂)ₚC₆H₄NR⁸⁰CONR⁷⁸R⁷⁹;
R⁸⁵ is lower alkyl; or lower alkenyl;
R⁸⁶ is R⁷⁴; -[(CH₂)ᵤ-X]ₜ-(CH₂)ᵥNR⁷⁸R⁷⁹; -[(CH₂)ᵤ₋X]ₜ-(CH₂)ᵥ-C(=NR⁸⁰)NR⁷⁸R⁷⁹; X is -O-, -NR²⁰-, -S-,
-OCOO-, u is 1-3, t is 1-6, v is 1-3;
R⁸⁷ is R⁸⁴; -[(CH₂)ᵤ-X]ₜ-(CH₂)ᵥOR⁷⁸, -[(CH₂)ᵤ-X]ₜ-(CH₂)ᵥ-CONR⁷⁸R⁷⁹, -[(CH₂)ᵤ-X]ₜ-(CH₂)ᵥ-NR⁸⁰CONR⁷⁸R⁷⁹, -[(CH₂)ᵤ-X]ₜ-(CH₂)ᵥ SR⁷⁸; X is -O-, -NR²⁰-, -S-, -OCOO-, u is 1-3, t is 1-6, v is 1-3;
R⁸⁸ is phenyl, p-hydrxyphenyl, 2-naphthyl, 1-naphthyl, 4-chlorophenyl, 3-chlorophenyl, 2-chlorophenyl, 3,4-dichlorophenyl, 4-fluorophenyl, 3-fluorophenyl, 2-fluorophenyl, p-benzyloxyphenyl, p-biphenyl or p-benzoylphenyl.
with the proviso that in said chain of 12 α-amino acid residues Z the amino acid residues in positions 1 to 12 are:
- P1: of type C or of type D or of type E or of type F, or the residue is Pro;
- P2: of type E or of type D;
- P3: of type C, or the residue is Pro;
- P4: of type E or of type F or of type I or of type K;
- P5: of type E or of type D or or of type C or of type I or of type K or of type F, or the residue is Gly or Pro;
- P6: of type E or of type F, or of type I or of type K or of type D, or the residue is Gly;
- P7: of type E or of type F or of type I or of type C;
- P8: of type D or of type C, or the residue is Pro;
- P9: of type E or of type D or of type F ;
- P10: of type D or of type C or the residue is Pro;
- P11: of type E or of type D or of type C; and
- P12: of type C or of type D or of type E or of type F, or the residue is Pro; or
- P4 and P9 and/or P2 and P11, taken together, can form a group of type H; and at P6 and P7 also D-isomers being possible;
with the further proviso that said chain of 12 α-amino acid residues contains at least one residue of type I or of type K;
and pharmaceutically acceptable salts thereof.

2. Compounds according to claim 1 wherein the α-amino acid residues in position 1 - 12 of chain Z are:
- P1: of type C or of type D or of type E or of type F,
- P2: of type D or of type E;
- P3: of type C;
- P4: of type E or of type I or of type F;
- P5: of type E or of type I or of type F;
- P6: of type E or of type I or of type D;
- P7: of type E or of type I or of type C;
- P8: of type D;
- P9: of type E;
- P10: of type D or of type C,
- P11: of type E or of type D; or of type C and
- P12: of type C or of type D or of type E or of type F;
- at P6 and P7 also D-isomers being possible;
with the proviso that at least one of the amino acid residues is of type I.

3. Compounds according to claim 2 wherein the α-amino acid residues in position 1 - 12 of the chain Z are:
P1: Leu; Thr; or Arg;
- P2: Arg; or Trp;
- P3: Leu;
- P4: Lys; hArg; (BA)G; or Gln;
- P5: Lys; Gln; hArg; or (PeA)G;
- P6: Arg, Trp, hArg; (EGU)G;
- (EA)G; (PrA)G; (PeA)G or (BA)G;
- P7: Arg; (PeA)G; or Val
- P8: Trp; or Bip;
- P9: Lys; Arg; or hArg;
- P10: Tyr;
- P11: Arg; or Tyr; and
- P12: Val; or Arg
with the proviso that
- the amino acid residue in P4 is (BA)G; and/or
- the amino acid residue in P5 is (PeA)G; and/or
- the amino acid residue in P6 is (EGU)G or
(EA)G or (PrA)G or (PeA)G or (BA)G; and/or
- the amino acid residue in P7 is (PeA)G.

4. A compound of formula I according to claim 1 wherein the template is ^{D}Pro-^{L}Pro and the amino acid residues in position 1-12 are:
- P1: Leu;
- P2: Arg;
- P3: Leu;
- P4: Lys;
- P5: Lys;
- P6: (EA)G;
- P7: Arg;
- P8: Trp;
- P9: Lys;
- P10: Tyr;
- P11: Arg; and
- P12: Val

5. A compound of formula Ia according to claim 1 wherein the template is ^{D}Pro-^{L}Pro and the amino acid residues in position 1-12 are:
- P1: Leu;
- P2: Arg;
- P3: Leu;
- P4: hArg;
- P5: hArg;
- P6: (EGU)G;
- P7: Arg;
- P8: Trp;
- P9: hArg;
- P10: Tyr;
- P11 : Arg; and
- P12: Val

6. A compound of formula I according to claim 1 wherein the template is ^{D}Pro-^{L}Pro and the amino acid residues in position 1-12 are:
- P1: Leu;
- P2: Arg;
- P3: Leu;
- P4: Lys;
- P5: Lys;
- P6: (PrA)G;
- P7: Arg;
- P8: Trp;
- P9: Lys;
- P10: Tyr;
- P11: Arg; and
- P12: Val

7. A compound of formula I according to claim 1 wherein the template is ^{D}Pro-^{L}Pro; and the amino acid residues in position 1-12 are:
- P1: Leu;
- P2: Arg;
- P3: Leu;
- P4: Lys;
- P5: Lys;
- P6: (BA)G;
- P7: Arg;
- P8: Bip;
- P9: Lys;
- P10: Tyr;
- P11: Arg; and
- P12: Val

8. A compound of formula I according to claim 1 wherein the template is ^{D}Pro-^{L}Pro and the amino acid residues in position 1-12 are:
- P1: Leu;
- P2: Arg;
- P3: Leu;
- P4: (BA)G;
- P5: Lys;
- P6: (BA)G;
- P7: Arg;
- P8: Bip;
- P9: Lys;
- P10: Tyr;
- P11: Arg; and
- P12: Val

9. A compound of formula I according to claim 1 wherein the template is ^{D}Pro-^{L}Pro and the amino acid residues in position 1-12 are:
- P1: Leu;
- P2: Arg;
- P3: Leu;
- P4: Lys;
- P5: Lys;
- P6: (PrA)G;
- P7: Arg;
- P8: Bip;
- P9: Lys;
- P10: Tyr;
- P11: Arg; and
- P12: Val

10. A compound of formula I according to claim 1 wherein the template is ^{D}Pro-^{L}Pro and the amino acid residues in position 1-12 are:
P1: Arg;
- P2: Trp;
- P3: Leu;
- P4: Lys;
- P5: Lys;
- P6: Arg;
- P7: (PeA)G;
- P8: Trp;
- P9: Lys;
- P10: Tyr;
- P11: Tyr; and
- P12: Val

11. A compound of formula I according to claim 1 wherein the template is ^{D}Pro-^{L}Pro and the amino acid residues in position 1-12 are:
P1: Arg;
- P2: Trp;
- P3: Leu;
- P4: Gln;
- P5: (PeA)G;
- P6: Arg;
- P7: Arg;
- P8: Trp;
- P9: Lys;
- P10: Tyr;
- P11: Tyr; and
- P12: Arg

12. A compound of formula I according to claim 1 wherein the template is ^{D}Pro-^{L}Pro and the amino acid residues in position 1-12 are:
P1: Arg;
- P2: Trp;
- P3: Leu;
- P4: Lys;
- P5: (PeA)G;
- P6: Arg;
- P7: Arg;
- P8: Trp;
- P9: Lys;
- P10: Tyr;
- P11: Tyr; and
- P12: Val

13. A compound of formula I according to claim 1 wherein the template is ^{D}Pro-^{L}Pro and the amino acid residues in position 1-12 are:
P1: Thr;
- P2: Trp;
- P3: Leu;
- P4: Lys;
- P5: (PeA)G;
- P6: Arg;
- P7: Arg;
- P8: Trp;
- P9: Lys;
- P10: Tyr;
- P11: Tyr; and
P12: Arg

14. A compound of formula I according to claim 1 wherein the template is ^{D}Pro-^{L}Pro and the amino acid residues in position 1-12 are:
P1: Arg;
- P2: Trp;
- P3: Leu;
- P4: Gln;
- P5: Lys;
- P6: Arg;
- P7: (PeA)G;
- P8: Trp;
- P9: Lys;
- P10: Tyr;
- P11: Tyr; and
P12: Arg

15. A compound of formula I according to claim 1 wherein the template is ^{D}Pro-^{L}Pro and the amino acid residues in position 1-12 are:
P1: Thr;
- P2: Trp;
- P3: Leu;
- P4: Lys;
- P5: (PeA)G;
- P6: Arg;
- P7: Arg;
- P8: Trp;
- P9: Lys;
- P10: Tyr;
- P11: Tyr; and
P12: Arg

16. Enantiomers of the compounds of formula I as defined in claim 1.

17. Compounds according to any one of claims 1 to 16 for use as therapeutically active substances.

18. Compounds according the claim 17 having antibacterial activity.

19. A pharmaceutical composition containing a compound according to any one of claims 1 to 17 and a pharmaceutically inert carrier.

20. Compositions according to claim 19 in a form suitable for oral, topical, transdermal, injection, buccal, transmucosal, pulmonary or inhalation administration.

21. Compositions according to claim 18 or 19 in form of tablets, dragees, capsules, solutions, liquids, gels, plaster, creams, ointments, syrup, slurries, suspensions, spray, nebuliser or suppositories.

22. The use of compounds according to any one of claims 1 to 16 for the manufacture of a medicament for treating or preventing infections or diseases related to such infections, said disease being in particular Cystic Fibrosis.

23. The use of compounds according to any one of claims 1 to 16 as disinfectants or preservatives for foodstuffs, cosmetics, medicaments and other nutrient-containing materials.

24. A process for the manufacture of compounds according to any one of claims 1-15 which process comprises
(a) coupling an appropriately functionalized solid support with an appropriately N-protected derivative of that amino acid which in the desired end-product is in position 5, 6 or 7, any functional group which may be present in said N-protected amino acid derivative being likewise appropriately protected;
(b) removing the N-protecting group from the product thus obtained;
(c) coupling the product thus obtained with an appropriately N-protected derivative of that amino acid which in the desired end-product is one position nearer the N-terminal amino acid residue, any functional group which may be present in said N-protected amino acid derivative being likewise appropriately protected;
(d) removing the N-protecting group from the product thus obtained;
(e) repeating steps (c) and (d) until the N-terminal amino acid residue has been introduced;
(f) coupling the product thus obtained with
(fa) an appropiatly N-protected derivative of ^{L}Pro or ^{D}Pro
(fb) removing the N-protecting group from the product thus obtained; anf
(fc) coupling the product thus obtained with an appropiatly N-protected derivative of ^{D}Pro and, respectively ^{L}Pro;
(g) removing the N-protecting group from the product obtained in step (fc);
(h) coupling the product thus obtained with an appropriately N-protected derivative of that amino acid which in the desired end-product is in position 12, any functional group which may be present in said N-protected amino acid derivative being likewise appropriately protected;
(i) removing the N-protecting group from the product thus obtained;
(j) coupling the product thus obtained with an appropriately N-protected derivative of that amino acid which in the desired end-product is one position farther away from position 12, any functional group which may be present in said N-protected amino acid derivative being likewise appropriately protected;
(k) removing the N-protecting group from the product thus obtained;
(l) repeating steps (j) and (k) until all amino acid residues have been introduced;
(m) if desired, selectively deprotecting one or several protected functional group(s) present in the molecule and appropriately substituting the reactive group(s) thus liberated;
(o) detaching the product thus obtained from the solid support;
(p) cyclizing the product cleaved from the solid support;
(q) if desired, forming one or two interstrand linkage(s) between side-chains of appropriate amino acid residues at opposite positions of the β-strand region;
(r) removing any protecting groups present on functional groups of any members of the chain of amino acid residues and, if desired, any protecting group(s) which may in addition be present in the molecule;
(s) if desired guanidinylating any side-chain amino group present in the chain of amino acid residues; and
(t) if desired, converting the product thus obtained into a pharmaceutically acceptable salt or converting a pharmaceutically acceptable, or unacceptable, salt thus obtained into the corresponding free compound of formula I or into a different, pharmaceutically acceptable, salt.

25. A process for the manufacture of compounds according to any one of claims 1-15 which process comprises
(a') coupling an appropriately functionalized solid support
(a'a) coupling said appropriately functionalized solid support with an appropriately N-protected derivative of ^{L}Pro or ^{D}Pro
(a'b) removing the N-protecting group from the product thus obtained; and
(a'c) coupling the product thus obtained with an appropriately N-protected derivative of ^{D}Pro and, respectively, ^{L}Pro;
(b') removing the N-protecting group from the product obtained in step (a'c);
(c') coupling the product thus obtained with an appropriately N-protected derivative of that amino acid which in the desired end-product is one position nearer the N-terminal amino acid residue, any functional group which may be present in said N-protected amino acid derivative being likewise appropriately protected;
(d') removing the N-protecting group from the product thus obtained;
(e') coupling the product thus obtained with an appropriately N-protected derivative of that amino acid which in the desired end-product is one position farther away from position 12, any functional group which may be present in said N-protected amino acid derivative being likewise appropriately protected;
(f) removing the N-protecting group from the product thus obtained;
(g') repeating steps (e') and (f) until all amino acid residues have been introduced;
(h') if desired, selectively deprotecting one or several protected functional group(s) present in the molecule and appropriately substituting the reactive group(s) thus liberated;
(i') detaching the product thus obtained from the solid support;
(j') cyclizing the product cleaved from the solid support;
(k') if desired forming one or two interstrand linkage(s) between side-chains of appropriate amino acid residues at opposite positions of the β-strand region;
(l') removing any protecting groups present on functional groups of any members of the chain of amino acid residues and, if desired, any protecting group(s) which may in addition be present in the molecule;
(m') if desired guanidinylating any side-chain amino group present in the chain of amino acid residues; and
(n') if desired, converting the product thus obtained into a pharmaceutically acceptable salt or converting a pharmaceutically acceptable, or unacceptable, salt thus obtained into the corresponding free compound of formula I or into a different, pharmaceutically acceptable, salt.

26. A process according to claim 24 or 25 but wherein an amino acid residue of type I or K is introduced by coupling with a leaving group-containing acetylating agent, followed by nucleophilic displacement with an amine of the formula H₂NR⁸⁶ and, respectively, H₂NR⁸⁷ which, if necessary, is appropriately protected.

27. A process according to claim 26 wherein said leaving group-containing acetylating agent is bromo, chloro or iodo acetic acid.

28. A modification of the processes according to any one of claims 24 to 27 for the manufacture of compounds according to claim 16 in which enantiomers of all chiral starting materials are used.

## Patentansprüche

1. Verbindungen der allgemeinen Formel 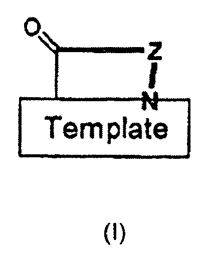 worin 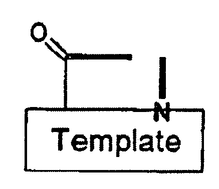 eine Gruppe bedeutet der Formel ^{D}Pro-^{L}Pro und ^{L}Pro-^{D}Pro
R⁸ bedeutet H ; Cl ; F ; CF₃; NO₂; niedriges Alkyl; niedriges Alkenyl; Aryl; Aryl - niedriges Alkyl; -(CH₂)ₒ(CHR⁶¹)ₛOR⁵⁵; -(CH₂)ₒ(CHR⁶¹)ₛSR⁵⁶; -(CH₂)ₒ(CHR⁶¹)NR³³R³⁴;
-(CH₂)ₒ(CHR⁶¹)ₛOCONR³³R⁷⁵; -(CH₂)ₒ(CHR⁶¹)ₛNR²⁰CONR³³R⁸²;
-(CH₂)ₒ(CHR⁶¹)ₛCOOR⁵⁷; -(CH₂)ₒ(CHR⁶¹)ₛCONR⁵⁸R⁵⁹, -(CH₂)ₒ(CHR⁶¹)ₛPO(OR⁶⁰)₂;
-(CH₂)ₒ(CHR⁶¹)ₛSO₂R⁶²; oder -(CH₂)ₒ₍CHR⁶¹)ₛCOR⁶⁴;
R²⁰ bedeutet H; Alkyl; Alkenyl; oder Aryl - niedriges Alkyl;
R³³ bedeutet H; Alkyl, Alkenyl; -(CH₂)ₘ(CHR⁶¹)ₛOR⁵⁵; -(CH₂)ₘ(CHR⁶¹)ₛNR³⁴R⁶³;
-(CH₂)ₘ(CHR⁶¹)ₛOCONR⁷⁵R⁸²; -(CH₂)ₘ(CHR⁶¹)ₛNR²⁰CONR⁷⁸R⁸²;
-(CH₂)ₒ(CHR⁶¹)ₛCOR⁶⁴; -(CH₂)ₒ(CHR⁶¹)ₛ-CONR⁵⁸R⁵⁹,
-(CH₂)ₒ(CHR⁶¹)ₛPO(OR⁶⁰)₂; -(CH₂)ₒ(CHR⁶¹)ₛ SO₂R⁶²; oder
-(CH₂)ₒ(CHR⁶¹)ₛC₆H₄R⁸;
R³⁴ bedeutet H; niedriges Alkyl; Aryl, oder Aryl - niedriges Alkyl;
R³³ und R³⁴ können gemeinsam formen: -(CH₂)₂-₆-; -(CH₂)₂O(CH₂)₂-; -(CH₂)₂S(CH₂)₂- oder -(CH₂)₂NR⁵⁷(CH₂)₂-;
R³⁷ bedeutet H; F; Br; Cl; NO₂; CF₃; niedriges Alkyl; -(CH₂)ₚ(CHR⁶¹)ₛOR⁵⁵;
-(CH₂)ₚ(CHR⁶¹)ₛNR³³R³⁴; -(CH₂)ₚ(CHR⁶¹)ₛOCONR³³R⁷⁵;
-(CH₂)ₚ(CHR⁶¹)ₛNR²⁰CONR³³R⁸²; -(CH₂)ₒ(CHR⁶¹)ₛCOOR⁵⁷;
-(CH₂)ₒ(CHR⁶¹)ₛCONR⁵⁸R⁵⁹; -(CH₂)ₒ(CHR⁶¹)ₛPO(OR⁶⁰)₂;
-(CH₂)ₒ(CHR⁶¹)ₛSO₂R⁶²; oder -(CH₂)ₒ(CHR⁶¹)ₛC₆H₄R⁸;
R⁵⁰ bedeutet H; niedriges Alkyl; oder Aryl - niedriges Alkyl;
R⁵⁵ bedeutet H; niedriges Alkyl; niedriges Alkenyl; Aryl - niedriges Alkyl;
-(CH₂)ₘ(CHR⁶¹)ₛOR⁵⁷; -(CH₂)ₘ(CHR⁶¹)ₛNR³⁴R⁶³;
-(CH₂)ₘ(CHR⁶¹)ₛOCONR⁷⁵R⁸²; -(CH₂)ₘ(CHR⁶¹)ₛNR²⁰CONR⁷⁸R⁸²;
-(CH₂)ₒ(CHR⁶¹)ₛCOR⁶⁴; -(CH₂)ₒ(CHR⁶¹)COOR⁵⁷;
oder -(CH₂)ₒ(CHR⁶¹)ₛCONR⁵⁸R⁵⁹;
R⁵⁶ bedeutet H; niedriges Alkyl; niedriges Alkenyl; Aryl - niedriges Alkyl;
-(CH₂)ₘ(CHR⁶¹)ₛOR⁵⁷; -(CH₂)ₘ(CHR⁶¹)ₛNR³⁴R⁶³;
-(CH₂)ₘ(CHR⁶¹)ₛOCONR⁷⁵R⁸²; -(CH₂)ₘ(CHR⁶¹)ₛNR²⁰CONR⁷⁸R⁸²;
-(CH₂)ₒ(CHR⁶¹)ₛ-COR⁶⁴; oder -(CH₂)ₒ(CHR⁶¹)ₛCONR⁵⁸R⁵⁹;
R⁵⁷ bedeutet H; niedriges Alkyl; niedriges Alkenyl; Aryl niedriges Alkyl; oder Heteroaryl niedriges Alkyl;
R⁵⁸ bedeutet H; niedriges Alkyl; niedriges Alkenyl; Aryl; Heteroaryl; Aryl - niedriges Alkyl; oder Heteroaryl - niedriges Alkyl;
R⁵⁹ bedeutet H; niedriges Alkyl; niedriges Alkenyl; Aryl; Heteroaryl; Aryl - niedriges Alkyl; oder Heteroaryl - niedriges Alkyl; oder
R⁵⁸ und R⁵⁹ können gemeinsam formen: -(CH₂)₂-₆-; -(CH₂)₂O(CH₂)₂-; -(CH₂)₂S(CH₂)₂-; oder -(CH₂)₂NR⁵⁷(CH₂)₂-;
R⁶⁰ bedeutet H; niedriges Alkyl; niedriges Alkenyl; Aryl; oder Aryl - niedriges Alkyl;
R⁶¹ bedeutet Alkyl; Alkenyl; Aryl; Heteroaryl; Aryl - niedriges Alkyl; Heteroaryl - niedriges Alkyl; -(CH₂)ₘOR⁵⁵; -(CH₂)ₘNR³³R³⁴; -(CH₂)ₘOCONR⁷⁵R⁸²; -(CH₂)ₘNR²⁰CONR⁷⁸R⁸²; -(CH₂)ₒCOOR³⁷; -(CH₂)ₒNR⁵⁸R⁵⁹; oder -(CH₂)ₒPO(COR⁶⁰)₂;
R⁶² bedeutet niedriges Alkyl; niedriges Alkenyl; Aryl, Heteroaryl; oder Aryl - niedriges Alkyl;
R⁶³ bedeutet H; niedriges Alkyl; niedriges Alkenyl; Aryl, Heteroaryl; Aryl - niedriges Alkyl; Heteroaryl - niedriges Alkyl;
-COR⁶⁴ ; -COOR⁵⁷; -CONR⁵⁸R⁵⁹; -SO₂R⁶²; oder -PO(OR⁶⁰)₂; oder
R³⁴ und R⁶³ können gemeinsam formen: -(CH₂)₂-₆-; -(CH₂)₂O(CH₂)₂-, -(CH₂)₂S(CH₂)₂-; oder -(CH₂)₂NR⁵⁷(CH₂)₂-;
R⁶⁴ bedeutet H; niedriges Alkyl; niedriges Alkenyl; Aryl; Heteroaryl; Aryl - niedriges Alkyl; Heteroaryl - niedriges Alkyl; -(CH₂)ₚ(CHR⁶¹)ₛOR⁶⁵; -(CH₂)ₚ(CHR⁶¹)ₛSR⁶⁶; oder
-(CH₂)ₚ(CHR⁶¹)ₛNR³⁴R⁶³; -(CH₂)ₚ(CHR⁶¹)ₛOCONR⁷⁵R⁸²,
-(CH₂) ₚ(CHR⁶¹)ₛNR²⁰CONR⁷⁸R⁸²;
R⁶⁵ bedeutet H; niedriges Alkyl; niedriges Alkenyl; Aryl, Aryl - niedriges Alkyl; Heteroaryl - niedriges Alkyl; -COR⁵⁷; -COOR⁵⁷; oder -CONR⁵⁸R⁵⁹;
R⁶⁶ bedeutet H; niedriges Alkyl; niedriges Alkenyl; Aryl; Aryl - niedriges Alkyl; Heteroaryl - niedriges Alkyl; oder -CONR⁵⁸R⁵⁹;
m ist 2 - 4, o ist 0 - 4; p ist 1 - 4; q ist 0 - 2; r ist 1 oder 2; s ist 0 oder 1;
Z ist eine Kette von 12 α-Aminosäureresten, wobei die Positionen der besagten Aminosäurereste in dieser Kette von der N-terminalen Aminosäure an gezählt werden, wobei diese Aminosäurereste, je nach der Position in den Ketten, Gly oder Pro sind oder ein Rest vom Typ
C: -NR²⁰CH(R⁷²)CO-;
D: -NR²⁰CH(R⁷³)CO-;
E: -NR²⁰CH(R⁷⁴)CO-;
F: -NR²⁰CH(R⁸⁴)CO-; und
H: -NR²⁰-CH(CO-)-(CH₂)₄₋₇-CH(CO-)-NR²⁰-;
-NR²⁰-CH(CO-)-(CH₂)ₚSS (CH₂)ₚ-CH(CO-)-NR²⁰-;
-NR²⁰-CH(CO-)-(-(CH₂)ₚNR²⁰CO(CH₂)ₚCH(CO-)-NR²⁰-; und
-NR²⁰-CH(CO-)-(-(CH₂)ₚNR²⁰CONR²⁰(CH₂)ₚCH(CO-)-NR²⁰-;
I: -NR⁸⁶CH₂CO-;
K: -NR⁸⁷CH₂CO-;
R⁷² bedeutet H, niedriges Alkyl; niedriges Alkenyl; -(CH₂)ₚ(CHR⁶¹)ₛOR⁸⁵; oder -(CH₂)ₚ(CHR⁶¹)ₛSR⁸⁵;
R⁷³ bedeutet -(CH₂)ₒR⁷⁷; -(CH₂)ᵣO(CH₂)ₒR⁷⁷; -(CH₂)ᵣS(CH₂)ₒR⁷⁷; oder -(CH₂)ᵣNR²⁰(CH₂)ₒR⁷⁷;
R⁷⁴ bedeutet -(CH₂)ₚNR⁷⁸R⁷⁹; -(CH₂)ₚNR⁷⁷R⁸⁰; -(CH₂)ₚC(=NR⁸⁰)NR⁷⁸R⁷⁹;
-(CH₂)ₚC(=NOR⁵⁰)NR⁷⁸R⁷⁹;
-(CH₂)ₚC(=NNR⁷⁸R⁷⁹)NR⁷⁸R⁷⁹; -(CH₂)ₚNR⁸⁰C(=NR⁸⁰) NR⁷⁸R⁷⁹;
-(CH₂)ₚN=C(NR⁷⁸R⁸⁰)NR⁷⁹R⁸⁰; -(CH₂)ₚC₆H₄NR⁷⁸R⁷⁹; -(CH₂)ₚC₆H₄NR⁷⁷R⁸⁰;
-(CH₂)ₚC₆H₄C(=NR⁸⁰)NR⁷⁸R⁷⁹; -(CH₂)ₚC₆H₄C(=NOR⁵⁰)NR⁷⁸R⁷⁹;
-(CH₂)ₚC₆H₄C(=NNR⁷⁸R⁷⁹)NR⁷⁸R⁷⁹; -(CH₂)ₚC₆H₄NR⁸⁰C(=NR⁸⁰) NR⁷⁸R⁷⁹;
-(CH₂)ₚC₆H₄N=C(NR⁷⁸R⁸⁰)NR⁷⁹R⁸⁰; -(CH₂)ᵣO(CH₂)ₘNR⁷⁸R⁷⁹; -(CH₂)ᵣO(CH₂)ₘNR⁷⁷R⁸⁰;
-(CH₂)ᵣO(CH₂)ₚC(=NR⁸⁰)NR⁷⁸R⁷⁹; -(CH₂)ᵣO(CH₂)ₚC(=NOR⁵⁰)NR⁷⁸R⁷⁹;
-(CH₂)ᵣO(CH₂)ₚC(=NNR⁷⁸R⁷⁹)NR⁷⁸R⁷⁹; -(CH₂)ᵣO(CH₂)ₘNR⁸⁰C(=NR⁷⁸)NR⁷⁸R⁷⁹;
-(CH₂)ᵣO(CH₂)ₘN=C(NR⁷⁸R⁸⁰)NR⁷⁹R⁸⁰; -(CH₂)ᵣO(CH₂)ₚC₆H₄CNR⁷⁸R⁷⁹;
-(CH₂)ᵣO(CH₂)ₚC₆H₄C(=NR⁸⁰)NR⁷⁸R⁷⁹; -(CH₂)ᵣO(CH₂)ₚC₆H₄C(=NOR⁵⁰)NR⁷⁸R⁷⁹;
-(CH₂)ᵣO(CH₂)ₚC₆H₄C(=NNR⁷⁸R⁷⁹)NR⁷⁸R⁷⁹,
-(CH₂)ᵣO(CH₂)ₚC₆H₄NR⁸⁰C(=NR⁸⁰)NR⁷⁸R⁷⁹; -(CH₂)ᵣS(CH₂)ₘNR⁷⁸R⁷⁹;
-(CH₂)ᵣS(CH₂)ₘNR⁷⁷R⁸⁰; -(CH₂)ᵣS(CH₂)ₚC(=NR⁸⁰)NR⁷⁸R⁷⁹;
-(CH₂)ᵣS(CH₂)ₚC(=NOR⁵⁰)NR⁷⁸R⁷⁹; -(CH₂)ᵣS(CH₂)ₚC(=NNR⁷⁸R⁷⁹)NR⁷⁸R⁷⁹;
-(CH₂)ᵣS(CH₂)ₘNR⁸⁰C(=NR⁸⁰)NR⁷⁸R⁷⁹; -(CH₂)ᵣS(CH₂)ₘN=C(NR⁷⁸R⁸⁰)NR⁷⁹R⁸⁰;
-(CH₂)ᵣS(CH₂)ₚC₆H₄CNR⁷⁸R⁷⁹; -(CH₂)ᵣS(CH₂)ₚC₆H₄C(=NR⁸⁰)NR⁷⁸R⁷⁹;
-(CH₂)ᵣS(CH₂)ₚC₆H₄C(=NOR⁵⁰)NR⁷⁸R⁷⁹; -(CH₂)ᵣS(CH₂)ₚC₆H₄C(=NNR⁷⁸R⁷⁹)NR⁷⁸R⁷⁹;
-(CH₂)ᵣS(CH₂)ₚC₆H₄NR⁸⁰C(=NR⁸⁰)NR⁷⁸R⁷⁹; -(CH₂)ₚNR⁸⁰COR⁶⁴;
-(CH₂)ₚNR⁸⁰COR⁷⁷; -(CH₂)ₚNR₈₀CONR⁷⁸R⁷⁹; oder -(CH₂)ₚC₆H₄NR⁸⁰CONR⁷⁸R⁷⁹;
R⁷⁵ bedeutet niedriges Alkyl; niedriges Alkenyl; oder Aryl - niedriges Alkyl; oder
R³³ und R⁷⁵ können gemeinsam formen: -(CH₂)₂₋₆-; -(CH₂)₂O(CH₂)₂-; -(CH₂)₂S(CH₂)₂-; oder -(CH₂)₂NR⁵⁷(CH₂)₂-; oder
R⁷⁵ und R⁸² können gemeinsam formen: -(CH₂)₂₋₆-; -(CH₂)₂O(CH₂)₂-; -(CH₂)₂S(CH₂)₂-; oder -(CH₂)₂NR⁵⁷(CH₂)₂-;
R⁷⁷ bedeutet R⁸⁸; oder eine der Heteroarylgruppen der folgenden Formeln 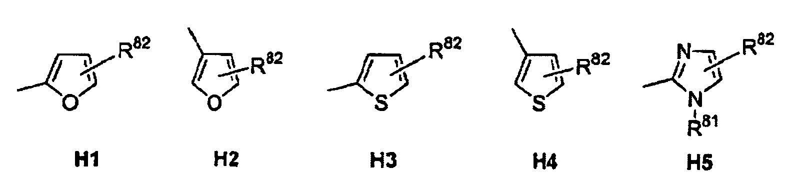 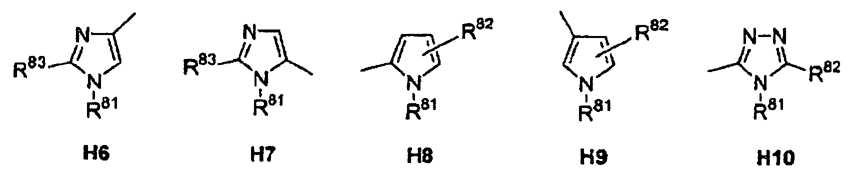 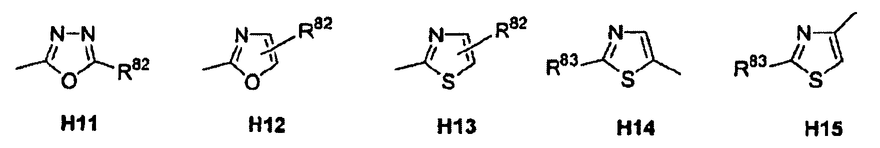 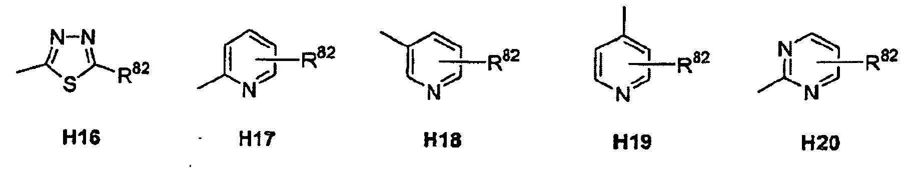 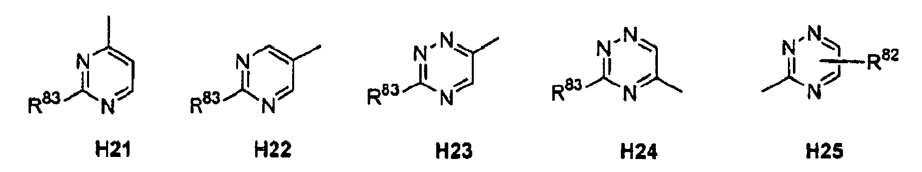 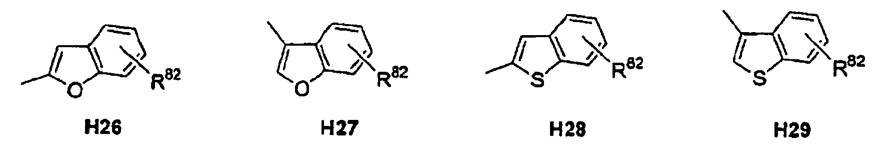 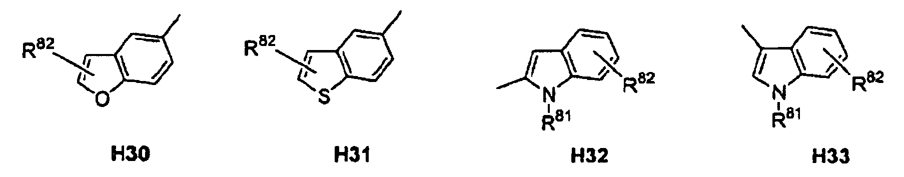 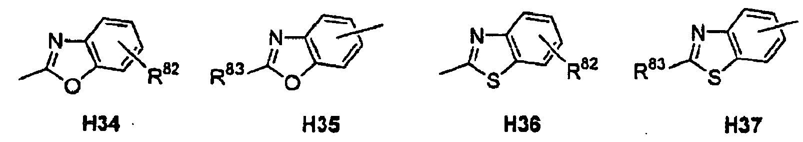 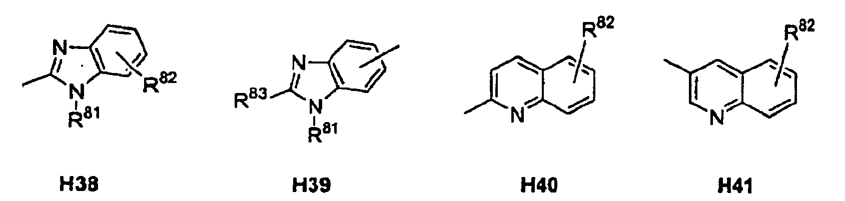 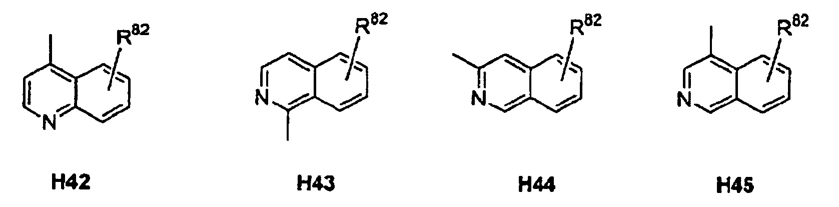 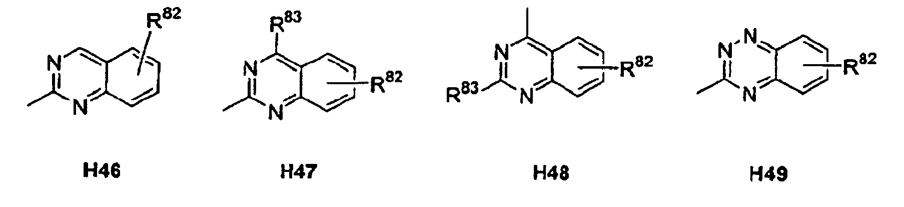 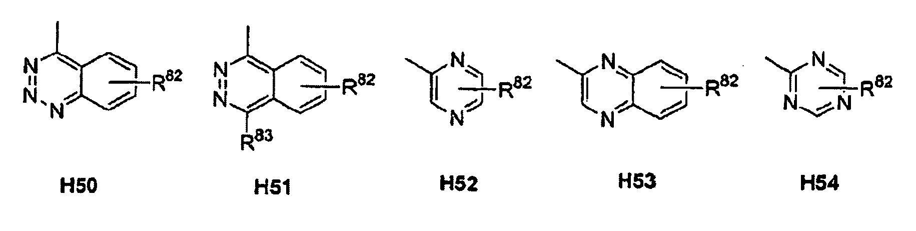
R⁷⁸ bedeutet H; niedriges Alkyl; Aryl; oder Aryl - niedriges Alkyl;
R⁷⁸ und R⁸² können gemeinsam formen: -(CH₂)₂₋₆-; -(CH₂)₂O(CH₂)₂-; -(CH₂)₂S(CH₂)₂-; oder -(CH₂)₂NR⁵⁷(CH₂)₂-;
R⁷⁹ bedeutet H; niedriges Alkyl; Aryl; oder Aryl - niedriges Alkyl; oder
R⁷⁸ und R⁷⁹ können gemeinsam formen -(CH₂)₂₋₇-; -(CH₂)₂O(CH₂)₂-; oder -(CH₂)₂NR⁵⁷(CH₂)₂-;
R⁸⁰ bedeutet H; oder niedriges Alkyl;
R⁸¹ bedeutet H; niedriges Alkyl; oder Aryl- niedriges Alkyl;
R⁸² bedeutet H; niedriges Alkyl; Aryl; Heteroaryl; oder Aryl - niedriges Alkyl;
R³³ und R⁸² können gemeinsam formen: -(CH₂)₂₋₆-; -(CH₂)₂O(CH₂)₂-; -(CH₂)₂S(CH₂)₂-; oder -(CH₂)₂NR⁵⁷(CH₂)₂-;
R⁸³ bedeutet H; niedriges Alkyl; Aryl; oder -NR⁷⁸R⁷⁹;
R⁸⁴ bedeutet -(CH₂)ₘ(CHR⁶¹)ₛOR⁷⁸; -(CH₂)ₘ(CHR⁶¹)ₛSR⁷⁸; -(CH₂)ₚCONR⁷⁸R⁷⁹;
-(CH₂)ₚNR⁸⁰CONR⁷⁸R⁷⁹; -(CH₂)ₚC₆H₄CONR⁷⁸R⁷⁹ oder
-(CH₂)ₚC₆H₄NR⁸⁰CONR⁷⁸R⁷⁹;
R⁸⁵ bedeutet niedriges Alkyl; oder niedriges Alkenyl;
R⁸⁶ bedeutet R⁷⁴; -[(CH₂)ᵤ-X]ₜ(CH₂)ᵥNR⁷⁸R⁷⁹; -[(CH₂)ᵤ -X]ₜ-(CH₂)ᵥ-C(=NR⁸⁰)NR⁷⁸R⁷⁹;
X ist -O-, -NR²⁰-, -S-, -OCOO-, u ist 1- 3, t ist 1 - 6, v ist 1 - 3;
R⁸⁷ bedeutet R⁸⁴; -[(CH₂)ᵤ-X]ₜ-(CH₂)ᵥOR⁷⁸; -[(CH₂)ᵤ -X]ₜ-(CH₂)ᵥ-CONR⁷⁸R⁷⁹; -[(CH₂)ᵤ -X]ₜ-(CH₂)ᵥ-NR⁸⁰CONR⁷⁸R⁷⁹; -[(CH₂)ᵤ-X]ₜ(CH₂)ᵥ SR⁷⁸;
X ist -O-, -NR²⁰-, -S-, -OCOO-, u ist 1- 3, t ist 1 - 6, v ist 1 - 3;
R⁸⁸ bedeutet Phenyl, p-Hydrxyphenyl, 2-Naphthyl, 1-Naphthyl, 4-Chlorophenyl,
3-Chlorophenyl, 2-Chlorophenyl, 3,4-Dichlorophenyl, 4-Fluorophenyl,
3-Fluorophenyl, 2-Fluorophenyl, p-Benzyloxyphenyl, p-Biphenyl oder
p-Benzoylphenyl.
mit der Bedingung, dass in der besagten Kette der 12 α-Aminosäurereste Z die Aminosäurereste in den Positionen 1 bis 12 sind:
- P1: vom Typ C oder vom Typ D oder vom Typ E oder vom Typ F, oder der Rest ist Pro;
- P2: vom Typ E oder vom Typ D;
- P3: vom Typ C, oder der Rest ist Pro;
- P4: vom Typ E oder vom Typ F oder vom Typ I oder vom Typ K;
- P5: vom Typ E oder vom Typ D oder vom Typ C oder vom Typ I oder vom Typ K oder vom Typ F, oder der Rest ist Gly oder Pro;
- P6: vom Typ E oder vom Typ F oder vom Typ I oder vom Typ K oder vom Typ D, oder der Rest ist Gly;
- P7: vom Typ E oder vom Typ F oder vom Typ I oder vom Typ C;
- P8: vom Typ D oder vom Typ C oder der Rest ist Pro;
- P9: vom Typ E oder vom Typ D oder vom Typ F;
- P10: vom Typ D oder vom Typ C, oder der Rest ist Pro;
- P11: vom Typ E oder vom Typ D oder vom Typ C; und
- P12: vom Typ C oder vom Typ D oder vom Typ E oder vom Typ F, oder der Rest ist Pro; oder
- P4 und P9 und/ oder P2 und P11 können gemeinsam eine Gruppe vom Typ H bilden;
und in P6 und P7 sind auch D-lsomere möglich;
mit der weiteren Bedingung, dass diese Kette der 12 α-Aminosäurereste mindestens einen Rest vom Typ I oder vom Typ K enthält;
und deren pharmazeutisch akzeptablen Salze.

2. Verbindungen nach Anspruch 1, in welchen die α-Aminosäurereste in den Positionen 1 bis 12 der Kette Z bedeuten:
- P1: vom Typ C oder vom Typ D oder vom Typ E oder vom Typ F,
- P2: vom Typ D oder vom Typ E;
- P3: vom Typ C;
- P4: vom Typ E oder vom Typ I oder vom Typ F;
- P5: vom Typ E oder vom Typ I oder vom Typ F;
- P6: vom Typ E oder vom Typ I oder vom Typ D;
- P7: vom Typ E oder vom Typ I oder vom Typ C;
- P8: vom Typ D;
- P9: vom Typ E;
- P10: vom Typ D oder vom Typ C,
- P11: vom Typ E oder vom Typ D oder vom Typ C; und
- P12: vom Typ C oder vom Typ D oder vom Typ E oder vom Typ F;
- in P6 und P7 sind auch D-lsomere möglich;
mit der Bedingung, dass mindestens einer der Aminosäurereste vom Typ I ist.

3. Verbindungen nach Anspruch 2, in welchen die α-Aminosäurereste in den Positionen 1 bis 12 der Kette Z bedeuten:
- P1: Leu; Tyr oder Arg;
- P2: Arg; oder Trp;
- P3: Leu;
- P4: Lys; hArg; (BA)G; oder Gln;
- P5: Lys; Gln; hArg; oder (PeA)G;
- P6: Arg, Trp, hArg; (EGU)G;
- (EA)G; (PrA)G; (PeA)G oder (BA)G;
- P7: Arg; (PeA)G; oder Val;
- P8: Trp; oder Bip;
- P9: Lys; Arg; oder hArg;
- P10: Tyr;
- P11: Arg; oder Tyr; und
- P12 Val; oder Arg
mit der Bedingung, dass
- der Aminosäurerest in P4 (BA)G ist; und/oder
- der Aminosäurerest in P5 (PeA)G ist; und/oder
- der Aminosäurerest in P6 (EGU)G oder (EA)G oder (PrA)G oder (PeA)G oder (BA)G ist; und/oder
- der Aminosäurerest in P7 (PeA)G ist.

4. Verbindung der Formel I nach Anspruch 1, in welcher die Template (Grundstruktur) ^{D}Pro - ^{L}pro ist, und die Aminosäurereste in den Positionen 1 bis 12 bedeuten:
- P1:Leu;
- P2: Arg;
- P3: Leu;
- P4: Lys;
- P5:Lys;
- P6: (EA)G;
- P7: Arg;
- P8: Trp;
- P9: Lys;
- P10: Tyr;
- P11: Arg; und
- P12:Val

5. Verbindung der Formel I nach Anspruch 1, in welcher die Template ^{D}pro- ^{L}Pro ist, und die Aminosäurereste in den Positionen 1 bis 12 bedeuten:
- P1:Leu;
- P2: Arg;
- P3: Leu;
- P4: hArg;
- P5: hArg;
- P6: (EGU)G;
- P7: Arg;
- P8:Trp;
- P9: hArg;
- P10: Tyr;
- P11: Arg; und
- P12:Val

6. Verbindung der Formel I nach Anspruch 1, in welcher die Template ^{D}Pro- ^{L}Pro ist, und die Aminosäurereste in den Positionen 1 bis 12 bedeuten:
- P1:Leu;
- P2: Arg;
- P3: Leu;
- P4: Lys;
- P5: Lys;
- P6: (PrA)G;
- P7: Arg;
- P8: Trp;
- P9: Lys;
- P10: Tyr;
- P11: Arg; und
- P12:Val

7. Verbindung der Formel I nach Anspruch 1, in welcher die Template ^{D}Pro- ^{L}Pro ist, und die Aminosäurereste in den Positionen 1 bis 12 bedeuten:
- P1:Leu;
- P2: Arg;
- P3: Leu;
- P4: Lys;
- P5: Lys;
- P6: (BA)G;
- P7: Arg;
- P8: Bip;
- P9: Lys;
- P10: Tyr;
- P11: Arg; und
- P12:Val

8. Verbindung der Formel I nach Anspruch 1, in welcher die Template ^{D}Pro- ^{L}Pro ist, und die Aminosäurereste in den Positionen 1 bis 12 bedeuten:
- P1: Leu;
- P2: Arg;
- P3: Leu;
- P4: (BA)G;
- P5: Lys;
- P6: (BA)G;
- P7: Arg;
- P8: Bip;
- P9: Lys;
- P10:Tyr;
- P11: Arg; und
- P12:Val

9. Verbindung der Formel I nach Anspruch 1, in welcher die Template ^{D}Pro - ^{L}Pro ist, und die Aminosäurereste in den Positionen 1 bis 12 bedeuten:
- P1:Leu;
- P2: Arg;
- P3: Leu;
- P4: Lys;
- P5: Lys;
- P6: (PrA)G;
- P7: Arg;
- P8: Bip;
- P9: Lys;
- P10:Tyr;
- P11: Arg; und
- P12:Val

10. Verbindung der Formel I nach Anspruch 1, in welcher die Template ^{D}pro- ^{L}Pro ist, und die Aminosäurereste in den Positionen 1 bis 12 bedeuten:
- P1:Arg;
- P2: Trp;
- P3: Leu;
- P4: Lys;
- P5: Lys;
- P6: Arg;
- P7: (PeA)G;
- P8:Trp;
- P9: Lys;
- P10: Tyr;
- P11: Tyr; und
- P12:Val

11. Verbindung der Formel I nach Anspruch 1, in welcher die Template ^{D}Pro - ^{L}Pro ist, und die Aminosäurereste in den Positionen 1 bis 12 bedeuten:
- P1:Arg;
- P2: Trp;
- P3: Leu;
- P4: Gln;
- P5: (PeA)G;
- P6: Arg;
- P7: Arg;
- P8: Trp;
- P9: Lys;
- P10: Tyr;
- P11: Tyr; und
- P12 : Arg

12. Verbindung der Formel I nach Anspruch 1, in welcher die Template ^{D}Pro- ^{L}Pro ist, und die Aminosäurereste in den Positionen 1 bis 12 bedeuten:
- P1: Arg;
- P2: Trp;
- P3: Leu;
- P4: Lys;
- P5: (PeA)G;
- P6: Arg;
- P7: Arg;
- P8: Trp;
- P9: Lys;
- P10: Tyr;
- P11: Tyr; und
- P12 : Val

13. Verbindung der Formel I nach Anspruch 1, in welcher die Template ^{D}Pro - ^{L}Pro ist, und die Aminosäurereste in den Positionen 1 bis 12 bedeuten:
- P1:Thr;
- P2: Trp;
- P3: Leu;
- P4: Lys;
- P5: (PeA)G;
- P6: Arg;
- P7: Arg;
- P8: Trp;
- P9: Lys;
- P10: Tyr;
- P11: Tyr; und
- P12 : Arg

14. Verbindung der Formel I nach Anspruch 1, in welcher die Template ^{D}Pro - ^{L}Pro ist, und die Aminosäurereste in den Positionen 1 bis 12 bedeuten:
- P1: Arg;
- P2: Trp;
- P3: Leu;
- P4: Gln;
- P5: Lys;
- P6: Arg;
- P7: (PeA)G;
- P8: Trp;
- P9: Lys;
- P10: Tyr;
- P11: Tyr; und
- P12 : Arg

15. Verbindung der Formel I nach Anspruch 1, in welcher die Template ^{D}Pro- ^{L}Pro ist, und die Aminosäurereste in den Positionen 1 bis 12 bedeuten:
- P1:Thr;
- P2: Trp;
- P3: Leu;
- P4: Lys;
- P5: (PeA)G;
- P6: Arg;
- P7: Arg;
- P8: Trp;
- P9: Lys;
- P10:Tyr;
- P11: Tyr; und
- P12 : Arg

16. Enantiomere der Verbindungen der Formel I gemäss der Definition nach Anspruch 1.

17. Verbindungen nach einem der Ansprüche 1 bis 16 für die Verwendung als therapeutisch aktive Wirkstoffe.

18. Verbindungen nach Anspruch 17, welche eine antibakterielle Aktivität aufweisen.

19. Pharmazeutische Zusammensetzung, welche eine Verbindung nach einem der Ansprüche 1 bis 17 und einen pharmazeutisch inerten Träger enthält.

20. Zusammensetzungen nach Anspruch 19 in einer Form, welche für die orale, topische, transdermale Verabreichung, für die Injektion, für die bukkale, transmukosale, pulmonale Verabreichung oder für die Inhalation geeignet ist.

21. Zusammensetzungen nach Anspruch 18 oder 19 in Form von Tabletten, Dragees, Kapseln, Lösungen, Flüssigkeiten, Gels, Pflaster, Cremen, Salben, Sirup, Emulsionen, Suspensionen, Spray, Zerstäuber oder Zäpfchen.

22. Verwendung der Verbindungen nach einem der Ansprüche 1 bis 16 für die Herstellung eines Medikaments zur Behandlung oder zur Vorbeugung von Infektionen oder mit solchen Infektionen verbundenen Krankheiten, wobei diese Krankheit insbesondere die zystische Fibrose ist.

23. Verwendung der Verbindungen nach einem der Ansprüche 1 bis 16 als Desinfektionsmittel oder Konservierungsmittel für Nahrungsmittel, Kosmetika, Medikamente oder andere Nährstoff-enthaltende Materialien.

24. Verfahren für die Herstellung von Verbindungen nach einem der Ansprüche 1 bis 15 umfassend
(a) Verbinden eines geeigneten funktionellen festen Trägers mit einem in geeigneter Weise N-geschützten Derivat von der Aminosäure, welche in dem gewünschten Endprodukt in der Position 5, 6 oder 7 ist, wobei jegliche funktionelle Gruppe, welche in dem besagten N-geschützten Aminosäurederivat anwesend sein kann, ebenfalls in geeigneter Weise geschützt ist;
(b) Entfernen der N-Schutzgruppe von dem derart erhaltenen Produkt;
(c) Verbinden des derart erhaltenen Produkts mit einem in geeigneter Weise N-geschützten Derivat von der Aminosäure, welche in dem gewünschten Endprodukt eine Position näher zum N-terminalen Aminosäurerest ist, wobei jegliche funktionelle Gruppe, welche in dem besagten N-geschützten Aminosäurederivat anwesend sein kann, ebenfalls in geeigneter Weise geschützt ist;
(d) Entfernen der N-Schutzgruppe von dem derart erhaltenen Produkt;
(e) Wiederholen der Stufen (c) und (d) bis der N-terminale Aminosäurerest eingeführt ist;
(f) Verbinden des derart erhaltenen Produkts mit
(fa) einem in geeigneter Weise N-geschützten Derivat von ^{L}Pro oder ^{D}pro;
(fb) Entfernen der N-Schutzgruppe von dem derart erhaltenen Produkt; und
(fc) Verbinden des derart erhaltenen Produkts mit einem in geeigneter Weise N-geschützten Derivat von ^{D}Pro resp. ^{L}Pro;
(g) Entfernen der N-Schutzgruppe von dem in der Stufe (fc) erhaltenen Produkt;
(h) Verbinden des derart erhaltenen Produkts mit einem in geeigneter Weise N-geschützten Derivat von der Aminosäure, welche in dem gewünschten Endprodukt in der Position 12 ist, wobei jegliche funktionelle Gruppe, welche in dem besagten N-geschützten Aminosäurederivat anwesend sein kann, ebenfalls in geeigneter Weise geschützt ist;
(i) Entfernen der N-Schutzgruppe von dem derart erhaltenen Produkt;
(j) Verbinden des derart erhaltenen Produkts mit einem in geeigneter Weise N-geschützten Derivat von der Aminosäure, welche in dem gewünschten Endprodukt eine Position von Position 12 entfernt ist, wobei jegliche funktionelle Gruppe, welche in dem besagten N-geschützten Aminosäurederivat anwesend sein kann, ebenfalls in geeigneter Weise geschützt ist;
(k) Entfernen der N-Schutzgruppe von dem derart erhaltenen Produkt;
(l) Wiederholen der Stufen (j) und (k) bis alle Aminosäurereste eingeführt sind;
(m) falls gewünscht, selektives Entschützen einer funktionellen Gruppe oder mehrerer funktionellen Gruppen, welche in dem Molekül anwesend sind, und geeignetes Substituieren der derart freien Reaktionsgruppe(n);
(o) Lösen des derart erhaltenen Produkts von dem festen Träger;
(p) Zyklisieren des von dem festen Träger abgespaltenen Produkts;
(q) falls gewünscht, Bilden einer oder zwei zwischenstrangiger Bindungen zwischen Seitenketten der geeigneten Aminosäurereste an gegenüberliegenden Positionen des β-Strang-Bereichs;
(r) Entfernen sämtlicher Schutzgruppen, welche an den funktionellen Gruppen aller Kettenglieder der Aminosäurereste anwesend sind, und falls gewünscht, sämtlicher Schutzgruppe(n), welche zusätzlich in dem Molekül anwesend sein kann;
(s) falls gewünscht, Guanidinylieren sämtlicher Aminogruppen der Seitenketten, welche in der Kette der Aminosäurereste anwesend sind; und
(t) falls gewünscht, Umsetzen des derart erhaltenen Produkts in ein pharmazeutisch akzeptables Salz oder Umsetzen eines derart erhaltenen pharmazeutisch akzeptablen oder nicht-akzeptablen Salzes in die entsprechende freie Verbindung der Formel I oder in ein anderes pharmazeutisch akzeptables Salz.

25. Verfahren für die Herstellung von Verbindungen nach einem der Ansprüche 1 bis 15 umfassend
(a') Verbinden eines geeigneten funktionellen festen Trägers
(a'a) Verbinden des geeigneten funktionellen festen Trägers mit einem in geeigneter Weise N-geschützten Derivat von ^{L}Pro oder ^{D}Pro
(a'b) Entfernen der N-Schutzgruppe von dem derart erhaltenen Produkt; und
(a'c) Verbinden des derart erhaltenen Produkts mit einem in geeigneter Weise N-geschützten Derivat von ^{D}Pro resp. ^{L}Pro;
(b') Entfernen der N-Schutzgruppe von dem in der Stufe (a'c) erhaltenen Produkt;
(c') Verbinden des derart erhaltenen Produkts mit einem in geeigneter Weise N-geschützten Derivat von der Aminosäure, welche in dem gewünschten Endprodukt eine Position näher zum N-terminalen Aminosäurerest ist, wobei jegliche funktionelle Gruppe, welche in dem besagten N-geschützten Aminosäurederivat anwesend sein kann, ebenfalls in geeigneter Weise geschützt ist;
(d') Entfernen der N-Schutzgruppe von dem derart erhaltenen Produkt;
(e') Verbinden des derart erhaltenen Produkts mit einem in geeigneter Weise N-geschützten Derivat von der Aminosäure, welche in dem gewünschten Endprodukt eine Position von Position 12 entfernt ist, wobei jegliche funktionelle Gruppe, welche in dem besagten N-geschützten Aminosäurederivat anwesend sein kann, ebenfalls in geeigneter Weise geschützt ist;
(f') Entfernen der N-Schutzgruppe von dem derart erhaltenen Produkt;
(g') Wiederholen der Stufen (e') und (f') bis alle Aminosäurereste eingeführt sind;
(h') falls gewünscht, selektives Entschützen einer funktionellen Gruppe oder mehrerer funktionellen Gruppen, welche in dem Molekül anwesend sind, und geeignetes Substituieren der derart freien Reaktionsgruppe(n);
(i') Lösen des derart erhaltenen Produkts von dem festen Träger;
(j') Zyklisieren des von dem festen Träger abgespaltenen Produkts;
(k') falls gewünscht, Bilden einer oder zwei zwischenstrangiger Bindungen zwischen Seitenketten der geeigneten Aminosäurereste an gegenüberliegenden Positionen des β-Strang-Bereichs;
(l') Entfernen sämtlicher Schutzgruppen, welche an den funktionellen Gruppen aller Kettenglieder der Aminosäurereste anwesend sind, und falls gewünscht, sämtlicher Schutzgruppe(n), welche zusätzlich in dem Molekül anwesend sein kann;
(m') falls gewünscht, Guanidinylieren sämtlicher Aminogruppen der Seitenketten, welche in der Kette der Aminosäurereste anwesend sind; und
(n') falls gewünscht, Umsetzen des derart erhaltenen Produkts in ein pharmazeutisch akzeptables Salz oder Umsetzen eines derart erhaltenen pharmazeutisch akzeptablen oder nicht-akzeptablen Salzes in die entsprechende freie Verbindung der Formel I oder in ein anderes pharmazeutisch akzeptables Salz.

26. Verfahren nach Anspruch 24 oder 25, worin jedoch ein Aminosäurerest des Typs I oder des Typs K eingeführt wird durch Verbinden mit einem Acetylierungsmittel, welches eine Abgangsgruppe umfasst, gefolgt von einer nukleophilen Verschiebung mit einem Amin der Formel H₂NR⁸⁶ resp. H₂NR⁸⁷, welches nötigenfalls in geeigneter Weise geschützt wird.

27. Verfahren nach Anspruch 26, wobei das Acetylierungsmittel, welches die Abgangsgruppe enthält, Bromo, Chloro oder lodo Essigsäure ist.

28. Modifikation des Verfahrens nach einem der Ansprüche 24 bis 27 für die Herstellung der Verbindungen nach Anspruch 16, in welchen Enantiomere von allen chiralen Ausgangsmaterialien verwendet werden.

## Revendications

1. Composés de la formule générale 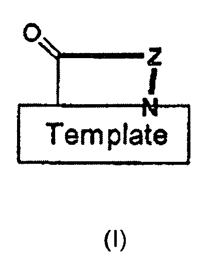 dans laquelle 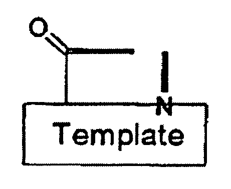 représente un groupe de la formule ^{D}Pro-^{L}Pro et ^{L}pro-^{D}Pro
R⁸ représente H ; Cl; F ; CF₃; NO₂; alkyle inférieur ; alcényle inférieur ; aryle ; aryl-alkyle inférieur ; -(CH₂)ₒ(CHR⁶¹)ₛOR⁵⁵; -(CH₂)ₒ(CHR⁶¹)ₛSR⁵⁶; -(CH₂)ₒ(CHR⁶¹)NR³³R³⁴;
-(CH₂)ₒ(CHR⁶¹)ₛOCONR³³R⁷⁵; -(CH₂)ₒ(CHR⁶¹)ₛNR²⁰CONR³³R⁸²;
-(CH₂)ₒ(CHR⁶¹)ₛ(COOR⁶¹)ₛCOOR⁵⁷; -(CH₂)ₒ(CHR⁶¹)ₛCONR⁵⁸R⁵⁹, -(CH₂)ₒ(CHR⁶¹)ₛPO(OR⁶⁰)₂;
-(CH₂)ₒ(CHR⁶¹)ₛSO₂R⁶²; ou -(CH₂)ₒ(CHR⁶¹)ₛCOR⁶⁴;
R²⁰ représente H ; alkyle ; alcényle; ou aryl-alkyle inférieur ;
R³³ représente H ; alkyle, alcényle; -(CH₂)ₘ(CHR⁶¹)ₛOR⁵⁵;
-(CH₂)ₘ(CHR⁶¹)ₛNR³⁴R⁶³; -(CH₂)ₘ(CHR⁶¹)ₛOCONR⁷⁵R⁸²;
-(CH₂)ₘ(CHR⁶¹)ₛNR²⁰CONR⁷⁸R⁸²; -(CH₂)ₒ(CHR⁶¹)ₛCOR⁶⁴;
-(CH₂)ₒ(CHR⁶¹)ₛCONR⁵⁸R⁵⁹, -(CH₂)ₒ(CHR⁶¹)ₛPO(OR⁶⁰)₂;
-(CH₂)ₒ(CHR⁶¹)ₛ SO₂R⁶²; ou -(CH₂)ₒ(CHR⁶¹)ₛC₆H₄R⁸;
R³⁴ représente H ; alkyle inférieur ; aryle, ou aryl-alkyle inférieur ;
R³³ et R³⁴ ensemble peuvent former : -(CH₂)₂₋₆-; -(CH₂)₂O(CH₂)₂-; -(CH₂)₂S(CH₂)₂-; ou -(CH₂)₂NR⁵⁷(CH₂)₂-;
R³⁷ représente H ; F ; Br ; Cl; NO₂; CF₃; alkyle inférieur ; -(CH₂)ₚ(CHR⁶¹)ₛOR⁵⁵;
-(CH₂)ₚ(CHR⁶¹)ₛNR³³R³⁴; -(CH₂)ₚ(CHR⁶¹)ₛOCONR³³R⁷⁵;
-(CH₂)ₚ(CHR⁶¹)ₛNR²⁰CONR³³R⁸²; -(CH₂)ₒ(CHR⁶¹)ₛCOOR⁵⁷;
-(CH₂)ₒ(CHR⁶¹)ₛCONR⁵⁸R⁵⁹; -(CH₂)ₒ(CHR⁶¹)ₛPO(OR⁶⁰)₂;
-(CH₂)ₒ(CHR⁶¹)ₛSO₂R⁶²; ou -(CH₂)ₒ(CHR⁶¹)ₛC₆H₄R⁸;
R⁵⁰ représente H ; alkyle inférieur ; ou aryl-alkyle inférieur ;
R⁵⁵ représente H ; alkyle inférieur ; alcényle inférieur ; aryl-alkyle inférieur ;
-(CH₂)ₘ(CHR⁶¹)ₛOR⁵⁷; -(CH₂)ₘ(CHR⁶¹)ₛNR³⁴R⁶³; -(CH₂)ₘ(CHR⁶¹)ₛOCONR⁷⁵R⁸²;
-(CH₂)ₘ(CHR⁶¹)ₛNR²⁰CONR⁷⁸R⁸²; -(CH₂)ₒ(CHR⁶¹)ₛCOR⁶⁴; -(CH₂)ₒ(CHR⁶¹)COOR⁵⁷; ou
-(CH₂)ₒ(CHR⁶¹)ₛCONR⁵⁸R⁵⁹;
R⁵⁶ représente H ; alkyle inférieur ; alcényle inférieur ; aryl-alkyle inférieur;
-(CH₂)ₘ(CHR⁶¹)ₛOR⁵⁷; -(CH₂)ₘ(CHR⁶¹)ₛNR³⁴R⁶³;
-(CH₂)ₘ(CHR⁶¹)ₛOCONR⁷⁵R⁸²; -(CH₂)ₘ(CHR⁶¹)ₛNR²⁰CONR⁷⁸R⁸²;
-(CH₂)ₒ(CHR⁶¹)ₛ-COR⁶⁴; ou -(CH₂)ₒ(CHR⁶¹)ₛCONR⁵⁸R⁵⁹;
R⁵⁷ représente H; alkyle inférieur ; alcényle inférieur ; aryle alkyle inférieur ; ou hétéroaryle alkyle inférieur ;
R⁵⁸ représente H; alkyle inférieur ; alcényle inférieur ; aryle ; hétéroaryle; aryl-alkyle inférieur ; ou hétéroaryl-alkyle inférieur ;
R⁵⁹ représente H ; alkyle inférieur ; alcényle inférieur ; aryle ; hétéroaryle ; aryl-alkyle inférieur ; ou hétéroaryl-alkyle inférieur ; ou
R⁵⁸ et R⁵⁹ ensemble peuvent former : -(CH₂)₂-₆-; -(CH₂)₂O(CH₂)₂-; -(CH₂)₂S(CH₂)₂-; ou -(CH₂)₂NR⁵⁷(CH₂)₂-;
R⁶⁰ représente H ; alkyle inférieur ; alcényle inférieur ; aryle ; ou aryl-alkyle inférieur;
R⁶¹ représente alkyle; alcényle; aryle ; hétéroaryle ; aryl-alkyle inférieur ; hétéroaryl-alkyle inférieur ; -(CH₂)ₘOR⁵⁵; -(CH₂)ₘNR³³R³⁴; -(CH₂)ₘOCONR⁷⁵R⁸²; -(CH₂)ₘNR²⁰CONR⁷⁸R⁸²;
-(CH₂)ₒCOOR³⁷; -(CH₂)ₒNR⁵⁸R⁵⁹; ou -(CH₂)ₒPO(COR⁶⁰)₂;
R⁶² représente alkyle inférieur ; alcényle inférieur ; aryle, hétéroaryle ; ou aryl-alkyle inférieur;
R⁶³ représente H ; alkyle inférieur ; alcényle inférieur ; aryle, hétéroaryle ; aryl-alkyle inférieur ; hétéroaryl-alkyle inférieur ;
-COR⁶⁴; -COOR⁵⁷; -CONR⁵⁸R⁵⁹; -SO₂R⁶²; ou -PO(OR⁶⁰)₂; ou
R³⁴ et R⁶³ ensemble peuvent former : -(CH₂)₂-₆-; -(CH₂)₂O(CH₂)₂-; -(CH₂)₂S(CH₂)₂-; ou -(CH₂)₂NR⁵⁷(CH₂)₂-;
R⁶⁴ représente H ; alkyle inférieur ; alcényle inférieur ; aryle; hétéroaryle ; aryl-alkyle inférieur ; hétéroaryl-alkyle inférieur;
-(CH₂)ₚ(CHR⁶¹)ₛOR⁶⁵; -(CH₂)ₚ(CHR⁶¹)ₛSR⁶⁶; ou -(CH₂)ₚ(CHR⁶¹)ₛNR³⁴R⁶³;
-(CH₂)ₚ(CHR⁶¹)ₛOCONR⁷⁵R⁸², -(CH₂)ₚ(CHR⁶¹)ₛNR²⁰CONR⁷⁸R⁸²;
R⁶⁵ représente H ; alkyle inférieur ; alcényle inférieur ; aryle, aryl-alkyle inférieur; hétéroaryl-alkyle inférieur ;
-COR⁵⁷; -COOR⁵⁷; ou -CONR⁵⁸R⁵⁹;
R⁶⁶ représente H ; alkyle intérieur; alcényle intérieur; aryle ; aryl-alkyle intérieur; hétéroaryl-alkyle inférieur ; ou -CONR⁵⁸R⁵⁹;
m est 2 - 4, 0 est 0 - 4 ; p est 1 - 4 ; q est 0 - 2 ; r est 1 ou 2 ; s est 0 ou 1 ;
Z est une chaîne de 12 résidus d'acides α-aminés, où les positions des dits résidus d'acides aminés dans ladite chaîne sont comptées à partir de l'acide aminé N-terminal, où ces résidus d'acides aminés sont, en fonction de leur position dans les chaînes, Gly ou Pro, ou un résidu du type
C: -NR²⁰CH(R⁷²)CO-;
D: -NR²⁰CH(R⁷³)CO-;
E: -NR²⁰CH(R⁷⁴)CO-;
F: -NR²⁰CH(R⁸⁴)CO-; et
H: -NR²⁰-CH(CO-)-(CH₂)₄₋₇-CH(CO-)-NR²⁰-;
-NR²⁰-CH(CO-)-(CH₂)ₚSS (CH₂)ₚ-CH(CO-)-NR²⁰-;
-NR²⁰-CH(CO-)-(-(CH₂)ₚNR²⁰CO(CH₂)ₚCH(CO-)-NR²⁰-; et
-NR²⁰-CH(CO-)-(-(CH₂)ₚNR²⁰CONR²⁰(CH₂)ₚCH(CO-)-NR²⁰-;
I : -NR⁸⁶CH₂CO-;
K: -NR⁸⁷CH₂CO-;
R⁷² représente H, alkyle inférieur ; alcényle inférieur; -(CH₂)ₚ(CHR⁶¹)ₛOR⁸⁵; ou -(CH₂)ₚ(CHR⁶¹)ₛSR⁸⁵;
R⁷³ représente -(CH₂)ₒR⁷⁷; -(CH₂)ᵣO(CH₂)ₒR⁷⁷; -(CH₂)ᵣS(CH₂)ₒR⁷⁷; ou -(CH₂)ᵣNR²⁰(CH₂)ₒR⁷⁷;
R⁷⁴ représente -(CH₂)ₚNR⁷⁸R⁷⁹; -(CH₂)ₚNR⁷⁷R⁸⁰; -(CH₂)ₚC(=NR⁸⁰)NR⁷⁸R⁷⁹;
-(CH₂)ₚC(=NOR⁵⁰)NR⁷⁸R⁷⁹;
-(CH₂)ₚC(=NNR⁷⁸R⁷⁹)NR⁷⁸R⁷⁹; -(CH₂)ₚNR⁸⁰C(=NR⁸⁰) NR⁷⁸R⁷⁹;
-(CH₂)ₚN=C(NR⁷⁸R⁸⁰)NR⁷⁹R⁸⁰; -(CH₂)ₚC₆H₄NR⁷⁸R⁷⁹; -(CH₂)ₚC₆H₄NR⁷⁷R⁸⁰;
-(CH₂)ₚC₆H₄C(=NR⁸⁰)NR⁷⁸R⁷⁹; -(CH₂)ₚC₆H₄C(=NOR⁵⁰)NR⁷⁸R⁷⁹;
-(CH₂)ₚC₆H₄C(=NNR⁷⁸R⁷⁹)NR⁷⁸R⁷⁹; -(CH₂)ₚC₆H₄NR⁸⁰C(=NR⁸⁰) NR⁷⁸R⁷⁹;
-(CH₂)ₚC₆H₄N=C(NR⁷⁸R⁸⁰)NR⁷⁹R⁸⁰; -(CH₂)ᵣO(CH₂)ₘNR⁷⁸R⁷⁹; -(CH₂)ᵣO(CH₂)ₘNR⁷⁷R⁸⁰;
-(CH₂)ᵣO(CH₂)ₚC(=NR⁸⁰)NR⁷⁸R⁷⁹; -(CH₂)ᵣO(CH₂)ₚC(=NOR⁵⁰)NR⁷⁸R⁷⁹;
-(CH₂)ᵣO(CH₂)ₚC(=NNR⁷⁸R⁷⁹)NR⁷⁸R⁷⁹; -(CH₂)ᵣO(CH₂)ₘNR⁸⁰C(=NR⁸⁰)NR⁷⁸R⁷⁹;
-(CH₂)ᵣO(CH₂)ₘN=C(NR⁷⁸R⁸⁰)NR⁷⁹R⁸⁰; -(CH₂)ᵣO(CH₂)ₚC₆H₄CNR⁷⁸R⁷⁹;
-(CH₂)ᵣO(CH₂)ₚC₆H₄C(=NR⁸⁰)NR⁷⁸R⁸⁰; -(CH₂)ᵣO(CH₂)ₚC₆H₄C(=NOR⁵⁰)NR⁷⁸R⁷⁹;
-(CH₂)ᵣO(CH₂)ₚC₆H₄C(=NNR⁷⁸R⁷⁹)NR⁷⁸R⁷⁹;
-(CH₂)ᵣO(CH₂)ₚC₆H₄NR⁸⁰C(=NR⁸⁰)NR⁷⁸R⁷⁹; -(CH₂)ᵣS(CH₂)ₘNR⁷⁸R⁷⁹;
-(CH₂)ᵣS(CH₂)ₘNR⁷⁷R⁸⁰; -(CH₂)ᵣS(CH₂)ₚC(=NR⁸⁰)NR⁷⁸R⁷⁹;
-(CH₂)ᵣS(CH₂)ₚC(=NOR⁵⁰)NR⁷⁸R⁷⁹; -(CH₂)ᵣS(CH₂)ₚC(=NNR⁷⁸R⁷⁹)NR⁷⁸R⁷⁹;
-(CH₂)ᵣS(CH₂)ₘNR⁸⁰C(=NR⁸⁰)NR⁷⁸R⁷⁹; -(CH₂)ᵣS(CH₂)ₘN=C(NR⁷⁸R⁸⁰)NR⁷⁹R⁸⁰;
-(CH₂)ᵣS(CH₂)ₚC₆H₄CNR⁷⁸R⁷⁹; -(CH₂)ᵣS(CH₂)ₚC₆H₄C(=NR⁸⁰)NR⁷⁸R⁷⁹;
-(CH₂)ᵣS(CH₂)ₚC₆H₄C(=NOR⁵⁰)NR⁷⁸R⁷⁹; -(CH₂)ᵣS(CH₂)ₚC₆H₄C(=NNR⁷⁸R⁷⁹)NR⁷⁸R⁷⁹;
-(CH₂)ᵣS(CH₂)ₚC₆H₄NR⁸⁰C(=NR⁸⁰)NR⁷⁸R⁷⁹; -(CH₂)ₚNR⁸⁰COR⁶⁴;
-(CH₂)ₚNR⁸⁰COR⁷⁷; -(CH₂)ₚNR⁸⁰CONR⁷⁸R⁷⁹; ou -(CH₂)ₚC₆H₄NR⁸⁰CONR⁷⁸R⁷⁹;
R⁷⁵ représente alkyle inférieur ; alcényle inférieur ; ou aryl-alkyle inférieur ; ou
R³³ et R⁷⁵ ensemble peuvent former: -(CH₂)₂-₆-; -(CH₂)₂O(CH₂)₂-; -(CH₂)₂S(CH₂)₂-; ou -(CH₂)₂NR⁵⁷(CH₂)₂- ; ou
R⁷⁵ et R⁸² ensemble peuvent former : -(CH₂)₂₋₆-; -(CH₂)₂O(CH₂)₂-; -(CH₂)₂S(CH₂)₂-; ou -(CH₂)₂NR-⁵⁷(CH₂)₂-;
R⁷⁷ représente R⁸⁸; ou un groupe hétéroaryle parmi ceux de la formule 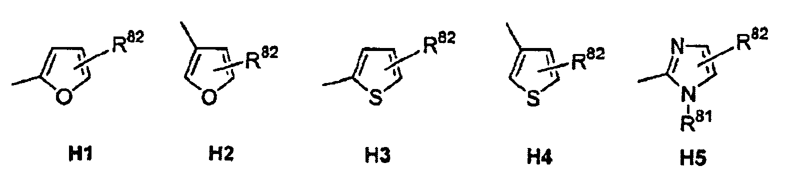 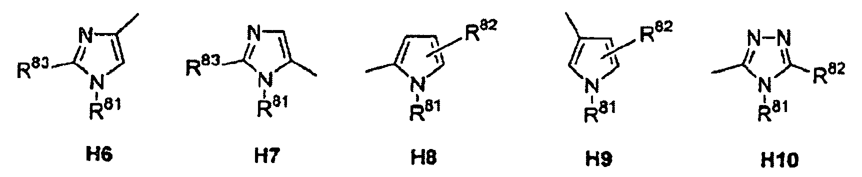 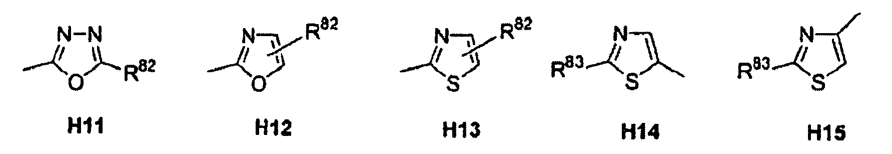 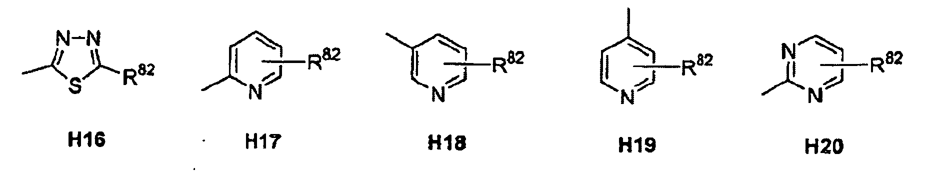 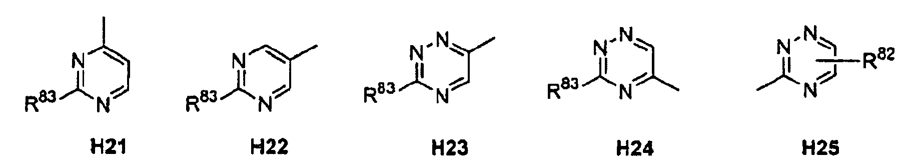 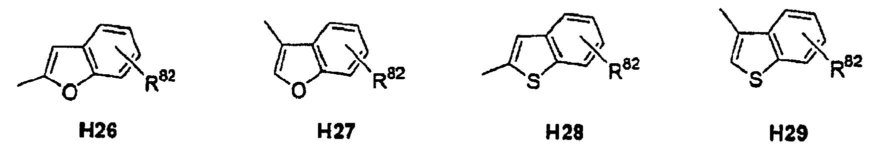 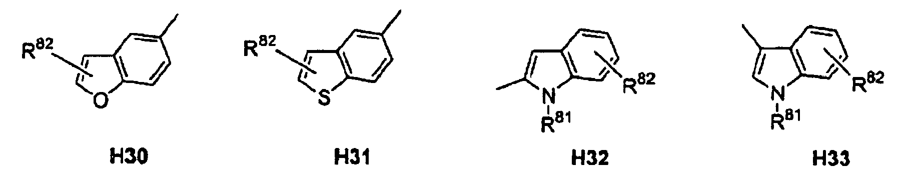 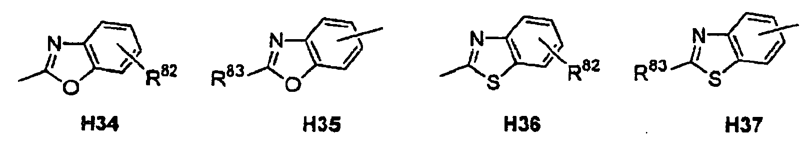 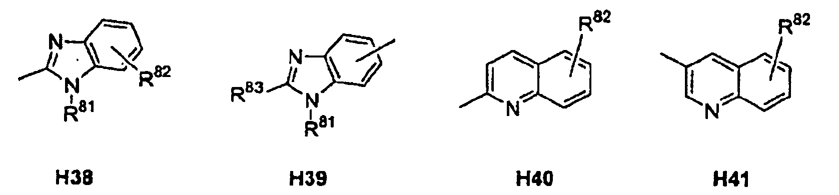 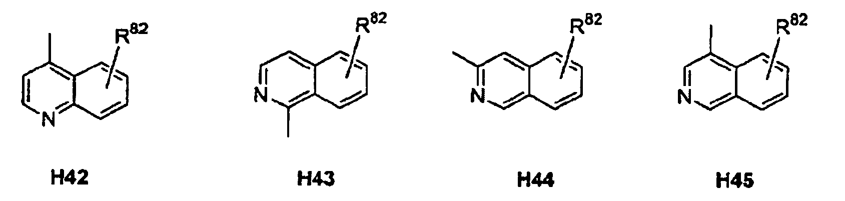 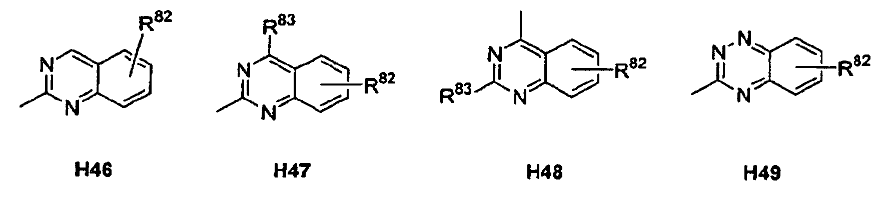 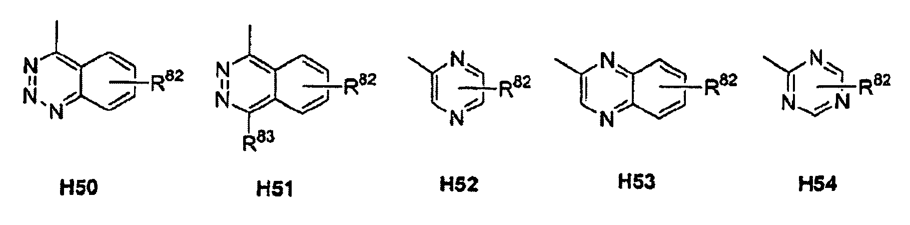
R⁷⁸ représente H ; alkyle inférieur ; aryle ; ou aryl-alkyle inférieur ;
R⁷⁸ et R⁸² ensemble peuvent former : -(CH₂)₂-₆- ; -(CH₂)₂O(CH₂)₂₋ ; -(CH₂)₂S(CH₂)₂-; ou -(CH₂)₂NR⁵⁷(CH₂)₂- ;
R⁷⁹ représente H ; alkyle inférieur ; aryle ; ou aryl-alkyle inférieur ; ou
R⁷⁸ et R⁷⁹, pris ensemble, peuvent être -(CH₂)₂₋₇- ; -(CH₂)₂O(CH₂)₂- ; ou -(CH₂)₂NR⁵⁷(CH₂)₂- ;
R⁸⁰ représente H ; ou alkyle inférieur ;
R⁸¹ représente H ; alkyle inférieur ; ou aryl-alkyle inférieur ;
R⁸² représente H ; alkyle inférieur ; aryle ; hétéroaryle ; ou aryl-alkyle inférieur ;
R³³ et R⁸² ensemble peuvent former : -(CH₂)₂₋₆- ; -(CH₂)₂O(CH₂)₂- ; -(CH₂)₂S(CH₂)₂- ; ou -(CH₂)₂NR⁵⁷(CH₂)₂- ;
R⁸³ représente H ; alkyle inférieur ; aryle ; ou -NR⁷⁸R⁷⁹ ;
R⁸⁴ représente -(CH₂)ₘ(CHR⁶¹)ₛOR⁷⁸ ; -(CH₂)ₘ(CHR⁶¹)ₛSR⁷⁸ ; -(CH₂)ₚCONR⁷⁸R⁷⁹ ;
-(CH₂)ₚNR⁸⁰CONR⁷⁸R⁷⁹ ; -(CH₂)ₚC₆H₄CONR⁷⁸R⁷⁹ ou
-(CH₂)ₚC₆H₄NR⁸⁰CONR⁷⁸R⁷⁹ ;
R⁸⁵ représente alkyle inférieur ; ou alcényle inférieur ;
R⁸⁶ représente R⁷⁴ ; -[(CH₂)ᵤ-X]ₜ-(CH2)ᵥNR⁷⁸R⁷⁹ ; -[(CH₂)ᵤ -X]ₜ-(CH₂)ᵥ-C(=NR⁸⁰)NR⁷⁸R⁷⁹ ;
X est -O-, -NR²⁰-, -S-, -OCOO-, u est 1-3, t est 1-6, v est 1-3 ;
R⁸⁷ représente R⁸⁴ ; -[(CH₂)ᵤ-X]ₜ-(CH₂)ᵥOR⁷⁸ ; -[(CH₂)ᵤ -X]ₜ-(CH₂)ᵥ-CONR⁷⁸R⁷⁹ ; -[(CH₂)ᵤ -X]ₜ-(CH₂)ᵥNR⁸⁰CONR⁷⁸R⁷⁹ ; -[(CH₂)ᵤ-X]ₜ-(CH₂)ᵥSR⁷⁸ ;
X est -O-, -NR²⁰-, -S-, -OCOO-, u est 1 - 3, t est 1 - 6, v est 1 - 3 ;
R⁸⁸ représente phényle, p-hydrxyphényle, 2-naphthyle, 1-naphthyle, 4-chlorophényle,
3-chlorophényle, 2-chlorophényle, 3,4-dichlorophényle, 4-fluorophényle,
3-fluorophényle, 2-fluorophényle, p-benzyloxyphényle, p-biphényle ou
p-benzoylphényle.
à condition que dans la chaîne de 12 résidus d'acides α-aminés Z les résidus d'acides aminés dans les positions 1 à 12 sont :
- P1 : du type C ou du type D ou du type E ou du type F, ou le résidu est Pro ;
- P2 : du type E ou du type D ;
- P3 : du type C, ou le résidu est Pro ;
- P4 : du type E ou du type F ou du type I ou du type K ;
- P5 : du type E ou du type D ou du type C ou du type I ou du type K ou du type F, ou le résidu est Gly ou Pro ;
- P6 : du type E ou du type F ou du type I ou du type K ou du type D, ou le résidu est Gly ;
- P7 : du type E ou du type F ou du type I ou du type C ;
- P8 : du type D ou du type C ou le résidu est Pro ;
- P9 : du type E ou du type D ou du type F ;
- P10 : du type D ou du type C, ou le résidu est Pro ;
- P11 : du type E ou du type D ou du type C ; et
- P12 : du type C ou du type D ou du type E ou du type F, ou le résidu est Pro ; ou
- P4 et P9 et/ ou P2 et P11, pris ensemble, peuvent former un groupe du type H ; et dans P6 et P7, des isomères D sont également possibles;
à la condition supplémentaire que ladite chaîne de 12 résidus d'acides α-aminés comprend au moins un résidu du type I ou du type K ;
et leurs sels pharmaceutiquement acceptables.

2. Des composés selon la revendication 1 dans lesquels les résidus d'acides α-aminés dans les positions 1 à 12 de la chaîne Z sont :
- P1 : du type C ou du type D ou du type E ou du type F,
- P2 : du type D ou du type E ;
- P3 : du type C ;
- P4 : du type E ou du type I ou du type F ;
- P5 : du type E ou du type I ou du type F ;
- P6 : du type E ou du type I ou du type D ;
- P7 : du type E ou du type I ou du type C ;
- P8 : du type D ;
- P9 : du type E ;
- P10 : du type D ou du type C,
- P11 : du type E ou du type D ou du type C ; et
- P12 : du type C ou du type D ou du type E ou du type F ;
- dans P6 et P7, des isomères D sont également possibles ;
à la condition qu'au moins un des résidus d'acides aminés est du type I.

3. Des composés selon la revendication 2 dans lesquels les résidus d'acides α-aminés dans les positions 1 à 12 de la chaîne Z représentent :
- P1 : Leu ; Tyr ou Arg ;
- P2 : Arg ; ou Trp ;
- P3 : Leu ;
- P4 : Lys ; hArg ; (BA)G ; ou Gln ;
- P5 : Lys ; Gln ; hArg ; ou (PeA)G ;
- P6 : Arg, Trp, hArg ; (EGU)G ;
- (EA)G ; (PrA)G ; (PeA)G ou (BA)G ;
- P7 : Arg ; (PeA)G ; ou Val ;
- P8 : Trp ; ou Bip ;
- P9 : Lys ; Arg ; ou hArg ;
- P10 : Tyr ;
- P11 : Arg ; ou Tyr ; et
- P12 Val ; ou Arg
à la condition que
- le résidu d'acide aminé dans P4 est (BA)G ; et/ou
- le résidu d'acide aminé dans P5 est (PeA)G ; et/ou
- le résidu d'acide aminé dans P6 est (EGU)G ou (EA)G ou (PrA)G ou (PeA)G ou (BA)G ; et/ou
- le résidu d'acide aminé dans P7 est (PeA)G.

4. Un composé de la formule I selon la revendication 1 dans lequel le template représente ^{D}Pro - ^{L}Pro, et les résidus d'acides aminés dans les positions 1 à 12 représentent :
- P1 : Leu ;
- P2 : Arg ;
- P3 : Leu ;
- P4 : Lys ;
- P5 : Lys ;
- P6 : (EA)G ;
- P7 : Arg ;
- P8 : Trp ;
- P9 : Lys ;
- P10 : Tyr ;
- P11 : Arg ; et
- P12 : Val

5. Un composé de la formule I selon la revendication 1 dans lequel le template représente ^{D}Pro - ^{L}Pro, et les résidus d'acides aminés dans les positions 1 à 12 représentent :
- P1 : Leu ;
- P2 : Arg ;
- P3 : Leu ;
- P4 : hArg ;
- P5 : hArg ;
- P6 : (EGU)G ;
- P7 : Arg ;
- P8 : Trp ;
- P9 : hArg ;
- P10 : Tyr ;
- P11 : Arg ; et
- P12 : Val

6. Un composé de la formule I selon la revendication 1 dans lequel le template représente ^{D}Pro - ^{L}Pro, et les résidus d'acides aminés dans les positions 1 à 12 représentent :
- P1 : Leu ;
- P2 : Arg ;
- P3 : Leu ;
- P4 : Lys ;
- P5 : Lys ;
- P6 : (PrA)G ;
- P7 : Arg ;
- P8 : Trp ;
- P9 : Lys ;
- P10 : Tyr ;
- P11 : Arg ; et
- P12 : Val

7. Un composé de la formule I selon la revendication 1 dans lequel le template représente ^{D}Pro - ^{L}Pro, et les résidus d'acides aminés dans les positions 1 à 12 représentent :
- P1 : Leu;
- P2 : Arg ;
- P3 : Leu ;
- P4 : Lys ;
- P5 : Lys ;
- P6 : (BA)G ;
- P7 : Arg ;
- P8 : Bip ;
- P9 : Lys ;
- P10 : Tyr ;
- P11 : Arg ; et
- P12 : Val

8. Un composé de la formule I selon la revendication 1 dans lequel le template représente ^{D}Pro - ^{L}Pro, et les résidus d'acides aminés dans les positions 1 à 12 représentent :
- P1 : Leu ;
- P2 : Arg ;
- P3 : Leu ;
- P4 : (BA)G ;
- P5 : Lys ;
- P6 : (BA)G ;
- P7 : Arg ;
- P8 : Bip ;
- P9 : Lys ;
- P10 : Tyr ;
- P11 : Arg ; et
- P12 : Val

9. Un composé de la formule I selon la revendication 1 dans lequel le template représente ^{D}Pro - ^{L}Pro, et les résidus d'acides aminés dans les positions 1 à 12 représentent :
- P1 : Leu ;
- P2 : Arg ;
- P3 : Leu ;
- P4 : Lys ;
- P5 : Lys ;
- P6 : (PrA)G ;
- P7 : Arg ;
- P8 : Bip ;
- P9 : Lys ;
- P10 : Tyr ;
- P11 : Arg ; et
- P12 : Val

10. Un composé de la formule I selon la revendication 1 dans lequel le template représente ^{D}Pro - ^{L}Pro, et les résidus d'acides aminés dans les positions 1 à 12 représentent :
- P1 : Arg ;
- P2 : Trp ;
- P3 : Leu ;
- P4 : Lys ;
- P5 : Lys ;
- P6 : Arg ;
- P7 : (PeA)G ;
- P8 : Trp ;
- P9 : Lys ;
- P10 : Tyr ;
- P11 : Tyr ; et
- P12 : Val

11. Un composé de la formule I selon la revendication 1 dans lequel le template représente ^{D}Pro - ^{L}Pro, et les résidus d'acides aminés dans les positions 1 à 12 représentent :
- P1 : Arg ;
- P2 : Trp ;
- P3 : Leu ;
- P4 : Gln ;
- P5 : (PeA)G ;
- P6 : Arg ;
- P7 : Arg ;
- P8 : Trp ;
- P9 : Lys ;
- P10 : Tyr ;
- P11 : Tyr ; et
- P12 : Arg

12. Un composé de la formule I selon la revendication 1 dans lequel le template représente ^{D}Pro - ^{L}Pro, et les résidus d'acides aminés dans les positions 1 à 12 représentent :
- P1 : Arg ;
- P2 : Trp ;
- P3 : Leu ;
- P4 : Lys ;
- P5 : (PeA)G ;
- P6 : Arg ;
- P7 : Arg ;
- P8 : Trp ;
- P9 : Lys ;
- P10 : Tyr ;
- P11 : Tyr ; et
- P12 : Val

13. Un composé de la formule I selon la revendication 1 dans lequel le template représente ^{D}Pro - ^{L}Pro, et les résidus d'acides aminés dans les positions 1 à 12 représentent :
- P1 : Thr ;
- P2 : Trp ;
- P3 : Leu ;
- P4 : Lys ;
- P5 : (PeA)G ;
- P6 : Arg ;
- P7 : Arg ;
- P8 : Trp ;
- P9 : Lys ;
- P10 : Tyr ;
- P11 : Tyr ; et
- P12 : Arg

14. Un composé de la formule I selon la revendication 1 dans lequel le template représente ^{D}Pro - ^{L}Pro, et les résidus d'acides aminés dans les positions 1 à 12 représentent :
- P1 : Arg ;
- P2 : Trp ;
- P3 : Leu ;
- P4 : Gln ;
- P5 : Lys ;
- P6 : Arg ;
- P7 : (PeA)G ;
- P8 : Trp ;
- P9 : Lys ;
- P10 : Tyr ;
- P11 : Tyr ; et
- P12 : Arg

15. Un composé de la formule I selon la revendication 1 dans lequel le template représente ^{D}Pro - ^{L}Pro, et les résidus d'acides aminés dans les positions 1 à 12 représentent :
- P1 : Thr ;
- P2 : Trp ;
- P3 : Leu;
- P4 : Lys ;
- P5 : (PeA)G ;
- P6 : Arg ;
- P7 : Arg ;
- P8 : Trp ;
- P9 : Lys ;
- P10 : Tyr ;
- P11 : Tyr ; et
- P12 : Arg

16. Des énantiomères des composés de la formule I selon la définition dans la revendication 1.

17. Des composés selon l'une quelconque des revendications 1 à 16 pour l'utilisation en tant que substances thérapeutiquement actives.

18. Des composés selon la revendication 17 ayant une activité antibactérienne.

19. Une composition pharmaceutique comprenant un composé selon l'une quelconque des revendications 1 à 17 et un porteur pharmaceutiquement inerte.

20. Des compositions selon la revendication 19 sous une forme appropriée pour l'administration orale, topicale, transdermale, par injection, buccale, transmucosal, pulmonaire ou par inhalation.

21. Des compositions selon la revendication 18 ou 19 sous forme de tablettes, dragées, capsules, solutions, liquides, gels, sparadraps, crèmes, pommades, sirops, émulsions, suspensions, spray, vaporisateur ou suppositoires.

22. L'utilisation des composés selon l'une quelconque des revendications 1 à 16 pour la fabrication d'un médicament pour le traitement ou la prévention d'infections ou de maladies liées à de telles infections, cette maladie étant en particulier la fibrose cystique.

23. L'utilisation des composés selon l'une quelconque des revendications 1 à 16 en tant que désinfectants ou agents de conservation pour des denrées alimentaires, des cosmétiques, des médicaments et d'autres matières comprenant des substances nutritives.

24. Un procédé pour la fabrication de composés selon l'une quelconque des revendications 1 à 15 où ce procédé comprend
(a) coupler un support solide convenablement fonctionnalisé avec un dérivé convenablement N-protégé de cet acide aminé qui est dans le produit final souhaité à la position 5, 6 ou 7, tout groupe fonctionnel qui peut être présent dans le dit dérivé d'acide aminé N-protégé étant également protégé de manière appropriée;
(b) éliminer le groupe protecteur de N du produit ainsi obtenu;
(c) coupler le produit ainsi obtenu avec un dérivé convenablement N-protégé de cet acide aminé qui est dans le produit final souhaité une position plus proche du résidu d'acide aminé N-terminal, tout groupe fonctionnel qui peut être présent dans le dérivé d'acide aminé N-protégé étant également protégé de manière appropriée ;
(d) éliminer le groupe protecteur de N du produit ainsi obtenu;
(e) répéter les étapes (c) et (d) jusqu'à ce que le résidu d'acide aminé N-terminal ait été introduit ;
(f) coupler le produit ainsi obtenu avec
(fa) un dérivé convenablement N-protégé de ^{L}Pro ou ^{D}Pro ;
(fb) éliminer le groupe protecteur de N du produit ainsi obtenu ; et
(fc) coupler le produit ainsi obtenu avec un dérivé convenablement N-protégé de ^{D}Pro, respectivement ^{L}Pro;
(g) éliminer le groupe protecteur de N du produit obtenu dans l'étape (fc) ;
(h) coupler le produit ainsi obtenu avec un dérivé convenablement N-protégé de cet acide aminé qui est dans le produit final souhaité dans la position 12, tout groupe fonctionnel qui peut être présent dans le dérivé d'acide aminé N-protégé étant également protégé de manière appropriée ;
(i) éliminer le groupe protecteur de N du produit ainsi obtenu;
(j) coupler le produit ainsi obtenu avec un dérivé convenablement N-protégé de cet acide aminé qui est dans le produit final souhaité une position plus loin que la position 12, tout groupe fonctionnel qui peut être présent dans le dérivé d'acide aminé N-protégé étant également protégé de manière appropriée ;
(k) éliminer le groupe protecteur de N du produit ainsi obtenu ;
(l) répéter les étapes (j) et (k) jusqu'à ce que tous les résidus d'acides aminés ait été introduits;
(m) optionnellement, déprotection sélective d'un groupe fonctionnel protégé ou de plusieurs groupes fonctionnels protégés présents dans la molécule et substitution appropriée du groupe réactif ou des groupes réactifs ainsi libérés ;
(o) détacher le produit ainsi obtenu du support solide;
(p) cycliser le produit clivé du support solide ;
(q) optionnellement, formation d'un ou de deux liaisons entre brins entre des chaînes latérales des résidus d'acides aminés appropriés aux positions opposées de la zone du brin β ;
(r) éliminer tous les groupes protecteurs présents sur les groupes fonctionnels de tous les membres de la chaîne des résidus d'acides aminés et, optionnellement, tout groupe protecteur qui peut additonnellement être présent dans la molécule ;
(s) optionnellement, guanidinyser tout groupe aminé des chaînes latérales présent dans la chaîne des résidus d'acides aminés ; et
(t) optionnellement, convertir le produit ainsi obtenu en un sel pharmaceutiquement acceptable ou convertir un sel pharmaceutiquement acceptable ou non-acceptable ainsi obtenu en le composé libre correspondant de la formule I ou en un autre sel différent, pharmaceutiquement acceptable.

25. Un procédé pour la fabrication de composés selon l'une quelconque des revendications 1 à 15 où ce procédé comprend
(a') coupler un support solide convenablement fonctionnalisé
(a'a) coupler ce support solide convenablement fonctionnalisé avec un dérivé convenablement N-protégé de ^{L}Pro ou ^{D}Pro;
(a'b) éliminer le groupe protecteur de N du produit ainsi obtenu ; et
(a'c) coupler le produit ainsi obtenu avec un dérivé convenablement N-protégé de ^{D}Pro resp. ^{L}Pro ;
(b') éliminer le groupe protecteur de N du produit obtenu dans l'étape (a'c);
(c') coupler le produit ainsi obtenu avec un dérivé convenablement N-protégé de cet acide aminé qui est dans le produit final souhaité une position plus proche du résidu d'acide aminé N-terminal, tout groupe fonctionnel qui peut être présent dans le dérivé d'acide aminé N-protégé étant également protégé de manière appropriée ;
(d') éliminer le groupe protecteur de N du produit ainsi obtenu;
(e') coupler le produit ainsi obtenu avec un dérivé convenablement N-protégé de cet acide aminé qui est dans le produit final souhaité une position plus loin que la position 12, tout groupe fonctionnel qui peut être présent dans le dérivé d'acide aminé N-protégé étant également protégé de manière appropriée ;
(f') éliminer le groupe protecteur de N du produit ainsi obtenu;
(g') répéter les étapes (e') et (f') jusqu'à ce que tous les résidus d'acides aminés ait été introduits;
(h') optionnellement, déprotection sélective d'un groupe fonctionnel protégé ou de plusieurs groupes fonctionnels protégés présents dans la molécule et substitution appropriée du groupe réactif ou des groupes réactifs ainsi libérés ;
(i') détacher le produit ainsi obtenu du support solide;
(j') cycliser le produit clivé du support solide ;
(k') optionnellement, formation d'un ou de deux liaisons entre brins entre des chaînes latérales des résidus d'acides aminés appropriés aux positions opposées de la zone du brin β ;
(l') éliminer tous les groupes protecteurs présents sur les groupes fonctionnels de tous les membres de la chaîne des résidus d'acides aminés et, optionnellement, tout groupe protecteur qui peut additonnellement être présent dans la molécule ;
(m') optionnellement, guanidinyser tout groupe aminé des chaînes latérales présent dans la chaîne des résidus d'acides aminés ; et
(n') optionnellement, convertir le produit ainsi obtenu en un sel pharmaceutiquement acceptable ou convertir un sel pharmaceutiquement acceptable ou non-acceptable ainsi obtenu en le composé libre correspondant de la formule I ou en un autre sel différent, pharmaceutiquement acceptable.

26. Un procédé selon la revendication 24 ou 25, mais où un résidu d'acide aminé du type I ou du type K est introduit par couplage avec un agent acétylant contenant un groupe de départ, suivi par un déplacement nucléophilique avec une amine de la formule H₂NR⁸⁶ resp. H₂NR⁸⁷ qui est protégée de manière appropriée, si nécessaire.

27. Un procédé selon la revendication 26 où ledit agent acétylant contenant le groupe de départ est bromo, chloro ou iodo acide acétique.

28. Une modification du procédé selon l'une quelconque des revendications 24 à 27 pour la fabrication de composés selon la revendication 16 dans lesquels des énantiomères de toutes les matières de départ chirales sont utilisées.
